# EUROPEAN PATENT APPLICATION

(11) **EP 3 671 218 A1**
(43) Date of publication of application: **24.06.2020**
(21) Application number: 17921726.0
(22) Date of filing: 16.08.2017
(51) Int. Cl.: G01N 35/00

(54) **BLOOD ANALYZER AND CONTROL METHOD THEREFOR**

(71) Applicant: Beijing Precil Instrument Co., Ltd., Beijing 100085 (CN)
(72) Inventor: ZHANG, Mingwei, Beijing 100085 (CN); XU, Haiqing, Beijing 100085 (CN)
(74) Representative: KIPA AB
(86) International application number: PCT/CN2017/097712
(87) International publication number: WO 2019/033312

(57) **Abstract**

A blood analyzer (1) and a control method therefor, the blood analyzer (1) comprising a sample transport module (11) for transporting a sample to be tested, a dispensing module (13) for aspirating and discharging samples or reagents, a sample incubation module (14) for incubating samples, a reagent storage module (15) for storing the reagents, a transfer module (16) for transferring an assay cup, and a sample detection module (17) for detecting the samples. The dispensing module (13) is capable of transferring the samples from a sample delivery module (11) to the assay cup of the sample incubation module (14), and the dispensing module (13) may also transfer the reagents from a first reagent storage mechanism (151) to the assay cup of the sample incubation module (14) or the assay cup of the sample detection module (17); and the transfer module (16) is capable of transferring the assay cup in the sample incubation module (14) to the sample detection module (17). The described blood analyzer (1) may easily facilitate modular operation and is convenient for adding a module to achieve a corresponding function, thus being easy to expand.

## Description

### Technical Field

The present invention relates to the technical field of medical apparatuses, and in particular, to a blood analyzer for analyzing a sample such as blood and a control method therefor.

### Background Art

Fully-automatic blood coagulation analyzers are instruments used to analyze blood coagulation and anticoagulation, and fibrinolysis and antifibrinolysis functions of blood. At present, the fully-automatic blood coagulation analyzer generally comprises: a sample storage device, a reagent refrigeration device, a testing device, a sample adding device, a cup grasping device and an automatic cup feeding device. The workflow is basically as follows: the automatic cup feeding device carries a test cup to a cup grasping station, a cup grasping hand in the cup grasping device carries the blood coagulation test cup from the cup grasping station to a testing station of the testing device, a sample adding needle in the sample adding device carries a blood sample in the sample storage device and a reaction reagent in the reagent refrigeration device to the test cup of the testing station, the sample and the reagent react in the test cup, and the testing device measures the reaction result.

At present, when the fully-automatic blood coagulation analyzer uses an X/Y/Z movement mechanism to realize the automatic operation of blood coagulation inspection, there are problems of modularization and expansion being not easily achieved. When the sample adding device and the reagent refrigeration device for the fully-automatic blood coagulation analysis move independently to realize the automatic operation of blood coagulation inspection, the fully-automatic blood coagulation analyzer cannot achieve better functions due to the limitation of the layout of various components. In this case, the entire device of the fully-automatic blood coagulation analyzer is not easily modularized and expanded, and does not enable the fully-automatic blood coagulation analyzer to achieve better functions, affecting the use.

### Summary of the Invention

On this basis, it is necessary to provide a blood analyzer which can be easily modularized and expanded to solve the problems in the current fully-automatic blood coagulation analyzers of modularization and expansion being not easily achieved, and also to provide a control method applicable to the above blood analyzer.

The above objects are achieved by the following technical solutions.

A blood analyzer, comprising a sample transport module for transporting a sample to be tested, a dispensing module for aspirating and discharging the sample or a reagent, a sample incubation module for incubating the sample, a reagent storage module for storing the reagent, a transfer module for transferring a reaction cup, and a sample detection module for detecting the sample, wherein
the sample incubation module is provided in a disk-shaped structure, is provided with a plurality of placement stations for placing the reaction cup, and is capable of rotating and driving the reaction cup in the placement station to rotate;
the reagent storage module comprises a rotatable first reagent storage mechanism provided in a disk-shaped structure, the first reagent storage mechanism being capable of storing a plurality of reagents, and being arranged separated from the sample incubation module;
the transfer module is capable of transferring the reaction cup to the placement station of the sample incubation module;
the dispensing module is capable of transferring the sample from the sample transport module to the reaction cup in the sample incubation module, and is further capable of transferring the reagent from the first reagent storage mechanism to the reaction cup in the sample incubation module or to the reaction cup in the sample detection module; and
the transfer module is capable of transferring the reaction cup from the sample incubation module to the sample detection module.

A blood analyzer, comprising a sample transport module for transporting a sample to be tested, a dispensing module for aspirating and discharging the sample or a reagent, a sample incubation module for incubating the sample, a reagent storage module for storing the reagent, a transfer module for transferring a reaction cup, and a sample detection module for detecting the sample, wherein
the sample incubation module is provided in a disk-shaped structure, is provided with a plurality of placement stations for placing the reaction cup, and is capable of rotating and driving the reaction cup in the placement station to rotate;
the reagent storage module comprises a rotatable first reagent storage mechanism, the first reagent storage mechanism being capable of storing a plurality of reagents;
the sample detection module comprises a magnetic bead detection mechanism and an optical detection mechanism, the magnetic bead detection mechanism performing a magnetic bead detection on the sample, and the optical detection mechanism performing an optical detection on the sample;
the transfer module is capable of transferring the reaction cup to the placement station of the sample incubation module;
the dispensing module is capable of transferring the sample from the sample transport module to the reaction cup in the sample incubation module, and is further capable of transferring the reagent from the first reagent storage mechanism to the reaction cup in the sample incubation module or to the reaction cup in the sample detection module; and
the transfer module is capable of transferring the reaction cup from the sample incubation module to the magnetic bead detection mechanism or the optical detection mechanism.

A blood analyzer, a cup feeding module for transporting the reaction cup, a sample transport module for transporting a sample to be tested, a dispensing module for aspirating and discharging the sample or a reagent, a sample incubation module for incubating the sample, a transfer module for transferring the reaction cup, a reagent storage module for storing the reagent, a sample detection module for detecting the sample, and a recovery module, wherein
the sample incubation module is provided in a disk-shaped structure, is provided with a plurality of placement stations for placing the reaction cup, and is capable of rotating and driving the reaction cup in the placement station to rotate;
the reagent storage module comprises a rotatable first reagent storage mechanism, the first reagent storage mechanism being capable of storing a plurality of reagents;
the transfer module is capable of transferring the reaction cup to the placement station of the sample incubation module; the dispensing module is capable of transferring the sample from the sample transport module to the reaction cup in the sample incubation module, and is further capable of transferring the reagent from the first reagent storage mechanism to the reaction cup in the sample incubation module or to the reaction cup in the sample detection module;
the transfer module is capable of transferring the reaction cup from the sample incubation module to the sample detection module; the transfer module is further capable of transferring the reaction cup that has been detected by the sample detection module to the recovery module; and
the recovery module has a cup discarding opening, the cup feeding module has an empty cup picking-up position, the blood analyzer has a cup picking-up and placement position provided corresponding to the sample incubation module, the sample detection module has a detection position, and at least three of the empty cup picking-up position, the cup discarding opening, the cup picking-up and placement position and the detection position are arranged collinearly and correspond to a movement trajectory of the transfer module.

A control method for a blood analyzer, wherein the blood analyzer comprises a sample transport module for transporting a sample to be tested, a dispensing module for aspirating and discharging the sample or a reagent, a sample incubation module for incubating the sample, a transfer module for transferring the reaction cup, and a sample detection module for detecting the sample, wherein
the sample incubation module is provided in a disk-shaped structure, is provided with a plurality of placement stations for placing the reaction cup, and is capable of rotating and driving the reaction cup in the placement station to rotate;
the sample detection module comprises a magnetic bead detection mechanism and an optical detection mechanism, the magnetic bead detection mechanism performing a magnetic bead detection on the sample, and the optical detection mechanism performing an optical detection on the sample; and
the control method for the blood analyzer comprises the following steps:
obtaining detection information of a test item of the sample;
placing, which can be performed by the transfer module, the empty reaction cup to the placement station of the sample incubation module;
aspirating, by the dispensing module, the sample at the sample transport module or the reagent at the reagent storage module, and adding the sample or the reagent to the reaction cup in the sample incubation module; and
transferring, which can be performed by the transfer module, the reaction cup, in which the sample and the reagent have been added, to the magnetic bead detection mechanism or the optical detection mechanism.

With the use of the above technical solutions, the present invention has the beneficial effects as follows:
in the blood analyzer of the present invention, when the sample is being detected, the transfer module transfers the reaction cup to the sample incubation module, and the sample transport module transports the sample to be tested; the dispensing module can aspirate in the sample transported by the sample transport module and transfer the sample to the reaction cup in the sample incubation module, and the dispensing module can also aspirate in the reagent from the reagent storage module and transfer the reagent to the reaction cup in the sample incubation module; the transfer module then transports the reaction cup from the sample incubation module to the sample detection module for detection; each component of the blood analyzer of the present invention performs the above steps according to its arrangement, such that the blood analyzer can easily implement modular operations, and effectively solve the problems in the current fully-automatic blood coagulation analyzers of modularization and expansion being not easily achieved; and it is convenient to add modules to the blood analyzer to achieve the corresponding functions, such that the blood analyzer is easily expanded, and the blood analyzer of the present invention can also achieve better functions, improve the operation efficiency, and be convenient to use.

### Brief Description of the Drawings

Fig. 1 is a perspective view of a blood analyzer according to an embodiment of the present invention viewed from one direction;
Fig. 2 is a perspective view of the blood analyzer shown in Fig. 1 viewed from another direction;
Fig. 3 is a top view of the blood analyzer shown in Fig. 1;
Fig. 4 is a perspective view of a first reagent storage mechanism of the blood analyzer shown in Fig. 1;
Fig. 5 is a perspective view of a reagent disk of the first reagent storage mechanism shown in Fig. 4, with a reagent rack being removed from a chassis;
Fig. 6 is a perspective view of a sample transport module of the blood analyzer shown in Fig. 1;
Fig. 7 is a perspective view of the sample transport module shown in Fig. 6 with a sample transport top cover being removed;
Fig. 8 is a partial perspective view of a cup feeding module of the blood analyzer shown in Fig. 2;
Fig. 9 is a perspective view of a magnetic bead detection mechanism of the blood analyzer shown in Fig. 1;
Fig. 10 is a perspective view of a first optical detection mechanism of the blood analyzer shown in Fig. 1;
Fig. 11 is a perspective view of a first transfer mechanism of the blood analyzer shown in Fig. 1;
Fig. 12 is a perspective view of a second transfer mechanism of the blood analyzer shown in Fig. 1;
Fig. 13 is a perspective view of a sample incubation module of the blood analyzer described in Fig. 1;
Fig. 14 is a top view of the blood analyzer shown in Fig. 1, showing a recovery module;
Fig. 15 is a perspective view of a stirring module of the blood analyzer shown in Fig. 1;
Fig. 16 is a perspective view of a puncture needle mechanism of the blood analyzer shown in Fig. 1;
Fig. 17 is a perspective view of an integrated needle mechanism of the blood analyzer shown in Fig. 1;
Fig. 18 is a perspective view of a reagent needle mechanism of the blood analyzer shown in Fig. 1;
Fig. 19 is a top view of the blood analyzer shown in Fig. 1, showing a reagent needle avoidance space;
Fig. 20 shows schematic views of movement trajectories of a stirring rod of the stirring module shown in Fig. 15;
Fig. 21 is a perspective view of a blood analyzer according to another embodiment of the present invention viewed from one direction;
Fig. 22 is a perspective view of the blood analyzer shown in Fig. 21 viewed from another direction;
Fig. 23 is a top view of the blood analyzer shown in Fig. 21; and
Fig. 24 is a top view of a blood analyzer according to yet another embodiment of the present invention.

In the figures:
1 - Blood analyzer;
10 - Test housing;
   101 - Testing platform; 102 - Mounting frame; 103 - Cover plate; 104 - Placement cavity;
11 - Sample transport module;
   111 - Detection assembly; 112 - Rotation assembly; 113 - Scanning mechanism; 114 - Sampling assembly; 115 - Loading assembly; 116 - Unloading assembly; 117 - Reversing assembly; 118 - Sample transport floor; 119 - Sample transport roof; 1191 - Card swiping component;
12 - Cup feeding module;
   121 - Cup feeding bracket;
   122 - Reaction cup storage mechanism; 1221 - Reaction cup storage disk; 1222 - Film removal component;
   123 - Reaction cup transport mechanism;
   124 - Reaction cup transfer mechanism; 1241 - Reaction cup carrying component; 1242 - Blocking component;
13 - Dispensing module;
   131 - Puncture needle mechanism;
      1311 - Puncture needle support frame;
      1312 - Puncture needle lifting drive assembly; 13121 - Puncture needle lifting drive motor; 13122 - Puncture needle lifting transmission component;
      1313 - Puncture needle rotation drive assembly; 13131 - Puncture needle rotation drive motor; 13132 - Puncture needle rotation transmission component;
      1314 - Puncture needle mounting assembly; 13141 - Puncture needle rotating shaft; 13142 - Puncture needle rotating arm;
      1315 - Puncture needle;
   132 - Integrated needle mechanism;
      1321 - Integrated needle support frame;
      1322 - Integrated needle lifting drive assembly; 13221 - Integrated needle lifting drive motor; 13222 - Integrated needle lifting transmission component;
      1323 - Integrated needle rotation drive assembly; 13231 - Integrated needle rotation drive motor; 13232 - Integrated needle rotation transmission component;
      1324 - Integrated needle mounting assembly; 13241 - Integrated needle rotating shaft; 13242 - Integrated needle rotating arm;
      1325 - Integrated needle;
      1326 - Integrated needle level detection board;
   133 - Reagent needle mechanism;
      1331 - Reagent needle support frame;
      1332 - Reagent needle lifting drive assembly; 13321 - Reagent needle lifting drive motor; 13322 - Reagent needle lifting transmission component;
      1333 - Reagent needle rotation drive assembly; 13331 - Reagent needle rotation drive motor; 13332 - Reagent needle rotation transmission component;
      1334 - Reagent needle mounting assembly; 13341 - Reagent needle rotating shaft; 13342 - Reagent needle rotating arm;
      1335 - Reagent needle; 13351 - First reagent needle; 13352 - Second reagent needle;
      1336 - Reagent needle heating control board;
      1337 - Reagent needle level detection board;
   134 - Dispensing needle mechanism;
14 - Sample incubation module;
   141 - Incubation support frame;
   142 - Incubation drive mechanism; 1421 - Incubation drive motor; 1422 - Incubation transmission component;
   143 - Sample incubation mechanism; 1431 - Incubation disk; 14311 - Placement station; 1432 - Thermally insulating pot;
15 - Reagent storage module;
   151 - First reagent storage mechanism;
      1511 - Reagent mounting bracket;
      1512 - Reagent pot;
      1513 - Reagent disk; 15131 - Tray; 15132 - Reagent rack; 151321 - Storage station;
      1514 - First reagent drive assembly; 15141 - First reagent drive motor; 15142 - First reagent transmission component;
   152 - Second reagent storage mechanism;
16 - Transfer module;
   161 - First transfer mechanism; 1611 - First transfer mounting plate; 1612 - First grasping drive assembly; 1613 - First cup grasping portion; 1614 - First elastic member; 1615 - First transfer drive assembly; 1616 - First sliding track;
   162 - Second transfer mechanism; 1621 - Second transfer mounting plate; 1622 - Second grasping drive assembly; 1623 - Second cup grasping portion; 1624 - Second transfer drive assembly; 1625 - Second sliding track; 1626 - Third transfer drive assembly; 1627 - Third sliding track;
17 - Sample detection module;
   171 - Magnetic bead detection mechanism; 1711 - Magnetic bead support frame; 1712 - Magnetic bead detection seat; 17121 - Magnetic bead detection station; 1713 - Magnetic bead control board;
   172 - First optical detection mechanism; 1721 - First optical support frame; 1722 - First optical detection seat; 17221 - First optical detection station; 1723 - First optical receiving board;
   173 - Second optical detection mechanism;
18 - Recovery module;
   181 - First cup discarding opening; 182 - Second cup discarding opening;
19 - Stirring module;
   191 - First stirring support plate;
   192 - Second stirring support plate;
   193 - First stirring drive mechanism;
   194 - Second stirring drive mechanism; 1941 - Second stirring drive motor; 1942 - Second stirring transmission component;
   195 - Stirring mounting mechanism; 1951 - Stirring shaft; 1952 - Stirring cantilever;
   196 - Stirring rod;
20 - Cleaning module;
   201 - Puncture needle cleaning mechanism; 2011 - Puncture needle cleaning pool; 2012 - Reservoir;
   202 - Integrated needle cleaning mechanism;
   203 - Reagent needle cleaning mechanism;
   204 - Stirring rod cleaning mechanism;
21 - Liquid circuit module;
22 - Main control module.

### Detailed Description of Embodiments

In order to make the objects, technical solutions, and advantages of the present invention more apparent, the blood analyzer of the present invention will be further described below in detail through embodiments in conjunction with the accompanying drawings. It should be understood that the particular embodiments described herein are only used for illustrating the present invention and not intended to limit the present invention.

The serial numbers themselves for the components herein, for example, "first", "second", etc., are only used to distinguish the described objects, and do not have any sequential or technical meaning. As used in the present application, "connect" or "couple", unless otherwise specified, includes both direct and indirect connections (coupling). In the description of the present invention, it should be understood that the orientation or position relationship indicated by the terms "upper", "lower", "front", "back", "left", "right", "vertical" "horizontal", "top", "bottom", "inner", "outer", "clockwise", "counterclockwise" etc. are based on the orientation or position relationship shown in the accompanying drawings and are intended to facilitate the description of the present invention and simplify the description only, rather than indicating or implying that the device or element referred to must have a particular orientation or be constructed and operated in a particular orientation, and therefore will not to be interpreted as limiting the present invention.

In the present invention, unless otherwise explicitly specified and defined, the expression a first feature being "on" or "under" a second feature may be the case that the first feature is in direct contact with the second feature, or the first feature is in indirect contact with the second feature via an intermediate medium. Furthermore, the expression the first feature being "over", "above" and "on top of" the second feature may be the case that the first feature is directly above or obliquely above the second feature, or only means that the level of the first feature is higher than the second feature. The expression the first feature being "underneath", "below" and "beneath" the second feature may be the case that the first feature is directly below or obliquely below the second feature, or only means that the level of the first feature is less than the second feature.

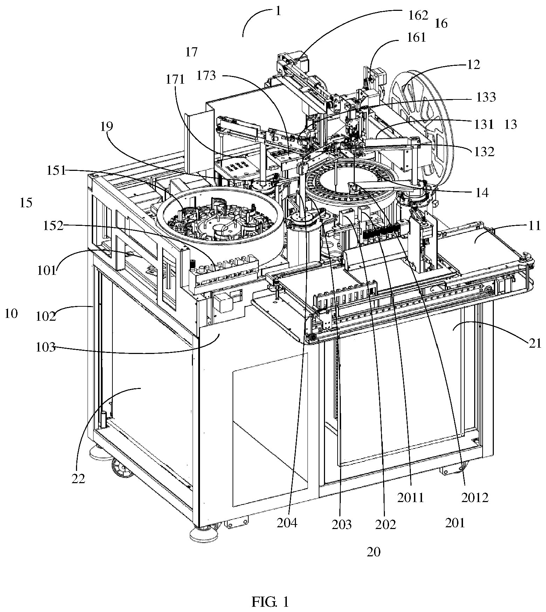

Referring to Figs. 1 to 3 and Figs. 21 to 24, the present invention provides a blood analyzer 1. The blood analyzer 1 is used to analyze and detect a sample to be tested, to obtain the corresponding detection result to meet the requirements of use. It should be noted that the specific type of the sample to be tested is not limited. In some embodiments, the sample to be tested include a solid sample or a liquid sample. It can be understood that when liquid sample is detected, the liquid sample needs to be placed on a sample rack. Further liquid samples include, but are not limited to, blood samples. When the blood analyzer 1 of the present invention is used to detect the blood samples, the blood samples are stored in test tubes and are sequentially placed on a test tube rack. The blood analyzer 1 of the present invention can easily implement modular operations, and effectively solve the problems in the current fully-automatic blood coagulation analyzers of modularization and expansion being not easily achieved; and it is convenient to add modules to the blood analyzer 1 to achieve the corresponding functions, such that the blood analyzer is easily expanded, and the blood analyzer 1 of the present invention can also achieve better functions, improve the operation efficiency, and be convenient to use.

In an implementation of the present invention, the blood analyzer 1 comprises a sample transport module 11, a cup feeding module 12, a dispensing module 13, a sample incubation module 14, a reagent storage module 15, a transfer module 16, and a sample detection module 17. The sample transport module 11 is used to transport a sample to be tested to achieve the automatic transport of the sample to be tested to improve the efficiency of sample feeding, thereby improving the working efficiency of the blood analyzer 1. The cup feeding module 12 is used to transport the reaction cup, so as to realize the automatic transport of empty reaction cups to improve the efficiency of transport. The dispensing module 13 is used to aspirate in and discharge the sample or a reagent, so as to add the sample or the reagent to the corresponding reaction cup. The sample incubation module 14 is used to incubate the sample such that the sample reaches the best reaction conditions, thereby facilitating the detection of a parameter of the sample. The reagent storage module 15 is used to store the reagent, and can store various reagents required for the detection of the sample, so as to facilitate the selection of the desired reagent, and to improve the efficiency of aspirating the reagent. The transfer module 16 is used to transfer the reaction cup such that the reaction cup can be moved to various positions of the blood analyzer 1, so as to realize the automatic analysis and detection of the sample, and to improve the operation efficiency. The sample detection module 17 is used to detect the sample to obtain the corresponding parameter of the sample. Of course, in other implementations of the present invention, the cup feeding module 12 can also be replaced, that is, the reaction cup is not transported by the cup feeding module 12, and the reaction cup can be directly placed in the sample incubation module 14. Optionally, the reaction cup transported by the cup feeding module 12 is usually a disposable consumable, of course, the reaction cup can also be recovered for reuse. Optionally, when the reaction cup can be reused, it is also possible to use no cup feeding module 12 to transport the reaction cup.

The sample incubation module 14 is provided in a disk-shaped structure. The sample incubation module 14 is provided with a plurality of placement stations 14311 for placing the reaction cup. The sample incubation module 14 can rotate and drive the reaction cup in the placement station 14311 to rotate. The sample incubation mechanism 143 has a heating function, and is used to heat the sample and the reagent in the reaction cup to realize the incubation function. The sample incubation module 14 can heat the sample and the reagent to about 34°C before the formal measurement to ensure the normal reaction.

The reagent storage module 15 comprises a rotatable first reagent storage mechanism 151 provided in a disk-shaped structure. The first reagent storage mechanism 151 can store a plurality of reagents, and can also implement cooling and automatic barcode recognition of the plurality of reagents. It should be noted that the first reagent storage mechanism 151 has a refrigeration function, and is used to store low-temperature reagents to achieve the preservation of the reagents.

Moreover, the first reagent storage mechanism 151 is arranged separated from the sample incubation module 14. That is, the first reagent storage mechanism 151 and the sample incubation module 14 are separately arranged, and there is a certain distance between the two. Preferably, in an embodiment of the present invention, the sample incubation module 14 and the first reagent storage mechanism 151 are arranged side by side. Of course, in other embodiments of the present invention, the sample incubation module 14 and the first reagent storage mechanism 151 may also be staggered. Moreover, the sample transport module 11 is located on one side of the sample incubation module 14 and the first reagent storage mechanism 151, and the cup feeding module 12, the transfer module 16 and the sample detection module 17 are located on the other side of the sample incubation module 14 and the first reagent storage mechanism 151. The cup feeding module 12 is located on the outside of the sample detection module 17 and the sample incubation module 14, the transfer module 16 is located above the sample detection module 17, and the transfer module 16 can move between the cup feeding module 12, the sample incubation module 14 and the sample detection module 17. The transfer module 16 can transfer the reaction cup transported by the cup feeding module 12 to the placement station 14311 of the sample incubation module 14, and the transfer module 16 can transfer the reaction cup from the sample incubation module 14 to the sample detection module 17.

The dispensing module 13 can transfer the sample from the sample transport module 11 to the reaction cup in the sample incubation module 14. The dispensing module 13 can further transfer the reagent from the first reagent storage mechanism 151 to the reaction cup in the sample incubation module 14 or to the reaction cup in the sample detection module 17. The dispensing module 13 aspirates in the sample from the sample transport module 11 and then adds the sample to the reaction cup in the sample incubation module 14. Subsequently, the dispensing module 13 in turn aspirates in the reagent from the first reagent storage mechanism 151 and transfers the reagent to the reaction cup in the sample incubation module 14.

The transfer module 16 can transfer an empty reaction cup transported by the cup feeding module 12 to the placement station 14311 of the sample incubation module 14. The transfer module 16 can transfer the reaction cup from the sample incubation module 14 to the sample detection module 17. The blood analyzer 1 is provided with a cup picking-up and placement position, a dispensing position and a cup picking-up position which surround the sample incubation module 14. The sample incubation module 14 is moved to the cup picking-up and placement position, and at the position of the cup picking-up and placement position, the transfer module 16 places the empty reaction cup grasped at the cup feeding module 12 to the placement station 14311 of the sample incubation module 14 corresponding to the cup picking-up and placement position. Subsequently, the sample incubation module 14 drives the reaction cup to move to the dispensing position at which the dispensing module 13 transfers the sample from the sample transport module 11 to the reaction cup in the sample incubation module 14 or the reagent from first reagent storage mechanism 151 to the reaction cup in the sample incubation module 14. After the sample and the reagent have been added to the reaction cup, the sample incubation module 14 drives the reaction cup to move to the cup picking-up and placement position or the cup picking-up position, at which the transfer module 16 grasps the reaction cup in which the sample and the reagent have been added, and transfers the reaction cup to the sample detection module 17 for detection. It can be understood that the cup picking-up and placement position and the cup picking-up position may be at the same position, and the transfer module 16 places or grasps the reaction cup at the same position. Of course, in order to improve the efficiency, the cup picking-up and placement position and the cup picking-up position are at two different positions, and the transfer module 16 can grasp the reaction cup at the different positions. It should be noted that there is no limit to the order in which the sample and the reagent are added. The sample can be added to the reaction cup before adding the reagent. Of course, it is also possible to add the reagent to reaction cup before adding the sample.

Moreover, the blood analyzer 1 is provided with a reagent aspirating position corresponding to the first reagent storage mechanism 151, and the dispensing module 13 aspirates in the reagent at the reagent aspirating position. A plurality of reagents are stored in the first reagent storage mechanism 151. When a certain reagent needs to be added to the sample, the rotation of the first reagent storage mechanism 151 causes the corresponding reagent to rotate to the reagent aspirating position, and at the same time, the dispensing module 13 is moved to the reagent aspirating position, and aspirates in the reagent at the reagent aspirating position. Subsequently, the dispensing module 13 is moved to the dispensing position of the sample incubation module 14, and at the same time, the reaction cup in the sample incubation module 14 is rotated to the dispensing position at which the dispensing module 13 adds the aspirated reagent to the reaction cup in the sample incubation module 14.

The sample transport module 11, the cup feeding module 12, the dispensing module 13, the sample incubation module 14, the reagent storage module 15, the transfer module 16 and the sample detection module 17 of the blood analyzer 1 of the present invention are arranged in the manner as described above, and are executed in the following order: the transfer module 16 transfers the empty reaction cup transported by the cup feeding module 12 to the placement station 14311 of the sample incubation module 14 at the cup picking-up and placement position, the sample transport module 11 transports the sample to be tested, the dispensing module 13 aspirates in the sample and then rotates to the dispensing position of the sample incubation module 14 so as to add the sample to the reaction cup in the sample incubation module 14, the dispensing module 13 aspirates in the reagent at the reagent aspirating position of the first reagent storage mechanism 151 and adds the reagent to the reaction cup in the sample incubation module 14, and after the reagent has been added to the reaction cup in the sample incubation module 14 and after a period of time has elapsed, the transfer module 16 transfers the reaction cup, in which the sample and the reagent have been added, to the detection module for detection, such that the parameter of the sample is obtained. After performing the above steps, the blood analyzer 1 of the present invention can easily implement modular operations; and it is also convenient to add modules to the blood analyzer 1 to achieve the corresponding functions, such that the blood analyzer is easily expanded, and the blood analyzer 1 of the present invention can also achieve better functions, improve the operation efficiency, and be convenient to use.

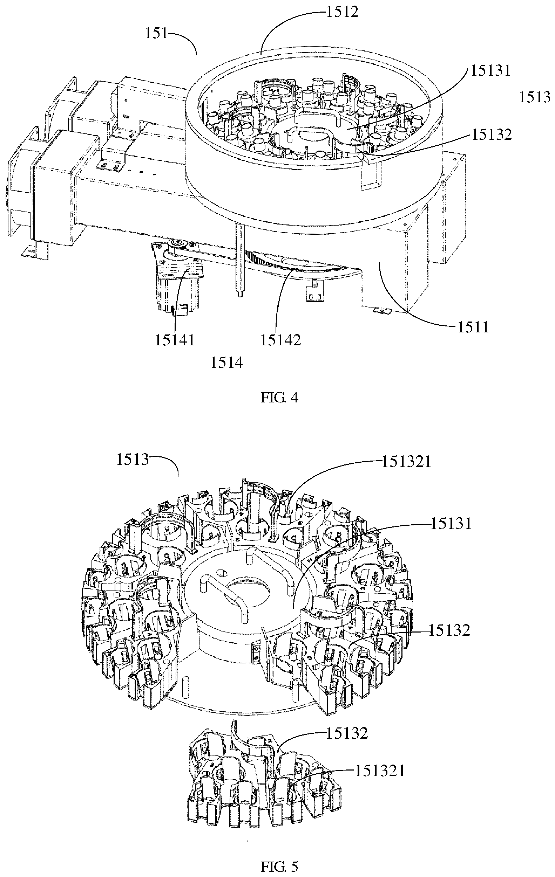

Moreover, referring to Figs. 1, 4 and 5, the first reagent storage mechanism 151 comprises a reagent mounting bracket 1511, a reagent pot 1512, a reagent disk 1513 arranged in the reagent pot 1512, and a first reagent drive assembly 1514 for driving the rotation of the reagent disk 1513. The reagent mounting bracket 1511 has a supporting function, and is mounted on the blood analyzer 1. The reagent pot 1512 is mounted on the reagent mounting bracket 1511. The reagent disk 1513 is used to store the reagent, and the reagent pot 1512 has a refrigerating function, and can refrigerate the reagent on the reagent disk 1513. The whole machine is in a turned-off state, and can support the continuous cooling of the reagent to a lower temperature, such that the reagent stays in the machine overnight. The first reagent drive assembly 1514 is connected to the reagent disk 1513 to drive the reagent disk 1513 to rotate the reagent thereon to the reagent aspirating position, which is convenient for the dispensing module 13 to aspirate in the reagent. The first reagent drive assembly 1514 comprises a first reagent drive motor 15141 and a first reagent transmission component 15142. The first reagent transmission component 15142 is in driving connection with the first reagent drive motor 15141 and the reagent disk 1513, and the first reagent drive motor 15141 drives the first reagent transmission component 15142 to rotate, so as to drive the reagent disk 1513 to rotate in the reagent pot 1512, which is convenient for aspirating the reagent.

The reagent disk 1513 comprises a tray 15131 provided in a circular shape and a plurality of reagent racks 15132 provided on the tray 15131. The tray 15131 is provided at the bottom of the reagent pot 1512. The tray 15131 is in driving connection with the first reagent transmission component 15142 in the reagent pot 1512. The first reagent transmission component 15142 can drive the tray 15131 to rotate in the reagent pot 1512. The tray 15131 can also be directly disengaged from the first reagent transmission component 15142 in an axial direction to achieve the removal of the reagent disk 1513. The plurality of reagent racks 15132 are arranged circumferentially on the tray 15131. The reagent racks 15132 can be directly disengaged from the tray 15131 in the axial direction. The reagent rack 15132 has a plurality of storage stations 151321 which can store reagent bottles of different specifications. When all the reagent bottles need to be removed from or placed in the reagent disk 1513 in one process, only the tray 15131 needs to be directly removed or placed in the axial direction. When some of the reagent bottles need to be removed or placed, if the reagent bottles to be operated are on one of the reagent racks 15132, only this reagent rack 15132 needs to be directly removed or placed in the axial direction, and if the reagent bottles to be operated are on different reagent racks 15132, the reagent bottles can be directly, separately removed from the reagent racks 15132 or the corresponding reagent bottles can be directly, separately placed in the empty space of the reagent racks 15132, or the reagent bottles can be removed or placed by removing or placing the reagent rack 15132 from/to the reagent disk 1513. This can make it possible to take into account the overall, partitioned and individual picking-up and placement of the reagent bottles during the picking-up and placement process of the reagent bottles, which is more flexible and further improves the overall efficiency of the automatic detection apparatus.

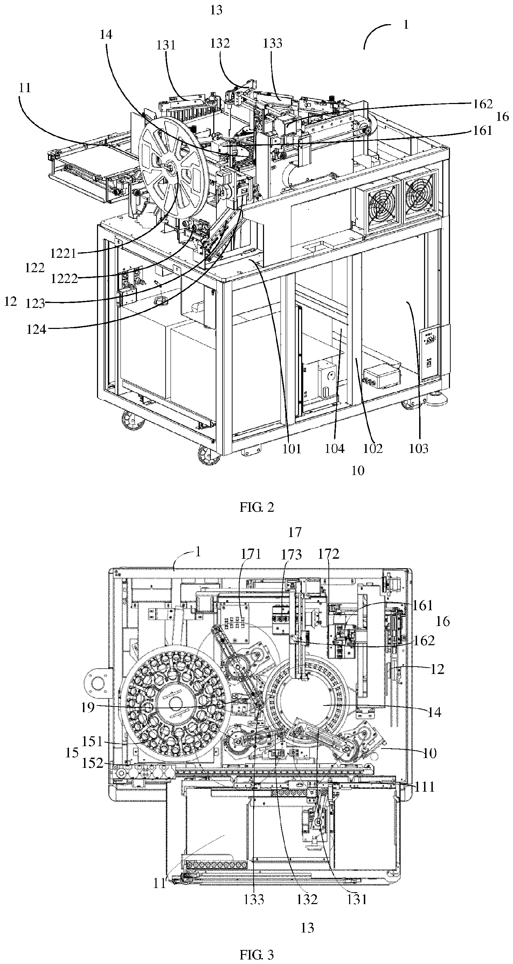

Referring to Figs. 1 and 3, further, the reagent storage module 15 further comprises a second reagent storage mechanism 152 for storing a reagent. The second reagent storage mechanism 152 is provided between the first reagent storage mechanism 151 and the sample transport module 11. The second reagent storage mechanism 152 is also used to store the reagent that reacts with the sample. The second reagent storage mechanism 152 can store the reagent at room temperature. The dispensing module 13 can aspirate in the reagent from the second reagent storage mechanism 152 and add the reagent to the sample incubation module 14. When the reagent that needs to be added to the sample is stored in the second reagent storage mechanism 152, the second reagent storage mechanism 152 can be moved to the position where the dispensing module 13 is located. In this case, the second reagent storage mechanism 152 is provided with a reagent extraction position corresponding to the blood analyzer. The integrated needle mechanism 132, after aspirating the reagent at the reagent extraction position, rotates to the dispensing position of the sample incubation module 14 to add the reagent to the reaction cup in placement station 14311. When the reagent that needs to be added to the sample is not available in the first reagent storage mechanism 151, the second reagent storage mechanism 152 can be controlled to move same outside the apparatus, a reagent bottle is temporarily added, the second reagent storage mechanism 152 is then moved to the reagent extraction position, and then filling and subsequent reagent suction operations are performed.

In addition, the second reagent storage mechanism 152 can also store an emergency sample, and the second reagent storage mechanism 152 can be moved to the dispensing module 13. The dispensing module 13 can aspirate in the emergency sample from the second reagent storage mechanism 152 and add the emergency sample to the sample incubation module 14. When there is an emergency sample that needs to be detected, the emergency sample is placed in the second reagent storage mechanism 152, the second reagent storage mechanism 152 drives the emergency sample to move to the dispensing module 13, and the sample is aspirated by the dispensing module 13 and transferred to a reaction cup in the sample incubation module 14, a queue-jumping detection is performed to shorten the detection time of the emergency sample, avoiding adjusting the positions of samples on the sample transport module 11 to transport the emergency sample by way of shutting down, and improving the operation efficiency.

The second reagent storage mechanism 152 comprises a second reagent drive assembly and a reagent transport rack. The second reagent drive assembly is arranged between the sample transport module 11 and the first reagent storage mechanism 151. The reagent transport rack is arranged on the second reagent drive mechanism. The reagent transport rack is provided with a plurality of reagent storage stations for placing reagents and a plurality of emergency sample storage stations for placing emergency samples, wherein the plurality of reagent storage stations are arranged in a row, and the plurality of emergency sample storage stations are also arranged in a row. The second reagent drive assembly can drive the movement of the reagent transport rack, such that the reagent transport rack can be moved to the dispensing module 13 for easy suction of the reagent and the sample.

Referring to Figs. 1 to 3, also in order to support the sample transport module 11, the cup feeding module 12, the dispensing module 13, the sample incubation module 14, the reagent storage module 15, the transfer module 16 and the sample detection module 17, the blood analyzer 1 further comprises a test housing 10. The test housing 10 has a placement cavity 104 and a testing platform 101 covering the placement cavity 104. The test housing 10 further comprises a mounting frame 102 and a plurality of cover plates 102 surrounding the mounting frame 102. Preferably, the mounting frame 102 is provided in the form of a rectangular parallelepiped, the plurality of cover plates 102 surrounds the periphery of the mounting frame 102, the testing platform 101 is mounted to the top of the mounting frame 102, and the placement cavity 104 is enclosed by the cover plate 102, the mounting frame 102 and the testing platform 101. The sample transport module 11, the dispensing module 13, the sample incubation module 14, the reagent storage module 15, the transfer module 16, and the sample detection module 17 are all located on the testing platform 101. The blood analyzer 1 further comprises a main control module 22 and a power supply module, wherein the power supply module is electrically connected to the main control module 22, the main control module 22 is electrically connected to the sample transport module 11, the dispensing module 13, the sample incubation module 14, the reagent storage module 15, the transfer module 16 and the sample detection module 17 respectively, and the main control module 22 and the power supply module are located in the placement cavity 104.

The testing platform 101 can have a supporting function to support the various moving components thereon, and the testing platform 101 can also have a partition function to divide the test housing 10 into a space above the testing platform 101 and the placement cavity 104 below the testing platform 101. The moving components of the blood analyzer 1 such as the sample transport module 11, the dispensing module 13, the sample incubation module 14, the reagent storage module 15, the transfer module 16 and the sample detection module 17 are located on the testing platform 101, whereas the non-moving components of the blood analyzer 1 such as the main control module 22 and the power supply module are arranged in the placement cavity 104. This can make it possible to organize the structure of the blood analyzer 1, avoiding the complicated structure and therefore restricting the movement process, and improving the working efficiency. The power supply module is used to energize and de-energize the entire blood analyzer 1 such that the blood analyzer 1 can operate normally. The control module is used to control the various components of the blood analyzer 1, such that each component can realize automatic operation, improving the working efficiency of the blood analyzer 1.

The main control module 22 is electrically connected to the sample transport module 11 to control the sample transport module 11 to automatically transfer the sample to be tested. The main control module 22 is electrically connected to the dispensing module 13. Specifically, the control module is electrically connected to the dispensing module 13 to control the dispensing module 13 to aspirate in the sample at the sample transport module 11 and adds the sample at the dispensing position of the sample incubation module 14, and to control the dispensing module 13 to aspirate in the reagent at the reagent aspirating position of the first reagent storage mechanism 151 and adds the reagent at the dispensing position of the sample incubation module 14. The main control module 22 is electrically connected to the sample incubation module 14 to control the rotation of the placement stations 14311 of the sample incubation module 14 to the cup picking-up and placement position, the dispensing position and the cup picking-up position, and it also possible to control the sample incubation module 14 to heat the sample for incubation. The main control module 22 is electrically connected to the first reagent storage mechanism 151 of the reagent storage module 15. The main control module 22 can control the rotation of the reagent at the corresponding position on the reagent storage module 15 to the reagent aspirating position, and can also control the first reagent storage mechanism 151 to refrigerate the reagent at a low temperature. The main control module 22 is electrically connected to the transfer module 16 to control the movement of the transfer module 16 between the cup feeding module 12, the cup picking-up and placement position and the cup picking-up position of the sample incubation module 14, and the sample detection module 17. The main control module 22 can also be electrically connected to sample detection module 17 to control the sample detection module 17 for detection. The main control module 22 being arranged in the placement cavity 104 of the test housing 10 can reduce the volume of each component and greatly reduce the space occupied by the testing platform 101, making the structure of the blood analyzer 1 compact, which is conducive to the miniaturization trend of the blood analyzer 1. In addition, the main control module 22 integrates the control over various components together, which facilitates maintenance operations and can also reduce the cost and failure rate of the machine.

In an embodiment of the present invention:
referring to Figs. 1 to 3, the dispensing module 13 comprises a puncture needle mechanism 131, an integrated needle mechanism 132 and a reagent needle mechanism 133. That is, the blood analyzer 1 of this embodiment is a three-needle mechanism, and the transfer of the sample and the reagent for the sample and then the detection of the sample are realized jointly by the puncture needle mechanism 131, the integrated needle mechanism 132 and the reagent needle mechanism 133. The puncture needle mechanism 131 is used to aspirate in and discharge the sample, the integrated needle mechanism 132 is used to aspirate in and discharge the sample or the reagent, and the reagent needle mechanism 133 is used to aspirate in and discharge the reagent.

The puncture needle mechanism 131 is located between the sample transport module 11 and the sample incubation module 14, and the puncture needle mechanism 131 can transfer the sample transported by the sample transport module 11 to the reaction cup in the sample incubation module 14. The puncture needle mechanism 131 aspirates in the sample from the sample transport module 11 and is then rotated to the sample incubation module 14, and adds the aspirated sample to the reaction cup in the sample incubation module 14. The integrated needle mechanism 132 is located between the sample incubation module 14, the reagent storage module 15 and the sample transport module 11, and the integrated needle mechanism 132 can transfer the sample from the first reagent storage mechanism 151 to the reaction cup in the sample incubation module 14, or the integrated needle mechanism 132 can transfer the sample from the sample transport module 11 to the reaction cup in the sample incubation module 14. The integrated needle mechanism 132 aspirates in the reagent from the reagent storage module 15 and is then rotated to the sample incubation module 14, and adds the aspirated reagent to the reaction cup with the sample in the sample incubation module 14. The integrated needle mechanism 132 can also aspirate in the sample from the sample transport module 11 and then be rotated to the sample incubation module 14, and add the aspirated sample to the reaction cup in the sample incubation module 14. The reagent needle mechanism 133 is located between the sample incubation module 14, the reagent storage module 15 and the sample detection module 17, and the reagent needle mechanism 133 can transfer the reagent from the first reagent storage mechanism 151 to the reaction cup in the sample incubation module 14 or to the reaction cup in the sample detection module 17. The reagent needle mechanism 133 aspirates in the reagent from the first reagent storage mechanism 151 and is then rotated to the sample incubation module 14, and adds the aspirated reagent to the reaction cup in the sample incubation module 14. Alternatively, the reagent needle mechanism 133 aspirates in the reagent from the first reagent storage mechanism 151 and is then rotated to the sample detection module 17, and adds the aspirated reagent to the reaction cup in the sample detection module 17.

Moreover, the dispensing positions comprises a sample adding position, a first reagent adding position and a second reagent adding position, and the reagent aspirating position comprises a first reagent aspirating position and a second reagent aspirating position. That is, the blood analyzer 1 is provided with a cup picking-up and placement position, a sample adding position, a first reagent adding position, a second reagent adding position and a cup picking-up position which surround the sample incubation module 14; and the blood analyzer 1 has a first reagent aspirating position and a second reagent aspirating position at the first reagent storage mechanism 151, and the blood analyzer 1 has a third reagent adding position at the sample detection module 17. At the sample adding position, the puncture needle mechanism 131 adds the aspirated sample to the reaction cup in the sample incubation module 14. At the second reagent adding position, the integrated needle mechanism 132 adds the aspirated sample or reagent to the reaction cup in the sample incubation module 14. At the first reagent adding position, the reagent needle mechanism 133 adds the aspirated reagent to the reaction cup in the sample incubation module 14, and at the third reagent adding position, adds the reagent to the reaction cup in the sample detection module 17. The integrated needle mechanism 132 aspirates in the reagent at the first reagent aspirating position, and the reagent needle mechanism 133 aspirates in the reagent at the second reagent aspirating position. When a certain reagent needs to be added to a sample, the rotation of the first reagent storage mechanism 151 causes the corresponding reagent to rotate to the first reagent aspirating position or the second reagent aspirating position, the reagent is aspirated by the integrated needle mechanism 132 at the first reagent aspirating position or is aspirated by the reagent needle mechanism 133 at the second reagent aspirating position, and the aspirated reagent is then added to the reaction cup in the sample incubation module 14.

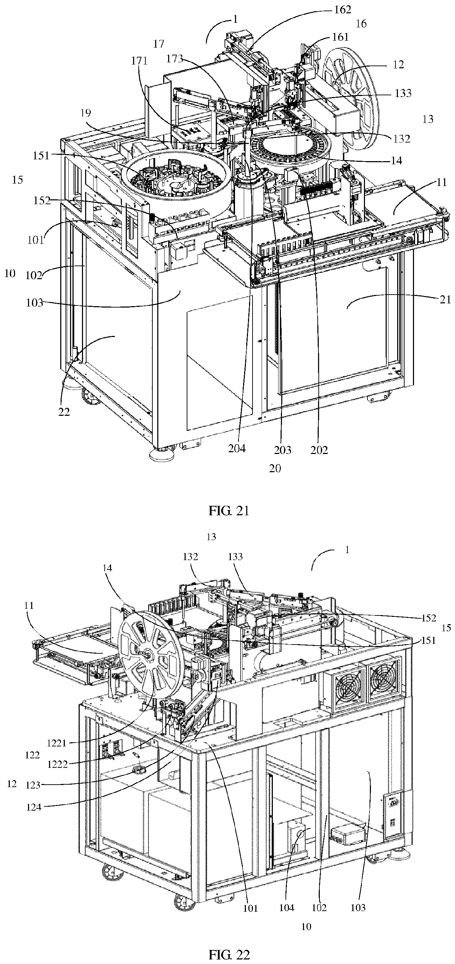

In another embodiment of the invention:
referring to Figs. 21 to 23, the dispensing module 13 comprises an integrated needle mechanism 132 and a reagent needle mechanism 133. That is, the blood analyzer 1 of this embodiment is a two-needle mechanism, and the transfer of the sample and the reagent for the sample and then the detection of the sample are realized jointly by the integrated needle mechanism 132 and the reagent needle mechanism 133. The integrated needle mechanism 132 is used to aspirate in and discharge the sample or the reagent, and the reagent needle mechanism 133 is used to aspirate in and discharge the reagent.

The integrated needle mechanism 132 is located between the sample incubation module 14, the reagent storage module 15 and the sample transport module 11, the integrated needle mechanism 132 can transfer the sample from the sample transport module 11 to the reaction cup in the sample incubation module 14, and the integrated needle mechanism 132 can also transfer the reagent from the first reagent storage mechanism 151 to the reaction cup in the sample incubation module 14. The integrated needle mechanism 132 aspirates in the sample from the sample transport module 11 and is then rotated to the sample incubation module 14, and adds the aspirated sample to the reaction cup in the sample incubation module 14. The integrated needle mechanism 132 can also aspirate in the reagent from the reagent storage module 15 and then be rotated to the sample incubation module 14, and add the aspirated reagent to the reaction cup with the sample in the sample incubation module 14. The reagent needle mechanism 133 is located between the sample incubation module 14, the reagent storage module 15 and the sample detection module 17, and the reagent needle mechanism 133 can transfer the reagent from the first reagent storage mechanism 151 to the sample incubation module 14 or to the sample detection module 17. The reagent needle mechanism 133 aspirates in the reagent from the first reagent storage mechanism 151 and is then rotated to the sample incubation module 14, and adds the aspirated reagent to the reaction cup in the sample incubation module 14. Alternatively, the reagent needle mechanism 133 aspirates in the reagent from the first reagent storage mechanism 151 and is then rotated to the sample detection module 17, and adds the aspirated reagent to the reaction cup in the sample detection module 17.

Moreover, the dispensing position comprises a sample adding position and a first reagent adding position, and the reagent aspirating position comprises a first reagent aspirating position and a second reagent aspirating position. That is, the blood analyzer 1 is provided with a cup picking-up and placement position, a sample adding position, a first reagent adding position and a cup picking-up position which surround the sample incubation module 14. The blood analyzer 1 has a first reagent aspirating position and a second reagent aspirating position at the first reagent storage mechanism 151, and the blood analyzer 1 has a third reagent adding position at the sample detection module 17. At the sample adding position, the integrated needle mechanism 132 can implement both the sample adding operation and the reagent adding operation. At the sample adding position, the integrated needle mechanism 132 adds the aspirated sample to the reaction cup in the sample incubation module 14, and adds the aspirated reagent to the reaction cup in the sample incubation module 14. At the first reagent adding position, the reagent needle mechanism 133 adds the aspirated reagent to the reaction cup in the sample incubation module 14, and at the third reagent adding position, adds the reagent to the reaction cup in the sample detection module 17. The integrated needle mechanism 132 aspirates in the reagent at the first reagent aspirating position, and the reagent needle mechanism 133 aspirates in the reagent at the second reagent aspirating position. When a certain reagent needs to be added to a sample, the rotation of the first reagent storage mechanism 151 causes the corresponding reagent to rotate to the first reagent aspirating position or the second reagent aspirating position, the reagent is aspirated by the integrated needle mechanism 132 at the first reagent aspirating position or is aspirated by the reagent needle mechanism 133 at the second reagent aspirating position, and the aspirated reagent is then added to the reaction cup in the sample incubation mechanism 143.

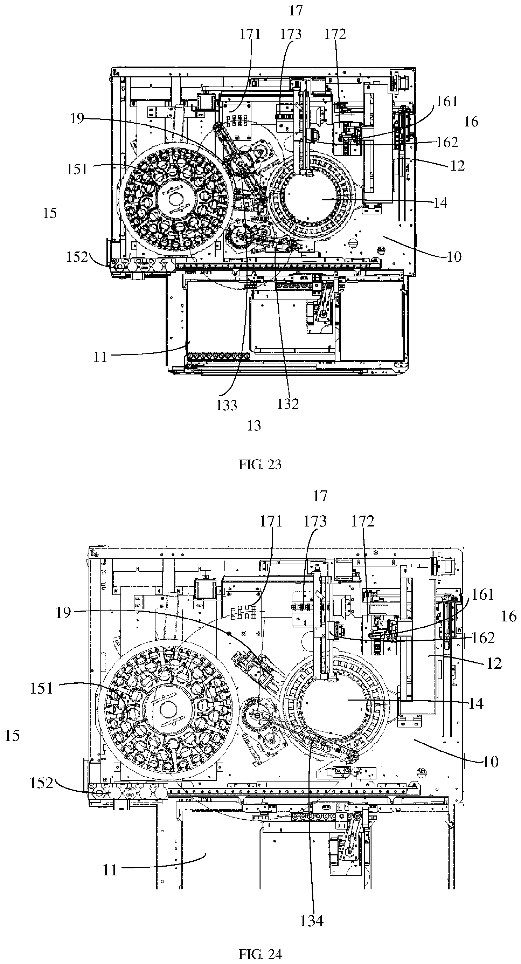

In yet another embodiment of the present invention:
referring to Fig. 24, the dispensing module 13 comprises a dispensing needle mechanism 134. That is, the blood analyzer 1 of this embodiment is a one-needle mechanism, and the sample and the reagent are transferred by the dispensing needle mechanism 134, to realize the detection of the sample. The dispensing needle mechanism 134 is used to aspirate in and discharge the sample or the reagent.

The dispensing needle mechanism 13 can transfer the sample from the sample transport module 11 to the reaction cup in the sample incubation module 14. The dispensing module 13 can further transfer the reagent from the first reagent storage mechanism 151 to the reaction cup in the sample incubation module 14 or to the reaction cup in the sample detection module 17. It can be understood that the arrangement position of the dispensing needle mechanism 134 needs to make it possible to move the dispensing needle mechanism 134 to the sample transport module 11, the reagent storage module 15, the sample incubation module 14 and the sample detection module 17, in order to facilitate the transfer of the sample and the reagent. Preferably, in this embodiment, the dispensing needle mechanism 134 is located between the sample transport module 11 and the first reagent storage mechanism 151, and the dispensing needle mechanism 134 can aspirate in the sample from the sample transport module 11, aspirate in the reagent at the first reagent storage mechanism 151, can add the sample and the reagent at the sample incubation module 14, and can add the reagent at the sample detection module 17.

Moreover, the dispensing position comprises a filling position, and the blood analyzer 1 is provided with a cup picking-up and placement position, the filling position and a cup picking-up position which surround the sample incubation module 14. The blood analyzer 1 has a reagent aspirating position at the first reagent storage mechanism 151, and the blood analyzer 1 has a third reagent adding position at the sample detection module 17. It can be understood that the positions of the dispensing needle mechanism 134 at which the sample and the reagent are added to the sample incubation module 14 are at the same position, that is, the dispensing needle mechanism 134 can implement both the operation of adding the sample and the operation of adding the reagent on the sample adding position, which can facilitate the control over the dispensing needle mechanism 134. At the filling position, the dispensing needle mechanism 134 adds the aspirated sample to the reaction cup in the sample incubation module 14 and adds the aspirated reagent to the reaction cup in the sample incubation module 14, and at the third reagent adding position, adds a reagent to the reaction cup in the sample detection module 17. When a certain reagent needs to be added to a sample, the rotation of the first reagent storage mechanism 151 causes the corresponding reagent to rotate to the reagent aspirating position, the reagent is aspirated by the dispensing needle mechanism 134 at the reagent aspirating position, and the aspirated reagent is then added to the reaction cup in the sample incubation mechanism 143, or to the reaction cup in the sample detection module 17.

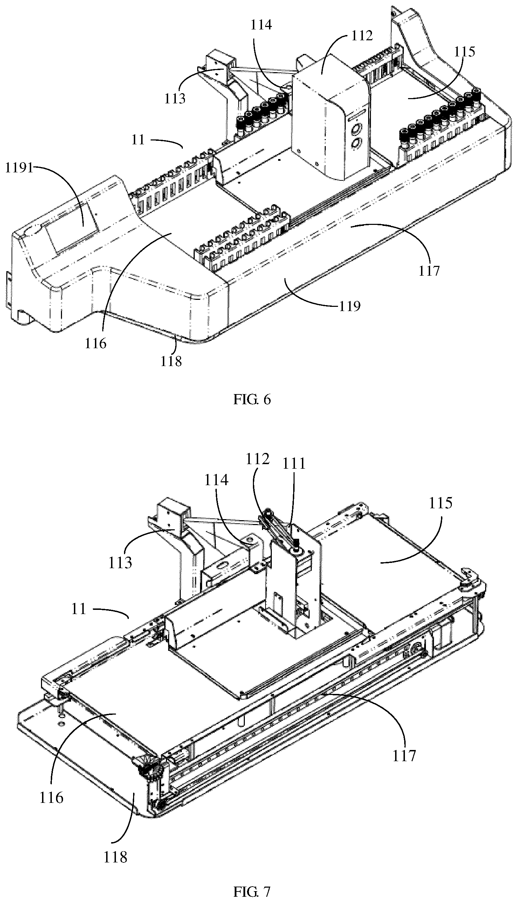

Referring to Figs. 1, 6 and 7, as a possible implementation, the sample transport module 11 comprises an automatic sample feeding mechanism, which is used to automatically transport a plurality of containers which are arranged on a sample rack, distributed in a row, and contain samples. The automatic sample feeding mechanism is provided with a puncture position and a non-puncture position, and the non-puncture position and the puncture position are arranged in sequence. It should be noted that when the dispensing module 13 aspirates in the sample by the puncture needle mechanism 131 and the integrated needle mechanism 132, the puncture needle mechanism 131 can aspirate in the sample from the container at the puncture position; and the integrated needle mechanism 132 can aspirate in the sample from the container at the non-puncture position. When the dispensing module 13 aspirates in the sample by the integrated needle mechanism 132, the integrated needle mechanism 132 can aspirate in the sample from the container at the non-puncture position. When the dispensing module 13 comprises a dispensing needle mechanism 134, the dispensing needle mechanism 134 can aspirate in the sample from the container at the non-puncture position. The distance between the non-puncture position and the puncture position is at least one multiple of the distance between two adjacent containers.

The main control module 22 comprises a main controller and a scanning mechanism 113, the scanning mechanism 113 is used to scan a detection code on the container which contains the sample and is transported by the sample transport module 11, and the main controller is electrically connected to the scanning mechanism 113, and obtains detection information of a test item of the sample in the container. Preferably, the scanning mechanism 113 is located at the sample incubation module 14 and is arranged to face toward the sample transport module 11. Of course, in other implementations of the present invention, the scanning mechanism 113 may also be located at another position, as long as it is ensured that the scanning mechanism 113 can detect the detection code on the container containing the sample. The scanning mechanism 113 is used in cooperation with the automatic sample feeding mechanism of the sample transport device.

Specifically, the automatic sample feeding mechanism comprises a detection assembly 111, a rotation assembly 112, and a sampling assembly 114 which are arranged in sequence. The detection assembly 111 is used to detect whether there is a container. The rotation assembly 112 is used to rotate the container to align the detection code on the container with the scanning mechanism 113 to facilitate scanning by the scanning mechanism 113. The puncture needle mechanism 131 can aspirate in the sample at the sampling assembly 114. The automatic sample feeding mechanism further comprises a sample transport floor 118, a loading assembly 115, a transport assembly (not shown), an unloading assembly 116 and a reversing assembly 117 provided between the loading assembly 115 and the unloading assembly 116. The loading assembly 115 is used to place a plurality of containers which are distributed in a row and contain samples, that is, a plurality of test tube racks are placed on the loading assembly 115, and multiple rows of test tubes containing samples to be tested are placed on each test tube rack. The unloading assembly 116 is used to recover the sample that has been detected. The transport assembly is used to realize the automatic transport of the test tube rack, such that the test tube rack runs between the loading assembly 115, the detection assembly 111, the rotation assembly 112, the sampling assembly 114, and the unloading assembly 116. The reversing assembly 117 is used to return the test tube containing the sample from the unloading assembly 116 to the loading assembly 115. The sample transport floor 118 has a supporting function. The loading assembly 115, the transport assembly, the detection assembly 111, the rotation assembly 112, the sampling assembly 114, the unloading assembly 116, and the reversing assembly 117 are all arranged on the sample transport floor 118. The automatic sample feeding mechanism further comprises a sample transport roof 119. The sample transport roof 119 covers the sample transport floor 118, such that each transmission component of the sample transport module 11 is hidden between the sample transport floor 118 and the sample transport roof 119, so as to avoid the operator's access, which affects the operation safety. A card swiping component 1191 is provided on the sample transport roof 119. The card swiping component 1191 can control the sample transport module 11 to start the automatic sample transport operation. Of course, in other implementations of the present invention, the detection assembly 111 and the scanning mechanism 113 can also be one assembly, namely, a detection and scanning assembly, which can detect whether there is a test tube and scan the detection code of the test tube.

Optionally, the loading assembly 115 comprises a loading drive motor and a loading push component. The loading drive motor is connected to the loading push component, such that the loading push component pushes the test tube rack to the transport assembly. The transport assembly comprises a transport drive motor and a transport transmission component. The transport drive motor is connected to the transport transmission component. The transport drive motor can drive the transport transmission component to perform a stepping motion, such that the distance of one step of movement of the test tube rack is the distance between two test tubes. The detection assembly 111 is used to detect the container, i.e. the test tube. The detection assembly 111 can detect whether there is a test tube in the test tube rack, and can also detect whether the test tube has a test tube cap. If there is a test tube cap, the reagent is aspirated by the puncture needle mechanism 131. If there is no test tube cap, the reagent is aspirated by the integrated needle mechanism 132 or the dispensing needle mechanism 134. The rotation assembly 112 is arranged corresponding to the scanning mechanism 113 such that the detection code on the test tube can be aligned with the scanning mechanism 113, facilitating obtaining the information of the detected item of the sample. When the detection code on the test tube does not correspond to the scanning mechanism 113, the rotation assembly 112 can rotate the test tube such that the detection code of the test tube faces toward the scanning mechanism 113, facilitating the entry of the information of the sample. The rotation assembly 112 comprises a test tube rotation drive motor and a test tube rotation transmission component. The test tube rotation drive motor is connected to the test tube rotation transmission component. The rotation of the test tube is driven by the test tube transmission component. The sampling assembly 114 has a sampling plate with a sampling hole, and the test tube moves to the sampling plate. The sampling hole is directly opposite the test tube, and the puncture needle mechanism 131 can extend into the test tube to aspirate in the sample. The unloading assembly 116 comprises an unloading drive motor and an unloading push component. The unloading drive motor is connected to the unloading push component, such that the unloading push component pushes the test tube rack out of the transport assembly. When the sample needs to be re-detected, the unloading push component can also push the test tube rack to the reversing assembly 117. The reversing assembly 117 comprises a reversing drive motor and a reversing push component. The reversing drive motor is connected to the reversing push component. The reversing push component pushes the test tube rack from the unloading assembly 116 back to the loading assembly 115 for re-detection. The transport assembly can realize the automatic transfer of the test tube rack between the detection assembly 111, the rotation assembly 112 and the sampling assembly 114, thereby achieving the automatic transport of the sample, and improving the efficiency.

When the automatic sample feeding mechanism transports the sample, a plurality of test tube racks are arranged in a row in the loading assembly 115, and a plurality of test tubes containing samples are arranged in a row on each of test tube racks. The loading assembly 115 can push the test tube rack to the transport assembly. Subsequently, the transport assembly moves, such that the test tube on the test tube rack is moved to the detection assembly 111 for detection, and scanned by the scanning mechanism 113 to extract the detection information of the item of the sample. Subsequently, the transport assembly moves the test tube to the sampling assembly 114. The dispensing module 13 aspirates in the sample from the test tube at the sampling assembly 114. The transport assembly then drives the test tube to move to the unloading assembly 116, so as to recover the detected test tube. When the sample needs to be re-detected, the reversing assembly 117 can return the sample that has been detected in the unloading assembly 116 back to the loading assembly 115 for re-detection, which greatly improves the detection efficiency of the re-detection of the sample, saves on time, and greatly improves the convenience of operation.

Of course, in other implementations of the present invention, a scanning position of the scanning mechanism 113 may also be located on the left side of the rotation assembly 112, that is, the test tube first passes through the rotation assembly 112 and then the scanning mechanism 113, and the rotation assembly 112 first rotates the detection code of the test tube to the desired position, and the transport assembly then drives the test tube to move to the scanning mechanism 113 for scanning. It should be noted that the distance of each movement of the transport assembly is the distance between two test tubes, which facilitates the corresponding movement of the test tube to the corresponding operation assembly. In addition, the rotation assembly 112 can only rotate the test tube with a test tube cap, such that the detection code is aligned with the position of the scanning mechanism 113. When there is no test tube cap on the test tube, the rotation assembly 112 cannot rotate the test tube without the test tube cap. In this case, in order to ensure the normal operation of the apparatus, the detection code on the test tube needs to face toward the scanning mechanism 113 without rotation, or the detection code is manually input to enable the sample to be detected normally.

Further, the detection assembly 111 is correspondingly provided with a detection position, the rotation assembly 112 is correspondingly provided with a rotation position, the scanning mechanism 113 is provided with a scanning position on the automatic sample feeding mechanism, and the sampling assembly 114 is correspondingly provided with a puncture position. The detection assembly 111 detects whether there is a test tube on the detection position, the rotation assembly 112 rotates the test tube on the rotation position, the scanning mechanism 113 scans the test tube on the scanning position, and the puncture needle mechanism 131 aspirates in the sample on the puncture sampling position of the sampling assembly 114. The distance between the detection position and the rotation position is at least one multiple of the distance between two adjacent containers, and the distance between the rotation position and the puncture position is at least one multiple of the distance between two adjacent containers. This can facilitate enabling the test tube to be just moved to the detection position, the rotation position, and the puncture position for the corresponding operations. Preferably, the distance between the puncture position and the rotation position is at least twice the distance between two test tubes. This makes it possible that when one of the test tubes to be detected on the detection position, and one of the remaining test tubes is just located on the rotation position for rotation operation, improving the efficiency. When the test tube does not have a test tube cap, the rotation assembly 112 cannot rotate the test tube. In this case, there is a requirement for the test tube to be placed on the test tube rack, such that the detection code on the test tube faces toward the scanning mechanism 113, facilitating the scanning mechanism 113 to obtain the detection information of the item of the sample. Of course, the test tube without the test tube cap can also be placed on the reagent transport rack of the second reagent storage mechanism 152 for transport. In this case, the basic information of the sample can be obtained by means of manual entry.

Still further, the automatic sample feeding mechanism is provided with a non-puncture position, a puncture position, a rotation position, and a detection position which are arranged in sequence. The integrated needle mechanism 132 and the dispensing needle mechanism 134 can such in the sample from the container at the non-puncture position. When the integrated needle mechanism 132 has a sufficient length to reach the non-puncture position of the transport assembly, the sample can be aspirated by the integrated needle mechanism 132 from the test tube without the test tube cap on the non-puncture position. The transport assembly transports the test tube to the non-puncture position, and the integrated needle mechanism 132 aspirates in the sample from the test tube at the non-puncture position. The distance between the non-puncture position and the puncture position is at least one multiple of the distance between two adjacent containers. This can facilitate enabling the test tube to be just moved to the detection position, the rotation position, and the non-puncture position for the corresponding operations. Preferably, the distance between the non-puncture position and the rotation position is at least eight times the distance between two test tubes. This makes it possible that when one of the test tubes to be detected on the rotation position, and one of the remaining test tubes is just located on the non-puncture position for sampling, improving the efficiency. Of course, in other implementations of the present invention, the distance between the non-puncture position and the rotation position can also be the distance relationship between the other two test tubes, as long as it is ensured that when one of the test tubes is detected on the rotation position, one of the remaining test tubes is just located at the non-puncture position for sampling.

It should be noted that the sample transport module 11 in the present invention has a general structure, and may be used in the blood analyzer 1 having the three-needle structure in which case the sample is aspirated from the test tube of this transport module by the puncture needle mechanism 131 and the integrated needle mechanism 132; may be used in the blood analyzer 1 which is a two-needle mechanism in which case the integrated needle mechanism 132 is used to aspirate in the sample from the test tube of this transport module; or may be used in the blood analyzer 1 having the one-needle structure in which case the dispensing needle mechanism 134 is used to aspirate in the sample from the test tube of the sample transport module 11.

When the blood analyzer 1 of the present invention uses the three-needle structure, if there are test tubes with caps and test tubes without caps among the test tubes transported by the sample transport module 11, the detection assembly 111 can detect whether there is a test tube on the test tube rack, and can also detect whether there is a test tube cap on the test tube. In this way, the transport assembly can transport the test tube with the test tube cap to the puncture position, and the sample is aspirated from the test tube by the puncture needle mechanism 131; and the transport assembly transports the test tube without the test tube cap to the non-puncture position, and the sample is aspirated from the test tube by the integrated needle mechanism 132. Of course, in other implementations of the present invention, the transport assembly can also transport the test tube without the test tube cap to the puncture position, and the sample is aspirated from the test tube by the puncture needle mechanism 131. Preferably, the sampling assembly 114 comprises a baffle, with one end of the baffle being fixed onto the sample transport floor 118, and the other end thereof being arranged horizontally. The horizontal end of the baffle is provided with a through hole through which the puncture needle mechanism 131 extends into the test tube and aspirates in the sample. After the suction is completed, the baffle will drive the test tube to rise together upon being pulled out of the test tube with the cap. The horizontal end of the baffle can limit the position of the test tube to ensure reliable suction operation. In this embodiment, the blood analyzer 1 can only aspirate in the sample from the sample transport module 11 by the puncture needle mechanism 131. The blood analyzer 1 can also aspirate in the sample from the sample transport module 11 by the puncture needle mechanism 131 together with the integrated needle mechanism 132 to improve the operation efficiency. In this case, it is necessary to avoid the problem of the puncture needle mechanism 131 being interfered with the integrated needle mechanism 132.

When the blood analyzer 1 of the present invention uses the two-needle structure, the blood analyzer 1 cannot aspirate in the sample from the test tube with a test tube cap, but can only aspirate in the sample from the test tube without the test tube cap, and whether there is a test tube cap on the test tube rack is detected by the detection assembly 111. In this way, the transport assembly transports the test tube without the test tube cap to the non-puncture position, the sample is aspirated from the test tube without the test tube cap by the integrated needle mechanism 132 at the non-puncture position and is transferred to the sample incubation module 14. Moreover, the detection code on each test tube without the test tube cap has a unified orientation, facilitating the scanning mechanism 113 to obtain the information of a test item that needs to be performed on the sample.

When the blood analyzer 1 of the present invention uses the one-needle structure, the blood analyzer 1 cannot aspirate in the sample from the test tube with a test tube cap, but can only aspirate in the sample from the test tube without the test tube cap, and whether there is a test tube cap on the test tube rack is detected by the detection assembly 111. In this way, the transport assembly transports the test tube without the test tube cap to the non-puncture position, the sample is aspirated from the test tube without the test tube cap by the dispensing needle mechanism 134 at the non-puncture position and is transferred to the sample incubation module 14. Moreover, the detection code on each test tube without the test tube cap has a unified orientation, facilitating the scanning mechanism 113 to obtain the information of a test item that needs to be performed on the sample.

Of course, the sample transport module 11 of the present invention can also be connected to other in-vitro diagnostic products, the sample transport module 11 can be removed, and the blood analyzer 1 and the other in-vitro diagnostic apparatuses are connected via a sample feeder to form an inspection line. The dispensing module 13 can aspirate in the sample from the sample feeder, so that the sample transport module 11 of the present invention has a wide application range.

Referring to Figs. 1 to 3, as a possible implementation, the transfer module 16 comprises a first transfer mechanism 161 and a second transfer mechanism 162. The first transfer mechanism 161 is movably arranged above the cup feeding module 12 and the sample incubation module 14. The first transfer mechanism 161 is used to transport the reaction cup transported by the cup feeding module 12 to the sample incubation module 14. The second transfer mechanism 162 is movably arranged above the sample incubation module 14 and the sample detection module 17. The second transfer mechanism 162 is used to transfer the reaction cup, in which the sample and the reagent have been added, from the sample incubation module 14 to the sample detection module 17. In order to improve the transfer efficiency of the reaction cup, the blood analyzer 1 of the present invention uses two transfer components, namely the first transfer mechanism 161 and the second transfer mechanism 162, wherein the empty reaction cup is transferred by the first transfer mechanism 161, and the reaction cup in which the sample and the reagent have been added is transferred by the second transfer mechanism 162, such that each of the first transfer mechanism 161 and the second transfer mechanism 162 has the corresponding function thereof, improving the working efficiency of the blood analyzer 1.

Moreover, the sample detection module 17 of the blood analyzer 1 of the present invention can also perform different forms of detection on the sample to obtain different performance parameters. The sample detection module 17 can complete data acquisition and result output of various parameter measurements. The sample detection module 17 in this embodiment can support the measurement of the sample in a coagulation method, an immunoturbidimetric method and a chromogenic substrate method. Specifically, the sample detection module 17 comprises a magnetic bead detection mechanism 171 and an optical detection mechanism. The magnetic bead detection mechanism 171 performs a magnetic bead detection on the sample, and the optical detection mechanism performs an optical detection on the sample. The magnetic bead detection mechanism 171 is arranged side by side with the optical detection mechanism, which can facilitate the transfer of the reaction cup. It should be noted that the magnetic bead detection mechanism 171 and the optical detection mechanism can add different reagents to the sample for detection, so that the application range is wide. In an implementation of the present invention, the first transfer module 16 transfers the empty reaction cup transported by the cup feeding module 12 to the placement station 14311 of the sample incubation module 14. The second transfer mechanism 162 can transfer the reaction cup, in which the sample and the reagent have been added, from the sample incubation module 14 to the optical detection mechanism or the magnetic bead detection mechanism 171. That is, the reaction cup is transported and transferred by the first transfer mechanism 161 and the second transfer mechanism 162. In another implementation of the present invention, the transfer module 16 can transfer the reaction cup, in which the sample and the reagent have been added, from the sample incubation module 14 to the optical detection mechanism or the magnetic bead detection mechanism 171, that is, the transfer module 16 can also transport and transfer the reaction cup by using only one transfer mechanism.

At the same time, there is a need to prevent the first transfer mechanism 161 from being idle when not transferring the empty reaction cup, which cannot allow the first transfer mechanism 161 to be fully utilized, as a result, the blood analyzer 1 cannot perform better functions. To this end, the blood analyzer 1 of the present invention is provided with two optical detection mechanisms, such that while the second transfer mechanism 162 still transfers the reaction cup, in which the sample and the reagent have been added, to the other optical detection mechanism and the magnetic bead detection mechanism 171, the first transfer mechanism 161 can, when idle, transfer a reaction cup, in which the sample and the reagent have been added, to one of the optical detection mechanisms, making the full use of the first transfer mechanism 161 and the second transfer mechanism 162 to improve the utilization, thereby improving the working efficiency of the blood analyzer 1. Moreover, the two optical detection mechanisms are separately arranged, which can also avoid the situation where a large optical detection mechanism cannot be placed, and increase the area of optical detection to make rational use of the space on the testing platform 101. Optionally, the two optical detection mechanisms can also be used to provide different optical detection stations.

Specifically, the two optical detection mechanism are respectively a first optical detection mechanism 172 and a second optical detection mechanism 173, and the magnetic bead detection mechanism 171, the second optical detection mechanism 173 and the first optical detection mechanism 172 are arranged in sequence. The first transfer mechanism 161 is movably arranged above the cup feeding module 12, the sample incubation module 14 and the first optical detection mechanism 172. The first transfer mechanism 161 is used to transport the reaction cup transported by the cup feeding module 12 to the sample incubation module 14, and transfer the reaction cup, in which the sample and the reagent have been added, from the sample incubation module 14 to the first optical detection mechanism 172. The second transfer mechanism 162 is movably arranged above the sample incubation module 14, the second optical detection mechanism 173 and the magnetic bead detection mechanism 171. The second transfer mechanism 162 is used to transfer the reaction cup, in which the sample and the reagent have been added, from the sample incubation module 14 to the second optical detection mechanism 173 or the magnetic bead detection mechanism 171.

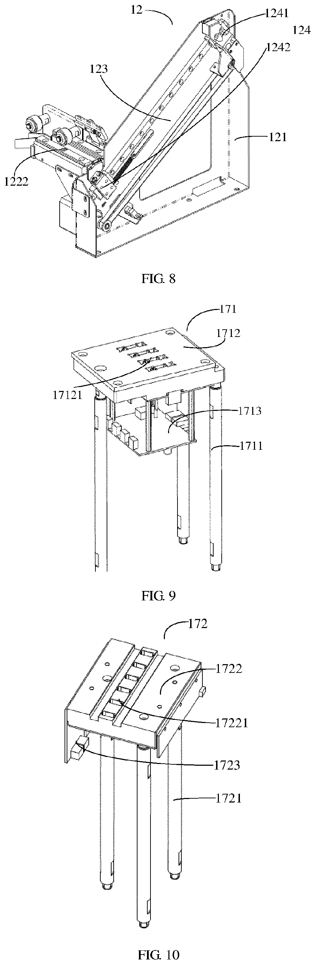

Referring to Figs. 1 and 9, the magnetic bead detection mechanism 171 comprises a magnetic bead support frame 1711, a magnetic bead detection component, a magnetic bead detection seat 1712, and a magnetic bead control board 1713. The magnetic bead detection seat 1712 is arranged at the top of the magnetic bead support frame 1711, and the magnetic bead detection seat 1712 is supported on the testing platform 101 by the magnetic bead support frame 1711. The magnetic bead detection component is arranged in the magnetic bead detection seat 1712. The magnetic bead detection seat 1712 is provided with a plurality of magnetic bead detection stations 17121. The second transfer mechanism 162 can transfer the reaction cup, in which the sample and the reagent have been added, from the sample incubation module 14 to the magnetic bead detection station 17121 on the magnetic bead detection seat 1712, and a magnetic bead detection can then be performed on the sample by the magnetic bead detection component in the magnetic bead detection seat 1712, to obtain the parameter of the sample. The magnetic bead control board 1713 is arranged below the magnetic bead detection seat 1712. The magnetic bead control board 1713 is electrically connected to the magnetic bead detection component to control the magnetic bead detection mechanism 171 for magnetic bead detection.

Moreover, the plurality of magnetic bead detection stations 17121 are arranged in an arc shape at the magnetic bead detection seat 1712, and the plurality of detection stations arranged in an arc shape correspond to a rotation trajectory of the reagent needle mechanism 133 or the dispensing needle mechanism 134. The blood analyzer 1 is provided with a third reagent adding position corresponding to the magnetic bead detection mechanism 171. The reagent needle mechanism 133 or the dispensing needle mechanism 134 aspirates in the reagent at the second reagent aspirating position of the first reagent storage mechanism 151 and is then rotated to the third reagent adding position on the magnetic bead detection mechanism 171 to add the reagent. The plurality of magnetic bead detection stations 17121 arranged in an arc shape can facilitate rotating the reagent needle mechanism 133 to the corresponding detection station to add the reagent. The third reagent adding position corresponds to the plurality of detection stations, and upon the reagent needle mechanism 133 or the dispensing needle mechanism 134 is rotated to above the corresponding detection station, the reagent needle mechanism 133 or the dispensing needle mechanism 134 can add the reagent to the reaction cup of the detection station. Optionally, the magnetic bead detection seat 1712 is provided with four magnetic bead detection stations 17121. The four magnetic bead detection stations 17121 are arranged on the arc-shaped movement trajectory of the reagent needle mechanism 133 or the dispensing needle mechanism 134.

Referring to Figs. 1 and 10, the first optical detection mechanism 172 comprises a first optical support frame 1721, a first optical detection component, a first optical detection seat 1722 and a first optical receiving board 1723. The first optical detection seat 1722 is arranged at the top of the first optical support frame 1721, and the first optical detection seat 1722 is supported on the testing platform 101 by the first optical support frame 1721. The first optical detection component is arranged in the first optical detection seat 1722. The first optical detection seat 1722 is provided with a plurality of first optical detection stations 17221. The first transfer mechanism 161 transfers the reaction cup, in which the sample and the reagent have been added, from the cup picking-up and placement position of the sample incubation mechanism 143 to the first optical detection station 17221 on the first optical detection seat 1722, and an optical detection can then be performed on the sample by the first optical detection component in the first optical detection seat 1722, to obtain the parameter of the sample. The first optical receiving board 1723 is arranged on a side of the first optical detection seat 1722. The first optical receiving board 1723 is electrically connected to the first optical detection component to control the first optical detection mechanism 172 for optical detection. Moreover, the first optical detection mechanism 172 further comprises a first optical emission board. The first optical emission board is arranged on the first optical detection seat 1722, and is arranged opposite the first optical receiving board 1723.

Moreover, the plurality of first optical detection stations 17221 are arranged in a straight line, which facilitates the first transfer mechanism 161 to transfer reaction cups to the first optical detection stations 17221 on the first optical detection seat 1722. Optionally, there are six first optical detection stations 17221, and the six first optical detection stations 17221 are all immunoturbidimetric detection stations, so that shading needs to be considered to ensure that the detection structure is accurate. It should be noted that the structure of the second optical detection mechanism 173 is exactly the same as the structure of the first optical detection mechanism 172, except that the second optical detection stations of the second optical detection mechanism 173 comprise a chromogenic substrate method reaction detection station and an immunoturbidimetric detection station, and shading needs to be considered for the immunoturbidimetric detection station. Of course, in other implementations of the present invention, the number of the first optical detection stations 17221 on the first optical detection mechanism and the number of the second optical detection stations on the second optical detection mechanism 173 are exactly the same, and the layout is also the same. The chromogenic substrate method reaction detection stations and the immunoturbidimetric detection stations may be arranged accordingly, that is, one or some of the first optical detection station 17221 are chromogenic substrate reaction detection stations, and the remaining first optical detection stations 17221 are immunoturbidimetric detection stations. Of course, it is also possible that the first optical detection stations 17221 are all chromogenic substrate method reaction detection stations or immunoturbidimetric detection stations. Preferably, the first optical detection stations 17221 comprises five immunoturbidimetric detection stations and one chromogenic substrate detection station. The arrangement of the second optical detection stations is exactly the same as that of the first optical detection stations 17221.

As a possible implementation, the main control module 22 is electrically connected to the transfer module 16, and the main control module 22 controls the transfer module 16 according to an instruction, so as to transfer the reaction cup from the sample incubation module 14 to the magnetic bead detection mechanism 171 or the optical detection mechanism. The main control module 22 can obtain the detection information of the items that need to be performed on the sample in the reaction cup. Subsequently, the main control module 22 controls the transfer module 16, so as to transfer the empty reaction cup transported by the cup feeding module 12 to the placement station 14311 of the sample incubation module 14. The main control module 22 then controls the dispensing module 13, so as to transfer the corresponding sample and various reagents required for the detection of the items of the sample to the corresponding reaction cups on sample incubation module 14. Subsequently, the main control module 22 controls the transfer module 16 according to the detection information of the item of the sample, so as to transfer the reaction cup, in which the sample and the reagent have been added, to the magnetic bead detection mechanism 171 or the optical detection mechanism, that is, when a magnetic bead detection is performed on the sample, the main control module 22 controls the transfer module 16 according to a control instruction, so as to transfer the reaction cup, in which the sample and the reagent have been added, to the magnetic bead detection mechanism 171; and when an optical detection is performed on the sample, the main control module 22 controls the transfer module 16 according to a control instruction, so as to transfer the reaction cup, in which the sample and the reagent have been added, to the optical detection mechanism. Further, the main control module 22 can transfer the sample for the optical detection to the first optical detection mechanism 172 or the second optical detection mechanism 173 according to the actual requirements of use. In this way, when one of the optical detection mechanisms is filled with reaction cups, the other can continue to receive the reaction cups, so that the number of the detected samples can be increased, improving the detection efficiency.

Optionally, the main controller of main control module 22 is further electrically connected to the dispensing module 13, the sample incubation module 14, the first reagent storage mechanism 151, the sample detection module 17 and the transfer module 16. The scanning mechanism 113 can transmit the obtained detection information of the test item of the sample to the main controller. The main controller controls the dispensing module 13 according to the detection information so as to transfer the sample transported by the sample transport module 11 to the reaction cup in the sample incubation module 14, and controls the dispensing module 13 so as to transfer the corresponding reagent from the first reagent storage mechanism 151 to the reaction cup in the sample incubation module 14. The main controller then controls the movement of the transfer module 16 according to the detection information, such that the transfer mechanism transfers the reaction cup, in which the sample and the reagent have been added, from the sample incubation module 14 to the sample detection module 17. The blood analyzer 1 of the present invention obtains the detection information of the item of the sample by the scanning mechanism 113, and controls the transfer module 16, the dispensing module 13, the sample incubation module 14, the first reagent storage mechanism 151 and the sample detection module 17 by the main controller, such that each module supports the process required for the detection information of the item of the sample to achieve the detection of the sample.

Further, when the detection information of the sample is magnetic bead detection information, the main control module 22 controls the movement of the transfer module 16 so as to transfer the reaction cup to the magnetic bead detection mechanism 171 for magnetic bead detection. When the detection information of the sample is optical detection information, the main control module 22 controls the movement of the transfer module 16 so as to transfer the reaction cup to the optical detection mechanism for optical detection.

Moreover, the first optical detection mechanism and the second optical detection mechanism 173 are provided with an immunoturbidimetric detection station and a chromogenic substrate detection station. In order to further increase the diversity of detections of the sample, when the optical detection information of the sample is immunoturbidimetric detection information, the main control module 22 controls the movement of the transfer module 16 so as to transfer the reaction cup to the immunoturbidimetric detection station on the optical detection mechanism for optical detection. When the optical detection information of the sample is chromogenic substrate detection information, the main control module 22 controls the movement of the transfer module 16 so as to transfer the reaction cup to the chromogenic substrate detection station on the optical detection mechanism for optical detection. The blood analyzer 1 of the present invention performs, on the sample, the magnetic bead detection and optical detection, and the immunoturbidimetric detection and chromogenic substrate detection based on the optical method, to achieve the detection of different items of the sample to obtain different parameters of the sample.

The blood analyzer 1 of the present invention can realize the measurement of blood coagulation, anticoagulation and fibrinolysis system functions, and can mainly perform laboratory inspections on thrombus and hemostasis. Detection indexes of hemostatic and thrombus molecular markers are closely related to various clinical diseases, such as atherosclerosis, cardiovascular and cerebrovascular diseases, diabetes, arteriovenous thrombosis, thromboangiitis obliterans, pulmonary embolism, pregnancy-induced hypertension syndrome, disseminated intravascular coagulation, hemolytic uremic syndrome, and chronic obstructive pneumonia. Of course, the blood analyzer 1 of the present invention can also be used to detect the parameters of the samples other than the blood samples. Moreover, the blood analyzer 1 of the present invention performs laboratory tests on thrombus and hemostasis usually by using the optical method and magnetic bead method for detection, wherein the magnetic bead detection mechanism 171 can detect the sample using the magnetic bead method, and the optical detection mechanism can detect the sample using the optical method. The magnetic bead detection mechanism 171 and the optical detection mechanism can add different reagents to the sample for detection, so that the application range is wide.

The magnetic bead method is to measure the blood coagulation function based on the change in viscosity during plasma coagulation. The magnetic bead detection mechanism 171 generates a magnetic field during detection. The magnetic field enables a magnetic bead to swing back and forth in the reaction cup under the action of the magnetic field. Since the blood sample will gradually coagulate after a reagent is added such that the magnetic bead swings smaller and smaller and finally stops, the time of blood coagulation is measured according to the time of the reciprocating swing of the magnetic bead, and the viscosity of the blood sample is then determined. For example, test items such as activated partial thromboplastin time (referred to as APTT), prothrombin time (referred to as PT), thrombin time (referred to as TT) and human fibrinogen (referred to as FIB) are detected using the magnetic bead method, and are supported by corresponding detection reagents.

When a sample is detected for the APTT test item, the loading assembly 115 of the sample transport module 11 is loaded with a test tube rack containing the sample, and the sample is transported to a pre-set position by the transport assembly. Subsequently, the dispensing module 13 aspirates in the sample at the pre-set position, and the first transfer mechanism 161 transfers a reaction cup from the cup feeding module 12 to the sample incubation module 14 at the same time. Subsequently, the dispensing module 13 transfers the sample to the reaction cup in the sample incubation module 14, and the transport assembly of the sample transport module 11 pushes the test tube rack, in which the sample has been aspirated, out of the pre-set position into the unloading assembly 116 while the sample is being transferred. Subsequently, the dispensing module 13 first aspirates in a mixed reagent from the reagent storage module 15 and transfers the mixed reagent to the reaction cup, in which the sample has been added, in the sample incubation module 14. After the sample incubation module 14 incubates the sample for a pre-set time, the dispensing module 13 then aspirates in a reaction reagent from the reagent storage module 15 and transfers the reaction reagent to the reaction cup, in which the sample and the mixed reagent have been added, in the sample incubation module 14. Subsequently, the second transfer mechanism 162 transfers the reaction cup, in which the sample and the reagents have been added, to the magnetic bead detection station 17121 of the magnetic bead detection mechanism 171 for magnetic bead detection. Waiting until the reaction is completed, the test result is obtained and compared with a standard result, if the test result significantly deviates from the range of the standard result, the test tube rack containing the sample can be returned by the reversing assembly 117 to the loading assembly 115 for re-detection, and the used reaction cup described above is discarded by the transfer module 16.

When a sample is detected for the PT and TT test items, the loading assembly 115 of the sample transport module 11 is loaded with a test tube rack containing the sample, and the sample is transported to a pre-set position by the transport assembly. Subsequently, the dispensing module 13 aspirates in the sample at the pre-set position, and the first transfer mechanism 161 transfers a reaction cup from the cup feeding module 12 to the sample incubation module 14 at the same time. Subsequently, the dispensing module 13 transfers the sample to the reaction cup in the sample incubation module 14, and the transport assembly of the sample transport module 11 pushes the test tube rack, in which the sample has been aspirated, out of the pre-set position into the unloading assembly 116 while the sample is being transferred. Subsequently, the dispensing module 13 aspirates in a reaction reagent from the reagent storage module 15 and transfers the reaction reagent to the reaction cup, in which the sample has been added, in the sample incubation module 14. Subsequently, the second transfer mechanism 162 transfers the reaction cup, in which the sample and the reagent have been added, to the magnetic bead detection station 17121 of the magnetic bead detection mechanism 171 for magnetic bead detection. Waiting until the reaction is completed, the test result is obtained and compared with a standard result, if the test result significantly deviates from the range of the standard result, the test tube rack containing the sample can be returned by the reversing assembly 117 to the loading assembly 115 for re-detection, and the used reaction cup described above is discarded by the transfer module 16.

When a sample is detected for the FIB test item, the loading assembly 115 of the sample transport module 11 is loaded with a test tube rack containing the sample, and the sample is transported to a pre-set position by the transport assembly. The dispensing module 13 then aspirates in a diluent from the reagent storage module 15, and the first transfer mechanism 161 transfers a reaction cup from the cup feeding module 12 to the sample incubation module 14 at the same time. Subsequently, the dispensing module 13 transfers the diluent to the reaction cup in the sample incubation module 14. Subsequently, the dispensing module 13 aspirates in the sample at the pre-set position and transfers the sample to the reaction cup in the sample incubation module 14, and the transport assembly of the sample transport module 11 pushes the test tube rack, in which the sample has been aspirated, out of the pre-set position into the unloading assembly 116 while the sample is being transferred. Subsequently, the sample incubation module 14 incubates the sample and the diluent in the reaction cup. After a pre-set time, the dispensing module 13 aspirates in a reaction reagent from the reagent storage module 15 and transfers the reaction reagent to the reaction cup, in which the sample and the diluent have been added, in the sample incubation module 14. Subsequently, the second transfer mechanism 162 transfers the reaction cup, in which the sample and the reagent have been added, to the magnetic bead detection station 17121 of the magnetic bead detection mechanism 171 for magnetic bead detection. Waiting until the reaction is completed, the test result is obtained and compared with a standard result, if the test result significantly deviates from the range of the standard result, the test tube rack containing the sample can be returned by the reversing assembly 117 to the loading assembly 115 for re-detection, and the used reaction cup described above is discarded by the transfer module 16.

The optical detection mechanism can be used to detect the sample using the optical method. The following is described by taking the sample being blood as an example: the optical method is to measure the blood coagulation function based on the change in turbidity during plasma coagulation. The optical method for the optical detection mechanism mainly use an immunoturbidimetric method and a chromogenic substrate method to detect the turbidity of blood during coagulation to obtain corresponding parameters.

The immunoturbidimetric method refers to a dynamic method for measuring antigen-antibody binding and the basic principle thereof is: when an antigen and an antibody react in a special dilution system and the ratio is appropriate (generally, the antibody is generally required to be over-dosed), the formed soluble immune complex is precipitated from the liquid phase under the action of a polymerization accelerator (polyethylene glycol, etc.) in the dilution system to form microparticles, which make the reaction solution appear turbid. When the concentration of the antibody is fixed, the amount of the formed immune complex increases as the amount of the antigen increases in the test sample, and the turbidity of the reaction solution also increases accordingly. By measuring the turbidity of the reaction solution and comparing same with a series of standards, the content of the antigen in the test sample can be calculated. For example, test items such as fibrin(-ogen) degradation products (referred to as FDP) and plasma D-dimer (referred to as DD) can be detected using the immunoturbidimetric method, and are supported by corresponding detection reagents.

When a sample is detected for the FDP test item, the loading assembly 115 of the sample transport module 11 is loaded with a test tube rack containing the sample, and the sample is transported to a pre-set position by the transport assembly. Subsequently, the dispensing module 13 aspirates in the sample at the pre-set position, and the first transfer mechanism 161 transfers a reaction cup from the cup feeding module 12 to the sample incubation module 14 at the same time. Subsequently, the dispensing module 13 transfers the sample to the reaction cup in the sample incubation module 14, and the transport assembly of the sample transport module 11 pushes the test tube rack, in which the sample has been aspirated, out of the pre-set position into the unloading assembly 116 while the sample is being transferred. Subsequently, the dispensing module 13 first aspirates in a mixed reagent from the reagent storage module 15 and transfers the mixed reagent to the reaction cup, in which the sample has been added, in the sample incubation module 14. After the sample incubation module 14 incubates the sample for a pre-set time, the dispensing module 13 then aspirates in a reaction reagent from the reagent storage module 15 and transfers the reaction reagent to the reaction cup, in which the sample and the mixed reagent have been added, in the sample incubation module 14. Subsequently, the first transfer mechanism 161 or the second transfer mechanism 162 transfers the reaction cup, in which the sample and the reagents have been added, to the immunoturbidimetric detection station of the first optical detection mechanism 172 or the second optical detection mechanism 173 for optical detection. Waiting until the reaction is completed, the test result is obtained and compared with a standard result, if the test result significantly deviates from the range of the standard result, the test tube rack containing the sample can be returned by the reversing assembly 117 to the loading assembly 115 for re-detection, and the used reaction cup described above is discarded by the transfer module 16.

When a sample is detected for the DD test item, the loading assembly 115 of the sample transport module 11 is loaded with a test tube rack containing the sample, and the sample is transported to a pre-set position by the transport assembly. The dispensing module 13 then aspirates in a diluent from the reagent storage module 15, and the first transfer mechanism 161 transfers a reaction cup from the cup feeding module 12 to the sample incubation module 14 at the same time. Subsequently, the dispensing module 13 transfers the diluent to the reaction cup in the sample incubation module 14. Subsequently, the dispensing module 13 aspirates in the sample at the pre-set position and transfers the sample to the reaction cup in the sample incubation module 14, and the transport assembly of the sample transport module 11 pushes the test tube rack, in which the sample has been aspirated, out of the pre-set position into the unloading assembly 116 while the sample is being transferred. Subsequently, the sample incubation module 14 incubates the sample and the diluent in the reaction cup. After a pre-set time, the dispensing module 13 aspirates in a reaction reagent from the reagent storage module 15 and transfers the reaction reagent to the reaction cup, in which the sample and the diluent have been added, in the sample incubation module 14. Subsequently, the first transfer mechanism 161 or the second transfer mechanism 162 transfers the reaction cup, in which the sample and the reagent have been added, to the immunoturbidimetric detection station of the first optical detection mechanism 172 or the second optical detection mechanism 173 for optical detection. Waiting until the reaction is completed, the test result is obtained and compared with a standard result, if the test result significantly deviates from the range of the standard result, the test tube rack containing the sample can be returned by the reversing assembly 117 to the loading assembly 115 for re-detection, and the used reaction cup described above is discarded by the transfer module 16.

With regard to the chromogenic substrate method, first, a compound that can be catalyzed by the thromboplastin to be detected is artificially synthesized, and the compound is connected to a color-producing substance, and the color-producing substance can be dissociated during the detection process, causing a color change in the sample. The activity of the detected thromboplastin can be inferred based on the color change. For example, test items such as antithrombin III (referred to as AT III) can be detected using the chromogenic substrate method, and are supported by corresponding detection reagents.

When a sample is detected for the AT III test item, the loading assembly 115 of the sample transport module 11 is loaded with a test tube rack containing the sample, and the sample is transported to a pre-set position by the transport assembly. The dispensing module 13 then aspirates in a diluent from the reagent storage module 15, and the first transfer mechanism 161 transfers a reaction cup from the cup feeding module 12 to the sample incubation module 14 at the same time. Subsequently, the dispensing module 13 transfers the diluent to the reaction cup in the sample incubation module 14. Subsequently, the dispensing module 13 aspirates in the sample at the pre-set position and transfers the sample to the reaction cup in the sample incubation module 14, and the transport assembly of the sample transport module 11 pushes the test tube rack, in which the sample has been aspirated, out of the pre-set position into the unloading assembly 116 while the sample is being transferred. Subsequently, the dispensing module 13 first aspirates in a mixed reagent from the reagent storage module 15 and transfers the mixed reagent to the reaction cup, in which the sample and the diluent have been added, in the sample incubation module 14. After the sample incubation module 14 incubates the sample in the reaction cup for a pre-set time, the dispensing module 13 then at the reagent storage module 15. Subsequently, the first transfer mechanism 161 or the second transfer mechanism 162 transfers the reaction cup, in which the sample and the reagent have been added, to the chromogenic substrate detection station of the first optical detection mechanism 172 or the second optical detection mechanism 173 for optical detection. Waiting until the reaction is completed, the test result is obtained and compared with a standard result, if the test result significantly deviates from the range of the standard result, the test tube rack containing the sample can be returned by the reversing assembly 117 to the loading assembly 115 for re-detection, and the used reaction cup described above is discarded by the transfer module 16.

It should be noted that the pre-set position in the present invention refers to the puncture position and the non-puncture position. The puncture needle mechanism 131 aspirates in the sample at the puncture position. The integrated needle 1325 aspirates in the sample at the non-puncture position. The dispensing needle mechanism 134 aspirates in the sample at the non-puncture position. Moreover, the blood analyzer 1 of the present invention can detect the above items whether using the one-needle mechanism, two-needle mechanism, or three-needle mechanism, but the action forms of the dispensing module 13 are different. The requirements of use can be met as long as it is ensured that the dispensing module 13 uses the corresponding needle mechanism to aspirate in the corresponding sample or reagent at the corresponding position according to the above requirements.

The default settings of the blood analyzer 1 of the present invention are seven test items, namely APTT, PT, TT, FIB, FDP, DD, and ATIII, wherein the APTT, PT, TT and FIB test items automatically follow the process of magnetic bead detection in which after the corresponding reagent has been added, the second transfer mechanism 162 transfers the reaction cup that needs to be subjected to the above detection to the magnetic bead detection station 17121 of the magnetic bead detection mechanism 171 for magnetic bead detection; and the FDP, DD and ATIII test items automatically follow the process of optical detection, in which for the FDP and DD test items, after the corresponding reagent has been added, the reaction cup that needs to be subjected to the above detection is transferred to the immunoturbidimetric detection station of the first optical detection mechanism 172 or the second optical detection mechanism 173 by the first transfer mechanism 161 or the second transfer mechanism 162 for optical detection; and for the ATIII test item, after the corresponding reagent has been added, the reaction cup that needs to be subjected to the above detection is transferred to the chromogenic substrate detection station of the first optical detection mechanism 172 or the second optical detection mechanism 173 by the first transfer mechanism 161 or the second transfer mechanism 162 for optical detection.

In addition, when the sample is re-detected, the same test method needs to be used for re-detection. For example, when the sample is detected for the APTT item, if the detection result significantly deviates from the standard result, the deviation in the results may be caused by the lack of the magnetic bead in the reaction cup and other external environments, the sample needs to be re-detected by the same method, and the detection result is obtained again in this case.

When the sample in one test tube needs to be detected for multiple different test items, the dispensing module 13 needs to transfer the sample from the test tube of the sample transport module 11 to multiple reaction cups of the sample incubation module 14 respectively. Each reaction cup corresponds to one test item and a corresponding reagent is added, and a corresponding parameter is obtained by using the corresponding detection method. Moreover, the blood sample can be detected for the above test items to obtain different parameters, making the blood detection results reliable, ready, and convenient for diagnosis.

When the blood analyzer 1 of the present invention is performing detection, the scanning mechanism 113 can scan the information of the detection code on the test tube and transmit the information to the main controller. The main controller can obtain the information of the test item to be performed on the sample, and the main controller then can control the detection method required for this test item and time used therefor to start different detection processes (such as optical method and magnetic bead method), detection mechanisms (such as optical detection mechanism and magnetic bead detection mechanism 171), etc., to realize the automatic operation of sample detection. When the blood analyzer 1 of the present invention detects other test items other than the above test items, such as blood coagulation factor VIII, the user can select and set the corresponding detection method and the corresponding reagent by himself/herself to start different detection processes and detection mechanisms.

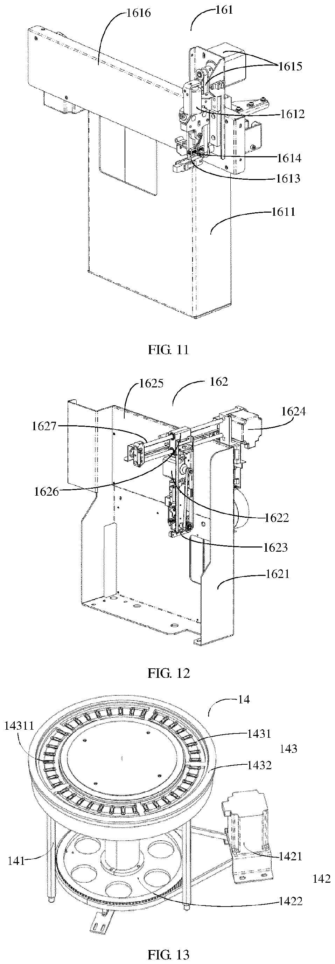

Referring to Figs. 1 and 11, the first transfer mechanism 161 comprises a first transfer mounting plate 1611, two oppositely-arranged first cup grasping portions 1613, a first grasping drive assembly 1612, a first elastic member 1614, a first transfer drive assembly 1615, and a first sliding track 1616. The two first cup grasping portions 1613 are oppositely arranged to grasp the reaction cup. The first grasping drive assembly 1612 is used to realize the grasping and releasing of the reaction cup. The first grasping drive assembly 1612 is connected to at least one of the two first cup grasping portions 1613, and drives the two first cup grasping portions 1613 to move toward each other and/or away from each other. When the two first cup grasping portions 1613 move toward each other, the reaction cup is clamped. When two first cup grasping portions 1613 move away from each other, the reaction cup is released. The first transfer drive assembly 1615 is used to transfer the reaction cup. The first transfer drive assembly 1615 is connected to the first grasping drive assembly 1612. The first transfer drive assembly 1615 can drive the first grasping drive assembly 1612 and the first cup grasping portion 1613 connected to the first grasping drive assembly 1612 to move along the first sliding track 1616, such that the first transfer drive assembly 1615 can transfer the reaction cup between the cup feeding module 12, the sample incubation module 14 and the first optical detection module via the first cup grasping portion 1613. The first transfer mounting plate 1611 has a supporting function. The first sliding track 1616 is arranged on a first transfer supporting plate, and the first transfer mechanism 161 is mounted on the testing platform 101 via the first transfer supporting plate.

The first grasping drive assembly 1612 can use a power source such as a motor, an electromagnet, an air cylinder and a hydraulic cylinder, which can either drive the movement of one of the two first cup grasping portions 1613 or drive the simultaneous movement of the two first cup grasping portions 1613. The first elastic member 1614 is arranged between the two first cup grasping portions 1613. The movement of the two first cup grasping portions 1613 toward or away from each other caused by the first grasping drive assembly 1612 enables the first elastic member 1614 to deform. In this case, the restoring force of the first elastic member 1614 is opposed to the movement which causes the deformation thereof, that is, when the two first cup grasping portions 1613 move toward each other, the first elastic member 1614 is deformed, and the restoring force of the first elastic member 1614 then causes the two first cup grasping portions 1613 to move away from each other. When the two first cup grasping portions 1613 move away from each other, the first elastic member 1614 is deformed, and the restoring force of the first elastic member 1614 then causes the two first cup grasping portions 1613 to move toward each other. The first transfer drive assembly 1615 comprises a first transfer carriage, a first transfer drive motor and a first transfer transmission component. The first grasping drive assembly 1612 is mounted on the first transfer carriage, and the first transfer carriage is also slidably mounted on the first sliding track 1616. The first transfer drive motor is in driving connection with the first transfer carriage via the first transfer transmission component, to drive the first transfer carriage to move along the first sliding track 1616 to realize the transfer operation of the reaction cup.

Moreover, the first transfer mechanism 161 further comprises a first ejector rod, which is located between the two first cup grasping portions 1613 and connected to the first grasping drive assembly 1612. The first grasping drive assembly 1612 can drive the first ejector rod to perform an extending or retracting motion. When the first ejector rod is extended, the first ejector rod can extend into the space between the two first cup grasping portions 1613, such that the two first cup grasping portions 1613 move away from each other. When the first ejector rod is retracted, the two first cup grasping portions 1613 move toward each other under the action of the first elastic member 1614.

Referring to Figs. 1 and 12, the second transfer mechanism 162 comprises a second transfer mounting plate 1621, two oppositely-arranged second cup grasping portions 1623, a second grasping drive assembly 1622, a second elastic member, a second transfer drive assembly 1624, a second sliding track 1625, a third transfer drive assembly 1626 and a third sliding track 1627. The two second cup grasping portions 1623 are oppositely arranged to grasp the reaction cup. The second grasping drive assembly 1622 is used to realize the grasping and releasing of the reaction cup. The second grasping drive assembly 1622 is connected to at least two of the two second cup grasping portions 1623, and drives the two second cup grasping portions 1623 to move toward each other and/or away from each other. When the two second cup grasping portions 1623 move toward each other, the reaction cup is clamped. When two second cup grasping portions 1623 move away from each other, the reaction cup is released. The second transfer drive assembly 1624 and the third transfer drive assembly 1626 are used to transfer the reaction cup. The second transfer drive assembly 1624 is slidably arranged on the second sliding track 1625. The third sliding track 1627 is arranged on the second transfer drive assembly 1624. The third transfer drive assembly 1626 is slidably arranged on the third sliding track 1627. The second grasping drive assembly 1622 is arranged on the third transfer drive assembly 1626. The second transfer drive assembly 1624 can drive the third sliding track 1627 and the third transfer drive assembly 1626 on the third sliding track 1627 to move along the second sliding track 1625. The third transfer drive assembly 1626 can drive the second grasping drive assembly 1622 and the second cup grasping portions 1623 connected to the second grasping drive assembly 1622 to move along the third sliding track 1627, such that the second transfer drive assembly 1624 and the third transfer drive assembly 1626 can transfer the reaction cup between the sample incubation module 14, the first optical detection module and the magnetic bead detection mechanism 171 via the second cup grasping portions 1623. It should be noted that the extension direction of the second sliding track 1625 is perpendicular to the extension direction of the third sliding track 1627, and the extension direction of the second sliding track 1625 is parallel to the extension direction of the first sliding track 1616. In this way, it is possible to increase the movement range of the second transfer mechanism 162 without interfering with the first transfer mechanism 161, and to increase the transport range of the second transfer mechanism 162, improving the operation efficiency. The second transfer mounting plate 1621 has a supporting function. The second sliding track 1625 is arranged on a second transfer supporting plate, and the second transfer mechanism 162 is mounted on the testing platform 101 via the second transfer supporting plate.

The ways in which the second grasping drive assembly 1622 and the first grasping drive assembly 1612 are arranged are basically the same, and the ways in which the second elastic member and the first elastic member 1614 are arranged are basically the same. Moreover, the second transfer mechanism 162 further comprises a second ejector rod. The arrangement position and control method of the second ejector rod are basically the same as those of the first ejector rod. The configurations of the second transfer drive assembly 1624 and the third transfer drive assembly 1626 are also basically the same as the first transfer drive assembly 1615. For the same or similar components, detailed descriptions are not provided here. Of course, in other implementations of the present invention, the second grasping drive assembly 1622 and the first grasping drive assembly 1612 may also be driven directly by an air cylinder or a hydraulic cylinder, or be implemented by using a motor in cooperation with a cam mechanism, a synchronous belt mechanism, a lead screw-nut mechanism, etc. Moreover, the forms of the first cup grasping portions 1613 and the second cup grasping portions 1623 are not limited, as long as the reaction cup can be grasped. Preferably, the first cup grasping portions 1613 and the second cup grasping portions 1623 are both clamping blocks or jaws.

It should be noted that the sample incubation module 14 is a hub scheduled by the blood analyzer 1 of the present invention. Referring to Figs. 1 to 3 and Fig. 13, the sample incubation module 14 drives an empty placement station 14311 to move to the cup picking-up and placement position, the first transfer mechanism 161 can move to the cup picking-up and placement position and place an empty reaction cup into a placement station 14311 of the sample incubation module 14. The sample incubation module 14 drives the reaction cup to move to the cup picking-up and placement position, the first transfer mechanism 161 can move to the cup picking-up and placement position and transfer the reaction cup, in which the sample and the reagent have been added, to the first optical detection mechanism 172. The sample incubation module 14 drives the reaction cup to move to the cup picking-up position. The second transfer mechanism 162 can move to the cup picking-up position and transfer the reaction cup, in which the sample and the reagent have been added, to the second optical detection mechanism 173 or the magnetic bead detection mechanism 171. The sample incubation module 14 drives the reaction cup to move to the sample adding position. The puncture needle mechanism 131 moves to the sample adding position and adds the sample to the reaction cup in the sample incubation module 14. The sample incubation module 14 drives the reaction cup to move to the first reagent adding position. The reagent needle mechanism 133 moves to the first reagent adding position and adds a reagent to the reaction cup in which the sample has been added. The sample incubation module 14 drives the reaction cup to move to the second reagent adding position. The integrated needle mechanism 132 moves to the second reagent adding position and adds the sample or the reagent to the reaction cup in which the sample has been added.

Referring to Figs. 1, 2 and 8, as a possible implementation, the cup feeding module 12 comprises a cup feeding bracket 121, a reaction cup storage mechanism 122, a reaction cup transport mechanism 123 and a reaction cup transfer mechanism 124. The reaction cup transport mechanism 123 is arranged obliquely, the reaction cup storage mechanism 122 is arranged at a lower position of the reaction cup transport mechanism 123, and the reaction cup transfer mechanism 124 can move on the reaction cup transport mechanism 123. Two ends of the reaction cup transport mechanism 123 are respectively at a lower position and a higher position, and the lower position is lower than the higher position. The reaction cup transport mechanism 123 can drive the reaction cup transfer mechanism 124 to move between the lower position and the higher position of the reaction cup transport mechanism 123, to achieve the transfer of the reaction cup. Specifically, the reaction cup transport mechanism 123 transports the reaction cup transfer mechanism 124 to the lower position, and the reaction cup storage mechanism 122 transports the reaction cup to the reaction cup transfer mechanism 124, the reaction cup transport mechanism 123 then transports the reaction cup transfer mechanism 124 to the higher position such that the reaction cup transfer mechanism 124 drives the reaction cup to move from the lower position to the higher position, and the first transfer mechanism 161 grasps the reaction cup on the reaction cup transfer mechanism 124 at the higher position. The reaction cup transfer mechanism 124 is provided with an empty cup picking-up position at which the first transfer mechanism 161 grasps the reaction cup. The cup feeding module 12 is used to realize the automatic transport of the empty reaction cup to improve the transport efficiency of the reaction cup, thereby improving the operation efficiency of the blood analyzer 1. The cup feeding bracket 121 is used to have a supporting function. The reaction cup storage mechanism 122, the reaction cup transport mechanism 123 and the reaction cup transfer mechanism 124 are all mounted on the cup feeding bracket 121. The reaction cup storage mechanism 122, the reaction cup transport mechanism 123 and the reaction cup transfer mechanism 124 are supported on the testing platform 101 by the cup feeding bracket 121, and are located on a side of the sample detection module 17 and the sample incubation module 14.

The empty reaction cup is stored on the reaction cup storage mechanism 122. The reaction cup storage mechanism 122 comprises two parts. One of the parts is a reaction cup storage disk 1221 provided in the form of a disk. Reaction cups are fixed by a thin film and are wound around the reaction cup storage disk 1221 in layers in the form of a disk. The other part is a film removal component 1222 for removing the thin film from a reaction cup so as to transport the reaction cup to the reaction cup transfer mechanism 124. It should be noted that the empty cup picking-up position is at a fixed position, and after the reaction cup transport mechanism 123 transports the reaction cup transfer mechanism 124 from the lower position to the higher position, the empty reaction cup now is located on the empty cup picking-up position. Of course, it is also possible to transport the empty reaction cup to the empty cup picking-up position manually or by another mechanism. The reaction cup transport mechanism 123 uses a synchronous belt structure or another similar structure such as a chain drive to implement the transport of the reaction cup. Moreover, the reaction cup transport mechanism 123 is arranged obliquely, the lower position of the reaction cup transport mechanism 123 is connected to the film removal component 1222, and the higher end of the reaction cup transport mechanism 123 is connected to the reaction cup transfer mechanism 124, so as to transport the reaction cup to the empty cup picking-up position of the reaction cup transfer mechanism 124. The obliquely-arranged reaction cup transport mechanism 123 can obliquely transport the reaction cup, avoiding the problem of the excessive overall height of the cup feeding module 12 caused by the reaction cup storage disk 1221, reducing the overall height of the cup feeding module 12, thereby reducing the overall height of the blood analyzer 1.

The reaction cup transfer mechanism 124 comprises a reaction cup carrying component 1241 and a blocking component 1242. The blocking component 1242 is movably arranged at the lower position of the reaction cup transport mechanism 123, and the reaction cup carrying component 1241 is movably arranged on the reaction cup transport mechanism 123. The reaction cup carrying component 1241 is used to carry the reaction cup. The reaction cup carrying component 1241 holds the reaction cup at the lower position of the reaction cup transport mechanism 123. Subsequently, the reaction cup transport mechanism 123 drives the reaction cup carrying component 1241 to move to the higher position. At this time, the first transfer mechanism 161 can grasp the reaction cup at the empty cup picking-up position. The blocking component 1242 is used to block the film removal component 1222, to prevent the reaction cup from falling from the film removal component 1222 when the reaction cup carrying component 1241 is moved from the lower position to the higher position.

When the blood analyzer 1 transport the empty reaction cup, the reaction cup storage disk 1221 rotates and releases the reaction cup fixed on the thin film, and the film removal component 1222 peels off the thin film from the reaction cup. At the same time, the reaction cup transport mechanism 123 drives the reaction cup carrying component 1241 of the reaction cup transfer mechanism 124 to move to the lower position, and the film removal component 1222 transports the reaction cup to the reaction cup carrying component 1241. Subsequently, the reaction cup transport mechanism 123 drives the reaction cup carrying component 1241 to move to the higher position. At the same time, the blocking component 1242 of the reaction cup transfer mechanism 124 blocks the film removal component 1222. At this time, the reaction cup is moved to the empty cup picking-up position. The transfer module 16 then transfers the reaction cup from the empty cup picking-up position to the cup picking-up and placement position on the sample incubation module 14.

It should be noted that the sample incubation module 14 is a hub scheduled by the blood analyzer 1 of the present invention. Referring to Figs. 1 to 3 and Fig. 13, the sample incubation module 14 drives an empty placement station 14311 to move to the cup picking-up and placement position, the first transfer mechanism 161 can move to the cup picking-up and placement position and place an empty reaction cup into a placement station 14311 of the sample incubation module 14. The sample incubation module 14 drives the reaction cup to move to the cup picking-up and placement position, the first transfer mechanism 161 can move to the cup picking-up and placement position and transfer the reaction cup, in which the sample and the reagent have been added, to the first optical detection mechanism 172. The sample incubation module 14 drives the reaction cup to move to the cup picking-up position. The second transfer mechanism 162 can move to the cup picking-up position and transfer the reaction cup, in which the sample and the reagent have been added, to the second optical detection mechanism 173 or the magnetic bead detection mechanism 171. The sample incubation module 14 drives the reaction cup to move to the filling position. The dispensing module 13 moves to the filling position and adds the sample or the reagent to the reaction cup in the sample incubation module 14.

It should also be noted that the cup picking-up and placement position, the filling position and the cup picking-up position of the sample incubation module 14 are at a fixed position on the sample incubation module 14, the dispensing module 13 transfers, at a fixed position all the time, the sample and the reagent to the sample incubation module 14, and the transfer module 16 transports or transfers, at a fixed position all the time, the reaction cup. When the sample incubation module 14 rotates, the cup picking-up and placement position, the filling position and the cup picking-up position do not move, the sample incubation module 14 can drive the reaction cup in the placement station 14311 thereon to the corresponding position to perform the corresponding operation.

Specifically, the sample incubation module 14 comprises an incubation support frame 141, an incubation drive mechanism 142 and a sample incubation mechanism 143. The incubation support frame 141 is used to support the sample incubation mechanism 143 on the testing platform 101. The incubation drive mechanism 142 is connected to the sample incubation mechanism 143, and drives the sample incubation mechanism 143 to rotate, such that the sample incubation mechanism 143 is moved to the cup picking-up and placement position, the filling position and the cup picking-up position, and performs the corresponding operations. Specifically, the sample incubation mechanism 143 comprises an incubation disk 1431, a heating component, and a thermally insulating pot 1432. The incubation disk 1431 is rotatably provided in thermally insulating pot 1432, and the heating component is provided between the incubation disk 1431 and thermally insulating pot 1432. The incubation disk 1431 is provided with a plurality of placement stations 14311. The plurality of placement stations 14311 are arranged in sequence in a radial direction of the incubation disk 1431. The reaction cup is placed in the placement station 14311 of the incubation disk 1431. When the incubation disk 1431 rotates, the sample and the reagent in the reaction cup will be subjected to a centrifugal force to evenly mix the sample and the reagent, so that it is ensured that the sample and the reagent are mixed evenly to ensure the accurate detection result of the sample.

The incubation drive mechanism 142 comprises an incubation drive motor 1421 and an incubation transmission component 1422. The incubation drive motor 1421 is fixed onto the testing platform 101. The incubation disk 1431 is in driving connection with an output end of the incubation drive motor 1421 via the incubation transmission component 1422, such that the incubation drive motor 1421 drives the incubation disk 1431 to rotate, and the incubation disk 1431 drives the placement station 14311 thereon to rotate to the cup picking-up and placement position, the filling position and the cup picking-up position so as to perform the corresponding operations. The incubation transmission component 1422 can use a synchronous belt conveying component or other components capable of achieving the transmission of movement, such as a lead screw, and a sprocket wheel.

Referring to Figs. 1 to 3 and Fig. 13, when the blood analyzer 1 of the present invention uses the three-needle mechanism, the sample incubation module 14 drives a reaction cup to move to the sample adding position. The puncture needle mechanism 131 moves to the sample adding position and adds a sample or a regent to the reaction cup in the sample incubation module 14. The sample incubation module 14 drives the reaction cup to move to the first reagent adding position. The reagent needle mechanism 133 moves to the first reagent adding position and adds the reagent to the reaction cup. The sample incubation module 14 drives the reaction cup to move to the second reagent adding position. The integrated needle mechanism 132 moves to the second reagent adding position and adds the sample or the reagent to the reaction cup in which the sample has been added.

Specifically, when the blood analyzer 1 works, the first transfer mechanism 161 grasps the empty reaction cup at the cup picking-up and placement position, and moves to the cup picking-up and placement position of the incubation disk 1431. At the same time, the incubation disk 1431 drives the placement station 14311 thereon to rotate to the cup picking-up and placement position, and the first transfer mechanism 161 places the empty reaction cup into the placement station 14311 at the cup picking-up and placement position. The puncture needle mechanism 131 aspirates in the sample at the puncture position of the sample transport module 11 and then rotates to the sample adding position of the incubation disk 1431. At the same time, the incubation disk 1431 then drives the placement station 14311 thereon to move to the sample adding position, and the puncture needle mechanism 131 adds the sample aspirated thereby to the reaction cup in the placement station 14311 at the sample adding position. The integrated needle mechanism 132 aspirates in a reagent at the first reagent aspirating position of the first reagent storage mechanism 151 and then rotates to the second reagent adding position of the incubation disk 1431. At the same time, the incubation disk 1431 drives the reaction cup, in which the sample has been added, in the placement station 14311 thereon to move to the second reagent adding position. At the second reagent adding position, the integrated needle mechanism 132 adds the reagent aspirated thereby to the reaction cup, in which the sample has been added, in the placement station 14311 at the second reagent adding position. The reagent needle mechanism 133 aspirates in a reagent at the second reagent aspirating position of the first reagent storage mechanism 151 and then rotates to the first reagent adding position of the incubation disk 1431. At the same time, the incubation disk 1431 drives the reaction cup, in which the sample has been added, in the placement station 14311 thereon to move to the first reagent adding position. At the first reagent adding position, the reagent needle mechanism 133 adds the reagent aspirated thereby to the reaction cup, in which the sample has been added, in the placement station 14311 at the first reagent adding position. The incubation disk 1431 drives the reaction cup thereon, in which the sample and the reagent have been added, to move to the cup picking-up and placement position. The first transfer mechanism 161 moves to the cup picking-up and placement position to grasp the reaction cup and transfers the reaction cup to the first optical detection station 17221 of the first optical detection mechanism 172. The incubation disk 1431 drives the reaction cup, in which the sample and the reagent have been added, to move to the cup picking-up position. The second transfer mechanism 162 moves to the cup picking-up position to grasp the reaction cup and transfers the reaction cup to the second optical detection station of the second optical detection mechanism 173 or to the magnetic bead detection station 17121 of the magnetic bead detection mechanism 171. When the integrated needle mechanism 132 is used to add the sample, the integrated needle mechanism 132 aspirates in the sample at the non-puncture position of the sample transport module 11, and the integrated needle mechanism 132 then moves to the second reagent adding position of the sample incubation module 14. At the same time, the incubation disk 1431 drives the empty reaction cup in the placement station 14311 thereon to move to the second reagent adding position. At the second reagent adding position, the integrated needle mechanism 132 adds the sample aspirated thereby to the reaction cup in the placement station 14311 at the second reagent adding position.

Referring to Figs. 21 to 23, when the blood analyzer 1 of the present invention uses the two-needle structure, the sample incubation module 14 drives a reaction cup to move to the sample adding position. The integrated needle mechanism 132 moves to the sample adding position and adds a sample or a reagent to the reaction cup. The sample incubation module 14 drives the reaction cup to move to the first reagent adding position. The reagent needle mechanism 133 moves to the first reagent adding position and adds the reagent to the reaction cup.

Specifically, when the blood analyzer 1 works, the first transfer mechanism 161 grasps the empty reaction cup at the cup picking-up and placement position, and moves to the cup picking-up and placement position of the incubation disk 1431. At the same time, the incubation disk 1431 drives the placement station 14311 thereon to rotate to the cup picking-up and placement position, and the first transfer mechanism 161 places the empty reaction cup into the placement station 14311 at the cup picking-up and placement position. The integrated needle mechanism 132 aspirates in the sample at the non-puncture position of the sample transport module 11 and then rotates to the sample adding position of the incubation disk 1431. At the same time, the incubation disk 1431 then drives the placement station 14311 thereon to move to the sample adding position, and the integrated needle mechanism 132 adds the sample aspirated thereby to the reaction cup in the placement station 14311 at the sample adding position. The integrated needle mechanism 132 aspirates in a reagent at the first reagent aspirating position of the first reagent storage mechanism 151 and then rotates to the sample adding position of the incubation disk 1431. At the same time, the incubation disk 1431 drives the reaction cup, in which the sample has been added, in the placement station 14311 thereon to move to the sample adding position. At the sample adding position, the integrated needle mechanism 132 adds the reagent aspirated thereby to the reaction cup, in which the sample has been added, in the placement station 14311 at the sample adding position. The reagent needle mechanism 133 aspirates in a reagent at the second reagent aspirating position of the first reagent storage mechanism 151 and then rotates to the first reagent adding position of the incubation disk 1431. At the same time, the incubation disk 1431 drives the reaction cup, in which the sample has been added, in the placement station 14311 thereon to move to the first reagent adding position. At the first reagent adding position, the reagent needle mechanism 133 adds the reagent aspirated thereby to the reaction cup, in which the sample has been added, in the placement station 14311 at the first reagent adding position. The incubation disk 1431 drives the reaction cup thereon, in which the sample and the reagent have been added, to move to the cup picking-up and placement position. The first transfer mechanism 161 moves to the cup picking-up and placement position to grasp the reaction cup and transfers the reaction cup to the first optical detection station 17221 of the first optical detection mechanism 172. The incubation disk 1431 drives the reaction cup, in which the sample and the reagent have been added, to move to the cup picking-up position. The second transfer mechanism 162 moves to the cup picking-up position to grasp the reaction cup and transfers the reaction cup to the second optical detection station of the second optical detection mechanism 173 or to the magnetic bead detection station 17121 of the magnetic bead detection mechanism 171.

Referring to Fig. 24, when the blood analyzer 1 of the present invention uses the one-needle structure, the sample incubation module 14 drives a reaction cup to move to the filling position. The dispensing needle mechanism 134 moves to the filling position and adds a sample or a reagent to the reaction cup in which the sample has been added.

Specifically, when the blood analyzer 1 works, the first transfer mechanism 161 grasps the empty reaction cup at the cup picking-up and placement position, and moves to the cup picking-up and placement position of the incubation disk 1431. At the same time, the incubation disk 1431 drives the placement station 14311 thereon to rotate to the cup picking-up and placement position, and the first transfer mechanism 161 places the empty reaction cup into the placement station 14311 at the cup picking-up and placement position. The dispensing needle mechanism 134 aspirates in the sample at the non-puncture position of the sample transport module 11 and then rotates to the filling position of the incubation disk 1431. At the same time, the incubation disk 1431 then drives the placement station 14311 thereon to move to the filling position, and the dispensing needle mechanism 134 adds the sample aspirated thereby to the reaction cup in the placement station 14311 at the filling position. The dispensing needle mechanism 134 aspirates in a reagent at the reagent aspirating position of the first reagent storage mechanism 151 and then rotates to the filling position of the incubation disk 1431. At the same time, the incubation disk 1431 drives the reaction cup, in which the sample has been added, in the placement station 14311 thereon to move to the filling position. At the filling position, the dispensing needle mechanism 134 adds the reagent aspirated thereby to the reaction cup, in which the sample has been added, in the placement station 14311 at the filling position. The incubation disk 1431 drives the reaction cup thereon, in which the sample and the reagent have been added, to move to the cup picking-up and placement position. The first transfer mechanism 161 moves to the cup picking-up and placement position to grasp the reaction cup and transfers the reaction cup to the first optical detection station 17221 of the first optical detection mechanism 172. The incubation disk 1431 drives the reaction cup, in which the sample and the reagent have been added, to move to the cup picking-up position. The second transfer mechanism 162 moves to the cup picking-up position to grasp the reaction cup and transfers the reaction cup to the second optical detection station of the second optical detection mechanism 173 or to the magnetic bead detection station 17121 of the magnetic bead detection mechanism 171.

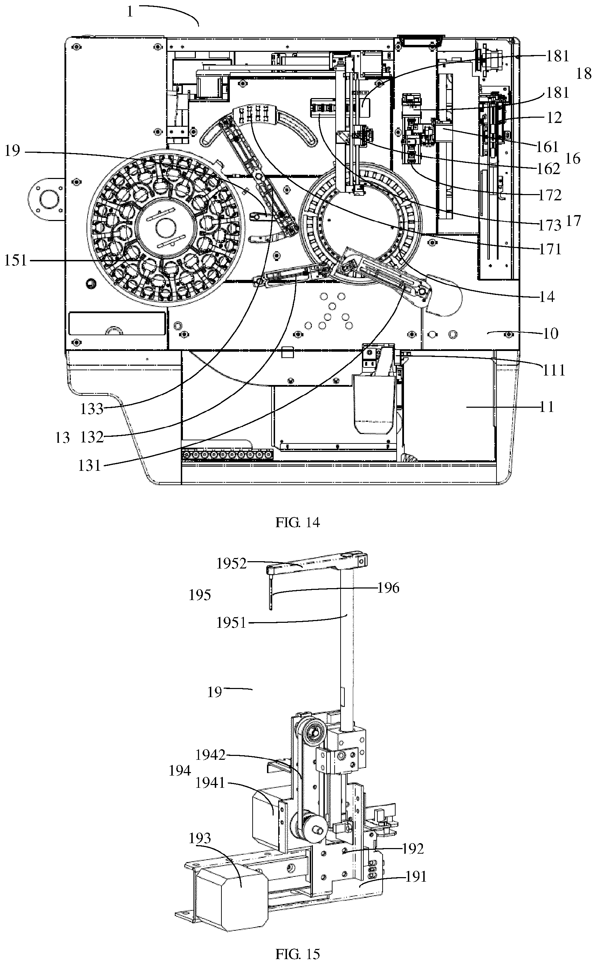

Optionally, referring to Fig. 14, the blood analyzer 1 further comprises a recovery module 18. The recovery module 18 comprises a first recovery mechanism and a second recovery mechanism. The first recovery mechanism is provided on one side of the first optical detection mechanism 172, and the second recovery mechanism is provided on one side of the second optical detection mechanism 173. The recovery module 18 is used to recover the reaction cup that has been detected, to avoid the environmental pollution caused by the random flow of the sample and the reagent from the reaction cup. Since the magnetic bead detection mechanism 171, the first optical detection mechanism 172 and the second optical detection mechanism 173 are respectively implemented by the first transfer mechanism 161 and the second transfer mechanism 162, the first transfer mechanism 161 and the second transfer mechanism 162 respectively correspond to a cup discarding place in order to allow the reaction cup, in which the reaction is completed, to be discarded from the sample detection module 17. That is, the first transfer mechanism 161 further can discard the reaction cup that has been detected by the first optical detection mechanism 172 into the first recovery mechanism, and the second transfer mechanism 162 further can discard the reaction cup, which has been detected by the second optical detection mechanism 173 and the magnetic bead detection mechanism 171, into the second recovery mechanism. When the blood analyzer 1 issues an emptying instruction, the first transfer mechanism 161 and the second transfer mechanism 162 can also transfer all the reaction cups in the sample incubation module 14 and the sample detection module 17 to the first recovery mechanism and the second recovery mechanism.

Specifically, the first recovery mechanism has a first recovery component and a first recovery storage box connected to the first recovery component. The first recovery component has a first cup discarding opening 181. The first recovery component is arranged on a side of the first optical detection mechanism 172, and the first recovery storage box is arranged in the placement cavity 104 of the test housing 10. The first transfer mechanism 161 can grasp the reaction cup that has been detected on the first optical detection mechanism 172 and move the reaction cup to the first cup discarding opening 181 to discard the reaction cup. The discarded reaction cup enters the first recovery component through the first cup discarding opening 181, and then enters the first recovery storage box and is temporarily stored therein. After the first recovery storage box is filled with the reaction cups, the first recovery storage box is removed from the test housing 10 and is replaced, or the reaction cups are poured from the first recovery storage box to a specified position.

The second recovery mechanism has a second recovery component and a second recovery storage box connected to the second recovery component. The second recovery component has a second cup discarding opening 182. The second recovery component is arranged on a side of the second optical detection mechanism 173, and the second recovery storage box is arranged in the placement cavity 104 of the test housing 10. The second transfer mechanism 162 can grasp the reaction cup that has been detected on the second optical detection mechanism 173 and move the reaction cup to the second cup discarding opening 182 to discard the reaction cup. The discarded reaction cup enters the second recovery component through the second cup discarding opening 182, and then enters the second recovery storage box and is temporarily stored therein. After the second recovery storage box is filled with the reaction cups, the second recovery storage box is removed from the test housing 10 and is replaced, or the reaction cups are poured from the second recovery storage box to a specified position. Of course, in other implementations of the present invention, the first recovery storage box and the second recovery storage box may also have an integrated structure. It is also possible that the first recovery mechanism comprises only the first recovery component, and the second recovery mechanism comprises only the second recovery component. The bottoms of the first recovery component and the second recovery component are blocked by a sealing plate. When the first recovery component and the second recovery component need to be emptied, the sealing plate is opened such that the reaction cups fall from the first recovery component and the second recovery component into a designated receiving box.

It should be noted that for the first cup discarding opening 181 of the first recovery mechanism, at least three of the first cup discarding opening 181, the empty cup picking-up position, the cup picking-up and placement position and the first optical detection mechanism 172 are arranged collinearly and correspond to a movement trajectory of the first transfer mechanism 161. This enables the movement of the first transfer mechanism 161 to be a linear reciprocating movement, reducing the space occupied by the first transfer mechanism 161, thereby reducing the overall size of the blood analyzer 1. The first transfer mechanism 161 performing a linear movement can also prevent the first transfer mechanism 161 from interfering with the second transfer mechanism 162 or other components to ensure the reliability of the movement. Moreover, the first cup discarding opening 181, the empty cup picking-up position, the cup picking-up and placement position and the first optical detection mechanism 172 being arranged collinearly can also facilitate the first transfer mechanism 161 to transfer the reaction cup, which improves the operation efficiency. For example, it is possible for the first cup discarding opening 181, the empty cup picking-up position and the cup picking-up and placement position to be arranged collinearly, or it is also possible for the empty cup picking-up position, the cup picking-up and placement position and the first optical detection mechanism 172 to be arranged collinearly, or it is possible for all of the first cup discarding opening, the empty cup picking-up position, the cup picking-up and placement position and the first optical detection mechanism to be arranged collinearly, etc.

Referring to Figs. 1 to 3 and Fig. 15, optionally, the blood analyzer 1 further comprises a stirring module 19. The stirring module 19 is arranged close to the sample incubation module 14. The stirring module 19 is used to evenly mix the sample and the reagent in the reaction cup in the sample incubation module 14. The blood analyzer 1 is further provided with a stirring position corresponding to the sample incubation module 14, and the stirring module 19 stirs the reaction cup, in which the sample and the reagent have been added, in the sample incubation module 14 at the stirring position. When the sample and the reagent in the reaction cup still need to be mixed, the sample incubation mechanism 143 drives the reaction cup on the placement station 14311 to rotate to the stirring position. The stirring module 19 can extend into the reaction cup to stir the sample and the reagent to further mix the sample and the reagent evenly.

Specifically, the stirring module 19 comprises a first stirring support plate 191, a second stirring support plate 192, a first stirring drive mechanism 193, a second stirring drive mechanism 194, a stirring mounting mechanism 195, and a stirring rod 196. The first stirring support plate 191 and the second stirring support plate 192 can have a supporting function. The first stirring support plate 191 is fixed onto the testing platform 101. The first stirring drive mechanism 193 is mounted on the first stirring support plate 191. The second stirring support plate 192 is movably arranged on the first stirring drive mechanism 193. The second stirring drive mechanism 194 is mounted on the second stirring support plate 192. The stirring mounting mechanism 195 is mounted on the second stirring drive mechanism 194, and the stirring rod 196 is mounted on the stirring mounting mechanism 195. The first stirring drive mechanism 193 can drive the second stirring support plate 192, and the second stirring drive mechanism 194 and the stirring mounting mechanism 195 thereon to perform a reciprocating movement in a first direction, such that the stirring rod 196 performs a reciprocating movement in the first direction. The second stirring drive mechanism 194 can drive the stirring rod 196 mounting mechanism to perform a reciprocating movement in a second direction, such that the stirring rod 196 perform a reciprocating movement in the second direction. The second direction is different from, for example, perpendicular to, the first direction. The first stirring drive mechanism 193 and the second stirring drive mechanism 194 are used to drive the movement of the stirring rod 196 in different directions, which can fully stir the reaction solution. Preferably, the first stirring drive mechanism 193 and the second stirring drive mechanism 194 are synchronous belt mechanisms. Of course, the first stirring drive mechanism 193 and the second stirring drive mechanism 194 may also be lead screw drives, belt drives, etc.

The second stirring drive mechanism 194 comprises a second stirring drive motor 1941 and a second stirring transmission component 1942. The second stirring drive motor 1941 is arranged on the second stirring mounting plate. The second stirring drive motor 1941 is in driving connection with the stirring mounting mechanism 195 via the second stirring transmission component 1942, to drive the stirring mounting mechanism 195 so as to drive the stirring rod 196 to perform a reciprocating movement in the second direction. In this embodiment, the structure of the first stirring drive mechanism 193 is the same as that of the second stirring drive mechanism 194. Of course, the structure of the first stirring drive mechanism 193 may also be different from that of the second stirring drive mechanism 194. The stirring mounting mechanism 195 comprises a stirring shaft 1951 and a stirring cantilever 1952 provided on the stirring shaft 1951. One end of the stirring shaft 1951 is connected to the second stirring drive mechanism 194, and the other end of the stirring shaft 1951 extends vertically. One end of the stirring cantilever 1952 is mounted to the extended end of the stirring shaft 1951, and the other end of the stirring cantilever 1952 extends toward the end away from the stirring shaft 1951. The stirring rod 196 is mounted on the end of the stirring cantilever 1952 that is away from the stirring shaft 1951.

The blood analyzer 1 of the present invention can further mix the sample and the reagent in the sample incubation module 14 by the stirring module 19. Specifically, the first stirring drive mechanism 193 and the second stirring drive mechanism 194 drive the stirring mounting mechanism 195 to move, such that the stirring rod 196 on the stirring mounting mechanism 195 moves to the stirring position and extends into the reaction cup in the sample incubation module 14. Subsequently, both the first stirring drive mechanism 193 and the second stirring drive mechanism 194 move, which in turn makes the stirring rod 196 move in different directions to fully mix the sample and the reagent, ensuring the even mixing.

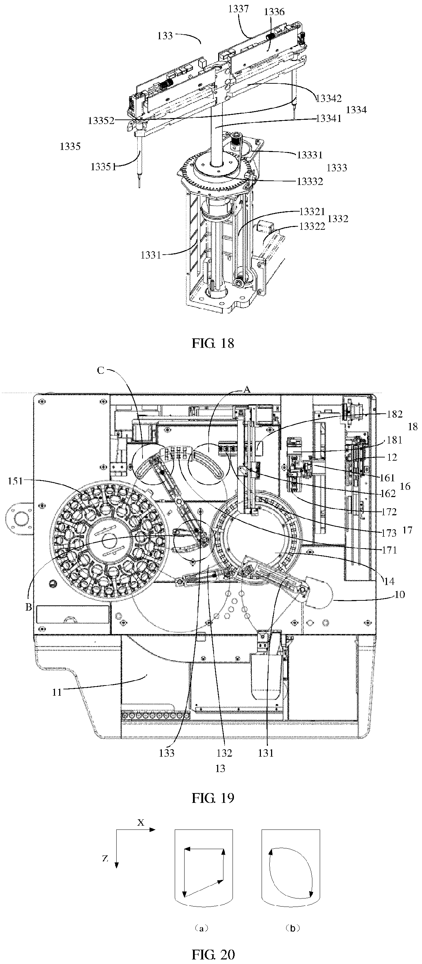

Referring to Figs. 15 and 20, Fig. 20 shows schematic views of movement trajectories of the stirring rod 196 of the stirring module 19 shown in Fig. 15. Preferably, when the test item of the sample is detected using the magnetic bead method, the stirring rod 196 stops at the original position, and stirring is not performed. When the test item of the sample is detected using the optical method, the stirring rod 196 performs a two-dimensional movement in the vertical plane. Specifically, referring to Fig. 20 (a), when the test item of the sample is detected using the chromogenic substrate method of the optical method, the information of the movement trajectory of the stirring rod 196 is as follows: after extending into the reaction cup, the stirring rod 196 starts from a stirring start point, first moves linearly by a distance in an oblique upward direction, then moves upwardly and linearly by a distance in the vertical direction, then moves back linearly in the horizontal direction to directly above the stirring start point, and then moves downwardly and linearly in the vertical direction to return to the stirring start point, thereby completing the movement trajectory of the stirring rod 196. Referring to Fig. 20 (a), when the test item of the sample is detected using the immunoturbidimetric method of the optical method, the information of the movement trajectory of the stirring rod 196 is as follows: after extending into the reaction cup, the stirring rod 196 starts from a stirring start point, first moves by a distance in an oblique downward direction with a convex curve trajectory, then moves in an oblique upward direction with a concave curve trajectory to return to the stirring start point, thereby completing the movement trajectory of the stirring rod 196.

The inventors have found after researches that the movement trajectory of the two-dimensional moving stirring rod 196 in the vertical plane can generate eddy currents over as long a distance as possible, and that the stirring efficiency in Fig. 20(a) is higher whereas fewer bubbles are generated during the stirring process in Fig. 20 (b) by comparing the two two-dimensional movement trajectories in the vertical plane in Figs. 20(a) and 20(b). Therefore, the movement trajectory of the stirring rod 196 shown in Fig. 20(a) is suitable for stirring a reaction solution using a detection methodology that is not sensitive to bubbles but has a strict time requirement, such as the chromogenic substrate method described above, whereas Fig. 20 (b) is suitable for stirring a reaction solution using a detection methodology that is sensitive to bubbles, such as the immunoturbidimetric method described above.

In one embodiment of the present invention, the blood analyzer 1 uses the puncture needle mechanism 131, the integrated needle mechanism 132 and the reagent needle mechanism 133 to transfer the sample and the reagent.

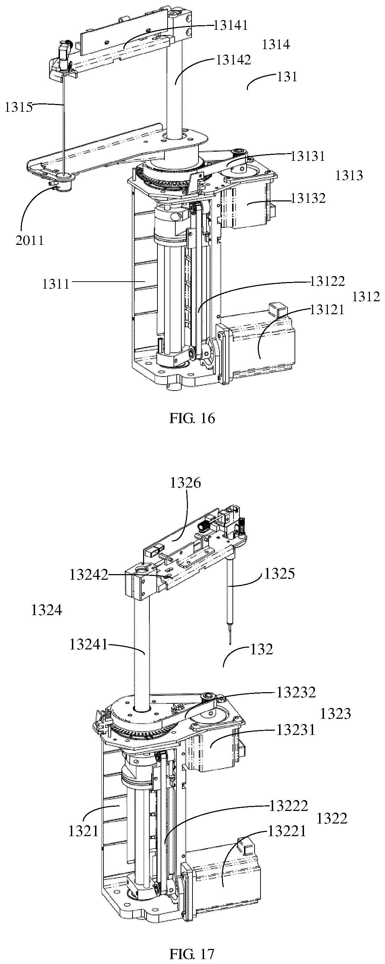

Referring to Figs. 1 to 3 and Fig. 16, the puncture needle mechanism 131 is used to pierce a sealed container, such as a test tube with a test tube cap, to aspirate in the sample. Of course, the puncture needle mechanism 131 can also aspirate in the sample from an open container, such as test tube without a test tube cap. The puncture needle mechanism 131 comprises a puncture needle support frame 1311, a puncture needle lifting drive assembly 1312, a puncture needle rotation drive assembly 1313, a puncture needle mounting assembly 1314, and a puncture needle 1315. The puncture needle support frame 1311 can have a supporting function. The puncture needle support frame 1311 is fixedly mounted on the testing platform 101, and is used to support the puncture needle lifting drive assembly 1312, the puncture needle rotation drive assembly 1313, the puncture needle mounting assembly 1314 and the puncture needle 1315 on the testing platform 101. The puncture needle 1315 is used to aspirate in and discharge the sample. The puncture needle mounting assembly 1314 is used to mount the puncture needle 1315, and is mounted on the puncture needle support frame 1311. The puncture needle lifting drive assembly 1312 is connected to the puncture needle mounting assembly 1314. The puncture needle lifting drive assembly 1312 can drive the puncture needle mounting assembly 1314 to move up and down, which in turn drives the movement of the puncture needle 1315 for sample suction and discharge operations. The puncture needle rotation drive assembly 1313 is also connected to the puncture needle mounting assembly 1314. The puncture needle rotation drive assembly 1313 can drive the puncture needle mounting assembly 1314 to perform a rotational movement, which in turn moves the puncture needle 1315 to the puncture position of the sample transport module 11 or the sample adding position of the sample incubation module 14.

Specifically, the puncture needle lifting drive assembly 1312 comprises a puncture needle lifting drive motor 13121 and a puncture needle lifting transmission component 13122. The puncture needle lifting drive motor 13121 is mounted on the puncture needle support frame 1311. The puncture needle lifting transmission component 13122 is mounted to the puncture needle lifting drive motor 13121 and the puncture needle mounting assembly 1314. An output shaft of the puncture needle lifting drive motor 13121 is in driving connection with the puncture needle mounting assembly 1314 via the puncture needle lifting transmission component 13122, to drive the puncture needle mounting assembly 1314 to move up and down, which in turn moves the puncture needle 1315 up and down. The puncture needle rotation drive assembly 1313 comprises a puncture needle rotation drive motor 13131 and a puncture needle rotation transmission component 13132. The puncture needle rotation drive motor 13131 is mounted on the puncture needle support frame 1311. The puncture needle rotation transmission component 13132 is mounted to the puncture needle rotation drive motor 13131 and the puncture needle mounting assembly 1314. An output shaft of the puncture needle rotation drive motor 13131 is in driving connection with the puncture needle mounting assembly 1314 via the puncture needle rotation transmission component 13132, to drive the puncture needle mounting assembly 1314 to perform a rotational movement, which in turn drives the puncture needle 1315 to rotate. The puncture needle mounting assembly 1314 comprises a puncture needle rotating shaft 13141 and a puncture needle rotating arm 13142. One end of the puncture needle rotating shaft 13141 is connected to the puncture needle rotation transmission component 13132 and the puncture needle lifting transmission component 13122, and the other end of the puncture needle rotating shaft 13141 extends vertically. One end of the puncture needle rotating arm 13142 is mounted to the extended end of the puncture needle rotating shaft 13141, and the other end of the puncture needle rotating arm 13142 extends in a direction facing away from the puncture needle rotating shaft 13141. The puncture needle 1315 is arranged on the end of the puncture needle rotating arm 13142 that is away from the puncture needle rotating shaft 13141. The puncture needle lifting transmission component 13122 and the puncture needle rotation transmission component 13132 may be synchronous belt conveying structures, and may also be chain drive structures and other structures to achieve the transmission of movement, of course.

When the sample is transferred by the puncture needle mechanism 131, the puncture needle rotation drive assembly 1313 drives the puncture needle rotating shaft 13141 to rotate the puncture needle rotating arm 13142, and the puncture needle rotating arm 13142 in turn drives the puncture needle 1315 to rotate to the puncture position of the sample transport module 11. The puncture lifting assembly drives the puncture needle rotating shaft 13141 to lower the puncture needle rotating arm 13142, and the puncture needle rotating arm 13142 in turn lowers the puncture needle 1315. The puncture needle 1315 aspirates in the sample at the puncture position. After the sample has been aspirated, the puncture lifting assembly drives the puncture needle rotating shaft 13141 to raise the puncture needle rotating arm 13142, and the puncture needle rotating arm 13142 in turn raises the puncture needle 1315. The puncture needle rotation drive assembly 1313 drives the puncture needle rotating shaft 13141 to rotate the puncture needle rotating arm 13142, and the puncture needle rotating arm 13142 in turn drives the puncture needle 1315 to rotate to the sample adding position of the sample incubation module 14. The puncture lifting assembly drives the puncture needle rotating shaft 13141 to lower the puncture needle rotating arm 13142, and the puncture needle rotating arm 13142 in turn lowers the puncture needle 1315. At the sample adding position, the puncture needle 1315 adds the sample to the reaction cup in the sample incubation module 14. After the sample has been added, the puncture lifting assembly drives the puncture needle rotating shaft 13141 to raise the puncture needle rotating arm 13142, and the puncture needle rotating arm 13142 in turn raises the puncture needle 1315.

Referring to Figs. 1 to 3 and Fig. 17, the integrated needle mechanism 132 is used to aspirate in a sample from an open container, such as a test tube without a test tube cap, or to aspirate in a reagent from a reagent bottle. The integrated needle mechanism 132 comprises an integrated needle support frame 1321, an integrated needle lifting drive assembly 1322, an integrated needle rotation drive assembly 1323, an integrated needle mounting assembly 1324, and an integrated needle 1325 having a heating function. Of course, in other implementations of the present invention, the integrated needle 1325 may have no heating function. The integrated needle support frame 1321 can have a supporting function. The integrated needle support frame 1321 is fixedly mounted on the testing platform 101, and is used to support the integrated needle lifting drive assembly 1322, the integrated needle rotation drive assembly 1323, the integrated needle mounting assembly 1324 and the integrated needle 1325 on the testing platform 101. The integrated needle 1325 is used to aspirate in and discharge the sample and the reagent. The integrated needle mounting assembly 1324 is used to mount the integrated needle 1325, and is mounted on the integrated needle support frame 1321. The integrated needle lifting drive assembly 1322 is connected to the integrated needle mounting assembly 1324. The integrated needle lifting drive assembly 1322 can drive the integrated needle mounting assembly 1324 to move up and down, which in turn drives the movement of the integrated needle 1325 for sample or reagent suction and discharge operations. The integrated needle rotation drive assembly 1323 is also connected to the integrated needle mounting assembly 1324. The integrated needle rotation drive assembly 1323 can drive the integrated needle mounting assembly 1324 to perform a rotational movement, which in turn moves the integrated needle 1325 to the non-puncture position of the sample transport module 11, the first reagent aspirating position of the first reagent storage mechanism 151 or the second reagent adding position of the sample incubation module 14.

Specifically, the integrated needle lifting drive assembly 1322 comprises an integrated needle lifting drive motor 13221 and an integrated needle lifting transmission component 13222. The integrated needle lifting drive motor 13221 is mounted on the integrated needle support frame 1321. The integrated needle lifting transmission component 13222 is mounted to the integrated needle lifting drive motor 13221 and the integrated needle mounting assembly 1324. An output shaft of the integrated needle lifting drive motor 13221 is in driving connection with the integrated needle mounting assembly 1324 via the integrated needle lifting transmission component 13222, to drive the integrated needle mounting assembly 1324 to move up and down, which in turn moves the integrated needle 1325 up and down. The integrated needle rotation drive assembly 1323 comprises an integrated needle rotation drive motor 13231 and an integrated needle rotation transmission component 13232. The integrated needle rotation drive motor 13231 is mounted on the integrated needle support frame 1321. The integrated needle rotation transmission component 13232 is mounted to the integrated needle rotation drive motor 13231 and the integrated needle mounting assembly 1324. An output shaft of the integrated needle rotation drive motor 13231 is in driving connection with the integrated needle mounting assembly 1324 via the integrated needle rotation transmission component 13232, to drive the integrated needle mounting assembly 1324 to perform a rotational movement, which in turn drives the integrated needle 1325 to rotate. The integrated needle mounting assembly 1324 comprises an integrated needle rotating shaft 13241 and an integrated needle rotating arm 13242. One end of the integrated needle rotating shaft 13241 is connected to the integrated needle rotation transmission component 13232 and the integrated needle lifting transmission component 13222, and the other end of the integrated needle rotating shaft 13241 extends vertically. One end of the integrated needle rotating arm 13242 is mounted to the extended end of the integrated needle rotating shaft 13241, and the other end of the integrated needle rotating arm 13242 extends in a direction facing away from the integrated needle rotating shaft 13241. The integrated needle 1325 is arranged on the end of the integrated needle rotating arm 13242 that is away from the integrated needle rotating shaft 13241. The integrated needle lifting transmission component 13222 and the integrated needle rotation transmission component 13232 may be synchronous belt conveying structures, and may also be chain drive structures and other structures to achieve the transmission of movement, of course.

When the sample is transferred by the integrated needle mechanism 132, the integrated needle lifting drive assembly 1322 drives the integrated needle rotating shaft 13241 to rotate the integrated needle rotating arm 13242, and the integrated needle rotating arm 13242 in turn drives the integrated needle 1325 to rotate to the non-puncture position of the sample transport module 11. The integrated needle lifting drive assembly 1322 drives the integrated needle rotating shaft 13241 to lower the integrated needle rotating arm 13242, and the integrated needle rotating arm 13242 in turn lowers the integrated needle 1325. The integrated needle 1325 aspirates in the sample at the non-puncture position. After the sample has been aspirated, the integrated needle lifting drive assembly 1322 drives the integrated needle rotating shaft 13241 to raise the integrated needle rotating arm 13242, and the integrated needle rotating arm 13242 in turn raises the integrated needle 1325. The integrated needle rotation drive assembly 1323 drives the integrated needle rotating shaft 13241 to rotate the integrated needle rotating arm 13242, and the integrated needle rotating arm 13242 in turn drives the integrated needle 1325 to rotate to the sample adding position of the sample incubation module 14. The integrated needle lifting drive assembly 1322 drives the integrated needle rotating shaft 13241 to lower the integrated needle rotating arm 13242, and the integrated needle rotating arm 13242 in turn lowers the integrated needle 1325. At the sample adding position, the integrated needle 1325 adds the sample to the reaction cup in the sample incubation module 14. After the sample has been added, the integrated needle lifting drive assembly 1322 drives the integrated needle rotating shaft 13241 to raise the integrated needle rotating arm 13242, and the integrated needle rotating arm 13242 in turn raises the integrated needle 1325.

When the reagent is transferred by the integrated needle mechanism 132, the integrated needle lifting drive assembly 1322 drives the integrated needle rotating shaft 13241 to rotate the integrated needle rotating arm 13242, and the integrated needle rotating arm 13242 in turn drives the integrated needle 1325 to rotate to the first reagent aspirating position of the first reagent storage mechanism 151. The integrated needle lifting drive assembly 1322 drives the integrated needle rotating shaft 13241 to lower the integrated needle rotating arm 13242, and the integrated needle rotating arm 13242 in turn lowers the integrated needle 1325. The integrated needle 1325 aspirates in the reagent at the first reagent aspirating position. After the reagent has been aspirated, the integrated needle lifting drive assembly 1322 drives the integrated needle rotating shaft 13241 to raise the integrated needle rotating arm 13242, and the integrated needle rotating arm 13242 in turn raises the integrated needle 1325. The integrated needle rotation drive assembly 1323 drives the integrated needle rotating shaft 13241 to rotate the integrated needle rotating arm 13242, and the integrated needle rotating arm 13242 in turn drives the integrated needle 1325 to rotate to the sample adding position of the sample incubation module 14. The integrated needle lifting drive assembly 1322 drives the integrated needle rotating shaft 13241 to lower the integrated needle rotating arm 13242, and the integrated needle rotating arm 13242 in turn lowers the integrated needle 1325. At the filling position, the integrated needle 1325 adds the reagent to the reaction cup in the sample incubation module 14. After the reagent has been added, the integrated needle lifting drive assembly 1322 drives the integrated needle rotating shaft 13241 to raise the integrated needle rotating arm 13242, and the integrated needle rotating arm 13242 in turn raises the integrated needle 1325.

The integrated needle mechanism 132 further comprises an integrated needle heating control board and an integrated needle level detection board 1326. The integrated needle 1325 is internally provided with a heating component. The integrated needle heating control board is electrically connected to the heating component, and the integrated needle heating control board controls the heating component such that same performs a heating operation. When the integrated needle 1325 aspirates in a reagent refrigerated in the first reagent storage mechanism 151, the heating component can heat the low-temperature reagent to a pre-set temperature, and then add the heated reagent to the reaction cup in the sample incubation module 14 to ensure the reaction effect. When the integrated needle 1325 aspirates in the sample, the integrated needle heating control board no longer controls the heating of the heating component. The integrated needle 1325 transfers the normal-temperature sample to the reaction cup in the sample incubation module 14. When the integrated needle 1325 aspirates in a normal-temperature reagent from the second reagent storage mechanism 152, the integrated needle heating control board no longer controls the heating of the heating component. The integrated needle 1325 transfers the normal-temperature reagent to the reaction cup in the sample incubation module 14. The integrated needle 1325 is electrically connected to the integrated needle level detection board 1326. When the integrated needle 1325 aspirates in a reagent from the reagent bottle, the integrated needle level detection board 1326 can control the integrated needle 1325 so as to detect the level of the reagent in the reagent bottle, thereby avoiding the lack of the reagent, which affects the operation.

Referring to Figs. 1 to 3 and Fig. 18, the reagent needle mechanism 133 comprises a reagent needle support frame 1331, reagent needle drive assemblies, a reagent needle mounting assembly 1334 and two reagent needles 1335. The reagent needle drive assemblies are in driving connection with the reagent needle mounting assembly 1334, and the two reagent needles 1335 are arranged on the reagent needle mounting assembly 1334. The reagent needle drive assemblies drive the reagent needle mounting assembly 1334 to move, such that the two reagent needles 1335 can move to the reagent aspirating position of the reagent storage module 15, the first reagent adding position of the sample incubation module 14 and the third reagent adding position of the magnetic bead detection mechanism 171. There are two reagent needle drive assemblies, which are respectively a reagent needle lifting drive assembly 1332 and reagent needle rotation drive assembly 1333. The reagent needle support frame 1331 can have a supporting function. The reagent needle support frame 1331 is fixedly mounted on the testing platform 101, and is used to support the reagent needle lifting drive assembly 1332, the reagent needle rotation drive assembly 1333, the reagent needle mounting assembly 1334 and the reagent needle 1335 on the testing platform 101. The reagent needle 1335 is used to aspirate in and discharge the reagent. The reagent needle mounting assembly 1334 is used to mount the reagent needle 1335, and is mounted on the reagent needle support frame 1331. The reagent needle lifting drive assembly 1332 is connected to the reagent needle mounting assembly 1334. The reagent needle lifting drive assembly 1332 can drive the reagent needle mounting assembly 1334 to move up and down, which in turn drives the movement of the reagent needle 1335 for reagent suction and discharge operations. The reagent needle rotation drive assembly 1333 is also connected to the reagent needle mounting assembly 1334. The reagent needle rotation drive assembly 1333 can drive the reagent needle mounting assembly 1334 to perform a rotational movement, which in turn moves the reagent needle 1335 to the second reagent aspirating position of the first reagent storage mechanism 151, the first reagent adding position of the sample incubation module 14 or the third reagent adding position of the magnetic bead detection mechanism 171.

Specifically, the reagent needle lifting drive assembly 1332 comprises a reagent needle lifting drive motor 13321 and a reagent needle lifting transmission component 13322. The reagent needle lifting drive motor 13321 is mounted on the reagent needle support frame 1331. The reagent needle lifting transmission component 13322 is mounted to the reagent needle lifting drive motor 13321 and the reagent needle mounting assembly 1334. An output shaft of the reagent needle lifting drive motor 13321 is in driving connection with the reagent needle mounting assembly 1334 via the reagent needle lifting transmission component 13322, to drive the reagent needle mounting assembly 1334 to move up and down, which in turn moves the reagent needle 1335 up and down. The reagent needle rotation drive assembly 1333 comprises a reagent needle rotation drive motor 13331 and a reagent needle rotation transmission component 13332. The reagent needle rotation drive motor 13331 is mounted on the reagent needle support frame 1331. The reagent needle rotation transmission component 13332 is mounted to the reagent needle rotation drive motor 13331 and the reagent needle mounting assembly 1334. An output shaft of the reagent needle rotation drive motor 13331 is in driving connection with the reagent needle mounting assembly 1334 via the reagent needle rotation transmission component 13332, to drive the reagent needle mounting assembly 1334 to perform a rotational movement, which in turn drives the reagent needle 1335 to rotate. The reagent needle mounting assembly 1334 comprises a reagent needle rotating shaft 13341 and two reagent needle rotating arms 13342. One end of the reagent needle rotating shaft 13341 is connected to the reagent needle rotation transmission component 13332 and the reagent needle lifting transmission component 13322, and the other end of the reagent needle rotating shaft 13341 extends vertically. One end of each of the two reagent needle rotating arms 13342 is mounted to the extended end of the reagent needle rotating shaft 13341, and the other end of each of the two reagent needle rotating arms 13342 extends in a direction facing away from the reagent needle rotating shaft 13341. Two reagent needles 1335 are respectively arranged on the end of each of the two reagent needle rotating arms 13342 that is away from the reagent needle rotating shaft 13341. The reagent needle lifting transmission component 13322 and the reagent needle rotation transmission component 13332 may be synchronous belt conveying structures, and may also be chain drive structures and other structures to achieve the transmission of movement, of course.

Moreover, at least one of the reagent needles 1335 has a heating function. That is, the reagent in the at least one of the reagent needles 1335 can be heated by the reagent needle 1335 via a reagent needle 1335 heating assembly. The reagent needle 1335 heating assembly can heat the reagent in one of the reagent needles 1335 alone or heat the reagents in the two reagent needles 1335. Preferably, the two reagent needles 1335 both have a heating function, that is, the reagent needle 1335 heating assembly comprises two reagent needle 1335 heating components. The two reagent needle 1335 heating components are respectively provided in the two reagent needles 1335 for heating the reagents in the reagent needles 1335. After the reagent needle 1335 aspirates in the reagent from the reagent storage mechanism, the reagent is stored inside the reagent needle 1335, and the reagent needle 1335 heats the reagent via the reagent needle 1335 heating component inside the reagent needle. After the temperature of the reagent reaches a pre-set temperature, the reagent is added to the reaction cup in the sample incubation module 14 or the reaction cup in the magnetic bead detection mechanism 171, the reaction speed between the sample and the reagent is increased, and the analysis efficiency of the blood analyzer 1 is improved. In addition, the reagent needle 1335 heating component can quickly and accurately heat the reagent in the reagent needle 1335, and keep the reagent at the pre-set temperature to ensure the accuracy and efficiency of the test result.

Preferably, after one of the reagent needles 1335 finishes aspirating the reagent, the reagent needle rotation drive assembly 1333 then drives the reagent needle mounting assembly 1334 to rotate, and this reagent needle 1335 can heat the reagent therein at any position. At the same time, under the drive of the reagent needle rotation drive assembly 1333, the other reagent needle 1335 can be rotated to the second reagent aspirating position in the first reagent storage mechanism 151 to aspirate in the reagent, to the first reagent adding position of the sample incubation module 14 to add the reagent, or to the third reagent adding position on the magnetic bead detection mechanism 171 to disgorge the reagent, or the reagent in this reagent needle 1335 can also be heated. That is, when one of the reagent needles 1335 is performing a heating operation, the other reagent needle 1335 may perform one or more of the following operations: aspirating the reagent, heating the reagent, and disgorging the reagent. Similarly, when the other reagent needle 1335 is heating the reagent therein, the one of the reagent needles 1335 can also perform the above operation(s). That is, when the other reagent needles 1335 is performing a heating operation, the one of the reagent needles 1335 may perform one or more of the following operations: aspirating the reagent, heating the reagent, and disgorging the reagent.

When the sample is transferred by the reagent needle mechanism 133, the reagent needle rotation drive assembly 1333 drives the reagent needle rotating shaft 13341 to rotate the reagent needle rotating arm 13342, and the reagent needle rotating arm 13342 in turn drives the reagent needle 1335 to rotate to the second reagent aspirating position of the first reagent storage mechanism 151. The reagent needle lifting drive assembly 1332 drives the reagent needle rotating shaft 13341 to lower the reagent needle rotating arm 13342, and the reagent needle rotating arm 13342 in turn lowers the reagent needle 1335. The reagent needle 1335 aspirates in the reagent at the second reagent aspirating position. After the reagent has been aspirated, the reagent needle lifting drive assembly 1332 drives the reagent needle rotating shaft 13341 to raise the reagent needle rotating arm 13342, and the reagent needle rotating arm 13342 in turn raises the reagent needle 1335. The reagent needle rotation drive assembly 1333 drives the reagent needle rotating shaft 13341 to rotate the reagent needle rotating arm 13342, and the reagent needle rotating arm 13342 in turn drives the reagent needle 1335 to rotate to the first reagent adding position of the sample incubation module 14. The reagent needle lifting drive assembly 1332 drives the reagent needle rotating shaft 13341 to lower the reagent needle rotating arm 13342, and the reagent needle rotating arm 13342 in turn lowers the reagent needle 1335. At the first reagent adding position, the reagent needle 1335 adds the reagent to the reaction cup in the sample incubation module 14. After the reagent has been added, the reagent needle lifting drive assembly 1332 drives the reagent needle rotating shaft 13341 to raise the reagent needle rotating arm 13342, and the reagent needle rotating arm 13342 in turn raises the reagent needle 1335. Alternatively, after the reagent has been aspirated, the reagent needle rotation drive assembly 1333 drives the reagent needle rotating shaft 13341 to rotate the reagent needle rotating arm 13342, and the reagent needle rotating arm 13342 in turn drives the reagent needle 1335 to rotate to the third reagent adding position of the magnetic bead detection mechanism 171. The reagent needle lifting drive assembly 1332 drives the reagent needle rotating shaft 13341 to lower the reagent needle rotating arm 13342, and the reagent needle rotating arm 13342 in turn lowers the reagent needle 1335. At the third reagent adding position, the reagent needle 1335 adds the reagent to the reaction cup in the magnetic bead detection mechanism 171. After the reagent has been added, the reagent needle lifting drive assembly 1332 drives the reagent needle rotating shaft 13341 to raise the reagent needle rotating arm 13342, and the reagent needle rotating arm 13342 in turn raises the reagent needle 1335.

The reagent needle mechanism 133 further comprises a reagent needle heating control board 1336. The reagent needle 1335 is internally provided with a heating component. The reagent needle heating control board 1336 is electrically connected to the heating component, and the reagent needle heating control board 1336 controls the heating component such that same performs a heating operation. When the reagent needle 1335 aspirates in a reagent refrigerated in the first reagent storage mechanism 151, the heating component can heat the low-temperature reagent to a pre-set temperature, and then add the heated reagent to the reaction cup in the sample incubation module 14 to ensure the reaction effect. The reagent needle mechanism 133 further comprises a reagent needle level detection board 1337. The reagent needle level detection board 1337 can detect the level of the reagent in the reagent bottle of the second reagent storage mechanism 152. When the level of the reagent in the reagent bottle is lower than a pre-set value, the reagent needle level detection board 1337 can feedback the information of too little reagent, prompting the operator to replace or add the reagent, thereby preventing the insufficient reagent from affecting the detection of the sample, and improving the detection efficiency.

Further, the two reagent needle rotating arms 13342 are arranged collinearly, and the two reagent needles 1335 are respectively arranged at the ends of the two reagent needle rotating arms 13342. That is, the two reagent needles 1335 are arranged at ° at the two reagent needle rotating arms 13342. The space where one of the reagent needles 1335 is located when the other reagent needle 1335 is performing the operation of aspirating the reagent or disgorging the reagent is exactly the same as the space where the other reagent needle 1335 is located when the one of the reagent needles 1335 is performing the operation of aspirating the reagent or disgorging the reagent, facilitating the design of an avoidance space. This can facilitate the positional arrangement and rotation control settings of the reagent needle mechanism 133, and optimize the overall structure of the blood analyzer 1, so that the positions of the two reagent needles 1335 are more controllable, avoiding interference during the rotation process, and facilitating the control over the reagent needle mechanism 133. Of course, the angle of the two reagent needles 1335 may be any angle between 30° and 180°, as long as the positional arrangement and rotation control settings of the reagent needle mechanism 133 can be guaranteed, thereby preventing the reagent needles 1335 from colliding with structures such as the first reagent storage mechanism 151 and the sample incubation module 14 when aspirating and discharging the reagent, and improving the use performance.

Each of the reagent needles 1335 functions cyclically in the order of cleaning, aspirating the reagent, heating the reagent, and disgorging the reagent. When one of the reagent needles 1335 is heating the reagent, the other reagent needle 1335 may perform one of or a combination of the following operations: cleaning, aspirating the reagent, heating the reagent, and disgorging the reagent. In order to facilitate the description of the reagent transfer of the reagent needle mechanism 133, the two reagent needles 1335 are respectively defined as a first reagent needle 13351 and a second reagent needle 13352. The structure of the first reagent needle 13351 is exactly the same as that of the second reagent needle 13352. When the reagent is transferred by the reagent needle mechanism 133, the first reagent needle 13351 is cleaned and is then rotated to the second reagent aspirating position of the first reagent storage mechanism 151 to aspirate in the reagent, and after the reagent has been aspirated, the first reagent needle 13351 heats the reagent; whereas, the second reagent needle 13352 is rotated to the corresponding position to perform the cleaning operation, the second reagent needle 13352 is then rotated to the second reagent aspirating position of the first reagent storage mechanism 151 to aspirate in the reagent, and then the second reagent needle 13352 heats the reagent. In addition, after the reagent in first reagent needle 13351 reaches a pre-set temperature, the first reagent needle 13351 is rotated to the first reagent adding position of the sample incubation module 14 or the third reagent adding position of the sample detection module 17 to add the reagent. Subsequently, the first reagent needle 13351 is rotated to the corresponding position to perform the cleaning operation, the first reagent needle 13351 is then rotated to the second reagent aspirating position of the first reagent storage mechanism 151 to aspirate in the reagent, and then the first reagent needle 13351 heats the reagent; whereas after the reagent in second reagent needle 13352 reaches a pre-set temperature, the second reagent needle 13352 is rotated to the second reagent adding position of the sample incubation module 14 or the third reagent adding position of sample detection module 17 to add the reagent.

Of course, in other implementations of the present invention, the reagent needle lifting drive assembly 1332 and the reagent needle rotation drive assembly 1333 can also separately drive the two reagent needles 1335. That is, when the reagent needle lifting drive assembly 1332 and the reagent needle rotation drive assembly 1333 drives one of the reagent needles 1335 to rotate to perform one or more of the following operations: aspirating the reagent, heating the reagent and disgorging the reagent, the other reagent needle 1335 is in a stationary state and heats the reagent. On the contrary, when the reagent needle lifting drive assembly 1332 and the reagent needle rotation drive assembly 1333 drives one of the reagent needles 1335 to rotate to perform one or more of the following operations: aspirating the reagent, heating the reagent and disgorging the reagent, the other reagent needle 1335 heats the reagent.

It should be noted that, referring to Fig. 1, the blood analyzer 1 further comprises a cleaning module 20. The cleaning module 20 comprises a puncture needle cleaning mechanism 201 for cleaning the puncture needle 1315 of the puncture needle mechanism 131, an integrated needle cleaning mechanism 202 for the integrated needle 1325 of the integrated needle mechanism 132, a reagent needle cleaning mechanism 203 for cleaning the two reagent needles 1335 of the reagent needle mechanism 133, and a stirring rod cleaning mechanism 204 for cleaning the stirring rod 196 of the stirring module 19. It is necessary to clean the puncture needle 1315 either before the puncture needle 1315 aspirates in the sample or after same disgorges the sample, to avoid contamination of the sample with the puncture needle 1315, which affects the detection. It is necessary to clean the integrated needle 1325 either before the integrated needle 1325 aspirates in the sample and the reagent or after same disgorges the sample and the reagent, to avoid contamination of the sample or the reagent with the integrated needle 1325, which affects the detection. It is necessary to clean the reagent needle 1335 either before the reagent needle 1335 aspirates in the sample or after same disgorges the sample, to avoid contamination of the reagent and the sample with the reagent needle 1335, which affects the detection. It is necessary to clean the stirring rod 196 either before or after the stirring rod 196 performs stirring, to avoid contamination of the reagent and the sample with the stirring rod 196, which affects the detection. The puncture needle cleaning mechanism 201 is arranged between the sample incubation module 14 and the sample transport module 11. The integrated needle cleaning mechanism 202 is arranged between the sample incubation module 14 and the sample transport module 11, and the integrated needle cleaning mechanism 202 is arranged adjacent to the puncture needle cleaning mechanism 201. The reagent needle cleaning mechanism 203 is arranged between the reagent storage module 15 and the sample incubation module 14. The stirring rod cleaning mechanism 204 is arranged between the reagent storage module 15 and the sample incubation module 14.

The reagent needle cleaning mechanism 203 comprises a reagent needle liquid supply assembly, a reagent needle cleaning pool and a reagent needle drainage pipeline. The overall contour of the reagent needle cleaning pool is in the shape of a cuboid, and the reagent needle cleaning pool is arranged between the sample incubation module 14 and the first reagent storage mechanism 151. The reagent needle liquid supply assembly is connected to the reagent needle 1335. The reagent needle liquid supply assembly is used to inject a cleaning liquid into an inner cavity of the reagent needle 1335. The reagent needle cleaning pool is internally provided with a reagent needle cleaning tank for accommodating the reagent needle 1335. The reagent needle drainage pipeline is connected to a lower part of the reagent needle cleaning tank. The reagent needle drainage pipeline is provided with a reagent needle suction power component. When the reagent needle 1335 is being cleaned, the reagent needle 1335 extends into the reagent needle cleaning tank, and the reagent needle 1335 liquid supply pipeline leads the cleaning liquid to the interior of the reagent needle 1335, and the cleaning liquid in turn enters the reagent needle cleaning pool through the reagent needle 1335. In this way, the interior and exterior of the reagent needle 1335 can be cleaned. The reagent needle suction power component can provide cleaning power to ensure clean cleaning. After the cleaning is completed, the reagent needle suction power component aspirates the cleaning liquid out of the reagent needle cleaning tank and the interior of the reagent needle 1335.

Optionally, the reagent needle liquid supply assembly comprises a reagent needle main cleaning pipe and two reagent needle branch cleaning pipes. The reagent needle main cleaning pipe and the two reagent needle branch cleaning pipes are both provided on the reagent needle mounting assembly 1334. One end of each of the reagent needle branch cleaning pipes is in communication with the corresponding reagent needle 1335, and the other end of each of the reagent needle branch cleaning pipes is in communication with the main cleaning pipe. The cleaning liquid enters the corresponding reagent needle branch cleaning pipe through the reagent needle main cleaning pipe, and cleans the corresponding reagent needle 1335 to ensure the cleaning of the reagent needle 1335. In addition, the two reagent needles 1335 jointly use the reagent needle main cleaning pipe to supply the cleaning liquid to the reagent needle branch cleaning pipes, reducing the number of pipelines, thereby reducing the complexity of the structure.

Optionally, the reagent needle cleaning mechanism 203 further comprises a reagent needle control assembly. The reagent needle control assembly comprises a reagent needle main control valve and two reagent needle branch control valves. The main cleaning pipe is provided with the reagent needle main control valve, and each branch cleaning pipe is provided with one reagent needle branch control valve. When one of the reagent needles 1335 is being cleaned, the reagent needle main control valve and the reagent needle branch control valve corresponding to the reagent needle 1335 are opened, and the other reagent needle branch control valve is closed. When one of the reagent needles 1335 is aspirating or disgorging the sample, the reagent needle branch control valve corresponding to the reagent needle 1335 is opened, and the reagent needle main control valve and the other reagent needle branch control valve are closed. When one of the reagent needles 1335 is performing heating, the reagent needle main control valve and the reagent needle branch control valve corresponding to the reagent needle 1335 are closed.

In the same way, the structures of the integrated needle cleaning mechanism 202 and the stirring rod cleaning mechanism 204 are exactly the same as the structure of the reagent needle cleaning mechanism 203, and the working principles are also the same. They will not be described in detail here. Moreover, in other implementations of the present invention, the structure of the puncture needle cleaning mechanism 201 may also be exactly the same as that of the reagent needle cleaning mechanism 203, and the working principle is also the same.

In this embodiment, the puncture needle cleaning mechanism 201 comprises a puncture needle cleaning pool 2011 and a reservoir, the puncture needle cleaning pool 2011 is arranged on the puncture needle mechanism 131, and the reservoir is arranged below the puncture needle cleaning pool 2011. The puncture needle cleaning pool 2011 is used to clean the puncture needle 1315 of the puncture needle mechanism 131, and the reservoir is used to receive the cleaning liquid leaked from the puncture needle cleaning pool 2011. Moreover, the puncture needle cleaning mechanism 201 further comprises a first puncture needle liquid supply assembly, a second puncture needle liquid supply assembly, a first puncture needle drainage pipeline, a second puncture needle drainage pipeline, a first puncture needle suction power component, and a second puncture needle suction power component. The puncture needle cleaning pool 2011 is provided on the puncture needle support frame 1311 via a connection plate, the puncture needle cleaning pool 2011 is arranged corresponding to the puncture needle 1315, and the reservoir is arranged between the sample incubation module 14 and the sample transport module 11.

The first puncture needle liquid supply assembly is in communication with the interior of the puncture needle 1315 for supplying the cleaning liquid to the interior of the puncture needle 1315 to clean the interior of the puncture needle 1315. The second puncture needle liquid supply assembly is in communication with the puncture needle cleaning pool 2011. The puncture needle 1315 extends into the puncture needle cleaning pool 2011, and the second puncture needle liquid supply assembly provides the cleaning liquid to the puncture needle cleaning pool 2011 to clean the exterior of the puncture needle 1315. The first puncture needle suction power component is arranged on the first puncture needle drainage pipeline, and the second puncture needle suction power component is arranged on the second puncture needle drainage pipeline. The first puncture needle drainage pipeline is in communication with the interior of the puncture needle 1315, and the second puncture needle drainage pipeline is in communication with the interior of the puncture needle cleaning pool 2011. After the puncture needle 1315 has been cleaned, the cleaning liquid inside the puncture needle 1315 is discharged through the first puncture needle drainage pipeline, and the cleaning liquid in the puncture needle cleaning pool 2011 is discharged through the second puncture needle drainage pipeline. When the puncture needle 1315 is being cleaned, the first puncture needle suction power component can be used as the power to clean an inner wall of the puncture needle 1315, and the second puncture needle suction power component can be used as the cleaning power to clean an outer wall of the puncture needle 1315, so as to ensure clean cleaning. After the cleaning is completed, the first puncture needle suction power component and the second puncture needle suction power component can also ensure that the cleaning liquid is aspirated cleanly. The reservoir is to prevent leakage of the puncture needle cleaning pool 2011, and has a protective function. Preferably, the first puncture needle suction power component uses a diaphragm pump as a power source, and the second puncture needle suction power component uses a peristaltic pump as a power source.

Referring to Fig. 19, the reagent needle mechanism 133 has a double-ended needle structure, and the reagent needle mechanism 133 may interfere with the components such as the second transfer mechanism 162, the stirring module 19, the sample incubation module 14 and the first reagent storage mechanism 151 when transferring the reagent, causing the blood analyzer 1 to stop. Therefore, avoidance spaces are defined for the two reagent needles 1335. When one of the reagent needles 1335 is operating, the other reagent needle 1335 should be located in the avoidance space. Furthermore, the testing platform 101 is provided with grooves at the avoidance spaces. When the reagent needle 1335 moves downward under the drive of the reagent needle lifting drive assembly 1332, the reagent needle 1335 can extend into the respective groove, so that the reagent needle 1335 can be prevent from interfering with the testing platform 110 when pricking, thereby ensuring the running reliability of the reagent needle 1335.

Specifically, when one of the reagent needles 1335 aspirates in the reagent at the first reagent storage mechanism 151, the space where the other reagent needle 1335 is located is a first avoidance space. When one of the reagent needles 1335 aspirates in the reagent at the second reagent aspirating position of the first reagent storage mechanism 151, the other reagent needle 1335 has an avoidance space at part A shown in Fig. 19, and the second transfer mechanism 162 needs to avoid the reagent needle 1335 for preventing collision. The reagent needle 1335 is also prevented from interfering with the other components when moving up and down. When one of the reagent needles 1335 adds the reagent at the sample detection module 17, the space where the other reagent needle 1335 is located is a second avoidance space. When one of the reagent needles 1335 adds the reagent at the third reagent adding position of the magnetic bead detection mechanism 171, the other reagent needle 1335 has an avoidance space at part B shown in Fig. 19 for preventing collision. The reagent needle 1335 is also prevented from interfering with the other components when moving up and down. When one of the reagent needles 1335 adds the reagent at the sample incubation module 14, the space where the other reagent needle 1335 is located is a third avoidance space. When one of the reagent needles 1335 adds the reagent at the first reagent adding position of the sample incubation module 14, the other reagent needle 1335 has an avoidance space at part C shown in Fig. 19 for preventing collision. The reagent needle 1335 is also prevented from interfering with the other components when moving up and down. When one of the reagent needles 1335 is being cleaned, the space where the other reagent needle 1335 is located is a fourth avoidance space. When one of the reagent needle 1335 is being cleaned in the reagent needle cleaning mechanism 203, the other reagent needle 1335 has the avoidance space at the part C shown in Fig. 19 for preventing collision. The reagent needle 1335 is also prevented from interfering with the other components when moving up and down.

Referring to Figs. 21 to 23, in another embodiment of the present invention, the blood analyzer 1 uses the integrated needle mechanism 132 and the reagent needle mechanism 133 to transfer the sample and the reagent.

It should be noted that the specific structures and movement modes of the integrated needle mechanism 132 and the reagent needle mechanism 133 of the two-needle structure in this embodiment are exactly the same as those of the integrated needle mechanism 132 and the reagent needle mechanism 133 of the three-needle structure in the above embodiment. However, the integrated needle mechanism 132 in this embodiment assumes the task of transferring the sample; whereas the integrated needle mechanism 132 in the above embodiment may assume the task of transferring the sample or not. In addition, the integrated needle cleaning mechanism 202 for cleaning the integrated needle 1325, the reagent needle cleaning mechanism 203 for cleaning the reagent needle 1335 and the avoidance design of the reagent needle mechanism 133 are exactly the same as those in the above embodiment, and the specific structures of the integrated needle mechanism 132 and the reagent needle mechanism 133 will not be described in detail here.

In another embodiment of the present invention, the first reagent storage mechanism 151 can automatically rotate the reagent required for the detection of the sample to the reagent aspirating position. Specifically, when the integrated needle mechanism 132 is aspirating the reagent, the first reagent drive assembly 1514 of the first reagent storage mechanism 151 drives the reagent disk 1513 to rotate the reagent bottles thereon, such that the corresponding reagent bottle moves to the first reagent aspirating position, and the integrated needle 1325 aspirates in the reagent and is then moved to the second reagent adding position of the sample incubation module 14 to add the reagent. When the reagent needle 1335 is aspirating the reagent, the first reagent drive assembly 1514 of the first reagent storage mechanism 151 drives the reagent disk 1513 to rotate the reagent bottles thereon, such that the corresponding reagent bottle moves to the second reagent aspirating position, and the reagent needle 1335 aspirates in the reagent and is then moved to the first reagent adding position of the sample incubation module 14 to add the reagent.

Referring to Fig. 24, in yet another embodiment of the present invention, the blood analyzer 1 uses the dispensing needle mechanism 134 to transfer the sample and the reagent.

Specifically, the dispensing needle mechanism 134 is used to aspirate in a sample from an open container, such as a test tube without a test tube cap, or to aspirate in a reagent from a reagent bottle. The dispensing needle mechanism 134 comprises a dispensing needle support frame, a dispensing needle lifting drive assembly, a dispensing needle rotation drive assembly, a dispensing needle mounting assembly, and a dispensing needle. The dispensing needle support frame can have a supporting function. The dispensing needle support frame is fixedly mounted on the testing platform 101, and is used to support the dispensing needle lifting drive assembly, the dispensing needle rotation drive assembly, the dispensing needle mounting assembly and the dispensing needle on the testing platform 101. The dispensing needle is used to aspirate in and discharge the sample and the reagent. The dispensing needle mounting assembly is used to mount the dispensing needle, and is mounted on the dispensing needle support frame. The dispensing needle lifting drive assembly is connected to the dispensing needle mounting assembly. The dispensing needle lifting drive assembly can drive the dispensing needle mounting assembly to move up and down, which in turn drives the movement of the dispensing needle for sample or reagent suction and discharge operations. The dispensing needle rotation drive assembly is also connected to the dispensing needle mounting assembly. The dispensing needle rotation drive assembly can drive the dispensing needle mounting assembly to perform a rotational movement, which in turn moves the dispensing needle to the non-puncture position of the sample transport module 11 to aspirate in the sample, to the first reagent aspirating position of the first reagent storage mechanism 151 to aspirate in the reagent, to the filling position of the sample incubation module 14 to add the sample or the reagent, or to the third reagent adding position of the sample detection module 17 to add the reagent. Optionally, the dispensing needle may have a heating function.

Specifically, the dispensing needle lifting drive assembly comprises a dispensing needle lifting drive motor and a dispensing needle lifting transmission component. The dispensing needle lifting drive motor is mounted on the dispensing needle support frame. The dispensing needle lifting transmission component is mounted to the dispensing needle lifting drive motor and the dispensing needle mounting assembly. An output shaft of the dispensing needle lifting drive motor is in driving connection with the dispensing needle mounting assembly via the dispensing needle lifting transmission component, to drive the dispensing needle mounting assembly to move up and down, which in turn moves the dispensing needle up and down. The dispensing needle rotation drive assembly comprises a dispensing needle rotation drive motor and a dispensing needle rotation transmission component. The dispensing needle rotation drive motor is mounted on the dispensing needle support frame. The dispensing needle rotation transmission component is mounted to the dispensing needle rotation drive motor and the dispensing needle mounting assembly. An output shaft of the dispensing needle rotation drive motor is in driving connection with the dispensing needle mounting assembly via the dispensing needle rotation transmission component, to drive the dispensing needle mounting assembly to perform a rotational movement, which in turn drives the dispensing needle to rotate. The dispensing needle mounting assembly comprises a dispensing needle rotating shaft and a dispensing needle rotating arm. One end of the dispensing needle rotating shaft is connected to the dispensing needle rotation transmission component and the dispensing needle lifting transmission component, and the other end of the dispensing needle rotating shaft extends vertically. One end of the dispensing needle rotating arm is mounted to the extended end of the dispensing needle rotating shaft, and the other end of the dispensing needle rotating arm extends in a direction facing away from the dispensing needle rotating shaft. The dispensing needle is arranged on the end of the dispensing needle rotating arm that is away from the dispensing needle rotating shaft. The dispensing needle lifting transmission component and the dispensing needle rotation transmission component may be synchronous belt conveying structures, and may also be chain drive structures and other structures to achieve the transmission of movement, of course.

When the sample is transferred by the dispensing needle mechanism 134, the dispensing needle lifting drive assembly drives the dispensing needle rotating shaft to rotate the dispensing needle rotating arm, and the dispensing needle rotating arm in turn drives the dispensing needle to rotate to the non-puncture position of the sample transport module 11. The dispensing needle lifting drive assembly drives the dispensing needle rotating shaft to lower the dispensing needle rotating arm, and the dispensing needle rotating arm in turn lowers the dispensing needle. The dispensing needle aspirates in the sample at the non-puncture position. After the sample has been aspirated, the dispensing needle lifting drive assembly drives the dispensing needle rotating shaft to raise the dispensing needle rotating arm, and the dispensing needle rotating arm in turn raises the dispensing needle. The dispensing needle rotation drive assembly drives the dispensing needle rotating shaft to rotate the dispensing needle rotating arm, and the dispensing needle rotating arm in turn drives the dispensing needle to rotate to the filling position of the sample incubation module 14. The dispensing needle lifting drive assembly drives the dispensing needle rotating shaft to lower the dispensing needle rotating arm, and the dispensing needle rotating arm in turn lowers the dispensing needle. At the filling position, the dispensing needle adds the sample to the reaction cup in the sample incubation module 14. After the sample has been added, the dispensing needle lifting drive assembly drives the dispensing needle rotating shaft to raise the dispensing needle rotating arm, and the dispensing needle rotating arm in turn raises the dispensing needle.

When the reagent is transferred by the dispensing needle mechanism 134, the dispensing needle lifting drive assembly drives the dispensing needle rotating shaft to rotate the dispensing needle rotating arm, and the dispensing needle rotating arm in turn drives the dispensing needle to rotate to the reagent aspirating position of the first reagent storage mechanism 151. The dispensing needle lifting drive assembly drives the dispensing needle rotating shaft to lower the dispensing needle rotating arm, and the dispensing needle rotating arm in turn lowers the dispensing needle. The dispensing needle aspirates in the reagent at the reagent aspirating position. After the reagent has been aspirated, the dispensing needle lifting drive assembly drives the dispensing needle rotating shaft to raise the dispensing needle rotating arm, and the dispensing needle rotating arm in turn raises the dispensing needle. The dispensing needle rotation drive assembly drives the dispensing needle rotating shaft to rotate the dispensing needle rotating arm, and the dispensing needle rotating arm in turn drives the dispensing needle to rotate to the filling position of the sample incubation module 14. The dispensing needle lifting drive assembly drives the dispensing needle rotating shaft to lower the dispensing needle rotating arm, and the dispensing needle rotating arm in turn lowers the dispensing needle. At the filling position, the dispensing needle adds the reagent to the reaction cup in the sample incubation module 14. Alternatively, the dispensing needle rotation drive assembly drives the dispensing needle rotating shaft to rotate the dispensing needle rotating arm, and the dispensing needle rotating arm in turn drives the dispensing needle to rotate to the third reagent adding position of the sample detection module 17. The dispensing needle lifting drive assembly drives the dispensing needle rotating shaft to lower the dispensing needle rotating arm, and the dispensing needle rotating arm in turn lowers the dispensing needle. At the third reagent adding position, the dispensing needle adds the reagent to the reaction cup in the sample detection module 17. After the reagent has been added, the dispensing needle lifting drive assembly drives the dispensing needle rotating shaft to raise the dispensing needle rotating arm, and the dispensing needle rotating arm in turn raises the dispensing needle.

In addition, the cleaning module 20 further comprises a dispensing needle cleaning mechanism, which is used to clean the dispensing needle. It is necessary to clean the dispensing needle either before the dispensing needle aspirates in the sample and the reagent or after same disgorges the sample and the reagent, to avoid contamination of the sample or the reagent with the dispensing needle, which affects the detection. The dispensing needle cleaning mechanism is arranged between the sample incubation module 14 and the sample transport module 11. Optionally, the specific structure of the dispensing needle cleaning mechanism may be exactly the same as that of the reagent needle cleaning mechanism 203, and the working principle is also the same. Of course, the specific structure of the dispensing needle cleaning mechanism may also be exactly the same as that of the puncture needle cleaning mechanism 201, and the working principle is the same.

Referring to Fig. 1, the blood analyzer 1 further comprises a liquid circuit module 21. The liquid circuit module 21 comprises a liquid circuit control mechanism, a liquid storage mechanism and a waste liquid mechanism, which are provided in the placement cavity 104. The liquid circuit control mechanism is in communication with the liquid storage mechanism and the waste liquid mechanism respectively. The liquid circuit control mechanism is also in communication with the puncture needle cleaning mechanism 201, the integrated needle cleaning mechanism 202, the reagent needle cleaning mechanism 203, and the stirring rod cleaning mechanism 204 respectively. When the puncture needle cleaning mechanism 201 is cleaning the puncture needle 1315, the cleaning liquid is transported from the liquid storage mechanism to the puncture needle 1315 and the first puncture needle cleaning pool 2011 through the first puncture needle liquid supply assembly and the second puncture needle liquid supply assembly. After the cleaning is completed, the cleaning liquid returns to the waste liquid mechanism through the first puncture needle drainage pipeline and the second puncture needle drainage pipeline. When the reagent needle cleaning mechanism 203 is cleaning the reagent needle 1335, the cleaning liquid is transported from the liquid storage mechanism to the reagent needle 1335 and the reagent needle cleaning pool through the reagent needle liquid supply assembly. After the cleaning is completed, the cleaning liquid returns to the waste liquid mechanism through the reagent needle drainage pipeline. The connection relationships between the integrated needle cleaning mechanism 202 and the stirring rod cleaning mechanism 204 and the liquid storage mechanism and the waste liquid mechanism are the same as the connection relationships between the reagent needle cleaning mechanism 203 and the liquid storage mechanism and the waste liquid mechanism.

Optionally, the liquid circuit control mechanism can supply, in a dosed manner, the cleaning liquid to the puncture needle cleaning mechanism 201, the integrated needle cleaning mechanism 202, the reagent needle cleaning mechanism 203, and the stirring rod cleaning mechanism 204. The supply of the cleaning liquid by using a dosing component can avoid the residue and overflow of the cleaning liquid, and ensure the cleaning effect, so as to ensure accurate detection results. Preferably, the dosing component may be a syringe. Of course, the dosing component may also be other components capable of transporting the cleaning liquid in a dosed manner.

In addition, the blood analyzer 1 further comprises a rocker arm and a display mounted on the rocker arm. The rocker arm can drive the display to move, such that the display pitches, rotates horizontally, translates forward and backward, or translates up and down, to have different positions to meet the use requirements of different operators for easy use. Moreover, the display has a touch screen, and the blood analyzer 1 can be controlled via the touch screen. The display is further equipped with a keyboard and a mouse for easy operation.

Referring to Figs. 1 to 3 and Figs. 21 to 24, another implementation of the present invention further provides a blood analyzer 1, comprising a sample transport module 11 for transporting a sample to be tested, a dispensing module 13 for aspirating and discharging the sample or a reagent, a sample incubation module 14 for incubating the sample, a reagent storage module 15 for storing the reagent, a transfer module 16 for transferring a reaction cup, and a sample detection module 17 for detecting the sample. The sample incubation module 14 is provided in a disk-shaped structure. The sample incubation module 14 is provided with a plurality of placement stations 14311 for placing the reaction cup. The sample incubation module 14 can rotate and drive the reaction cup in the placement station 14311 to rotate. The reagent storage module 15 comprises a rotatable first reagent storage mechanism 151. The first reagent storage mechanism 151 can store a plurality of reagents. The sample detection module 17 comprises a magnetic bead detection mechanism 171 and an optical detection mechanism. The magnetic bead detection mechanism 171 performs a magnetic bead detection on the sample, and the optical detection mechanism performs an optical detection on the sample. The transfer module 16 and the sample detection module 17 are located on one side of the sample incubation module 14, and the transfer module 16 can transfer the reaction cup to the placement station 14311 of the sample incubation module 14. The dispensing module 13 is located between the sample incubation module 14, the first reagent storage mechanism 151, the sample transport module 11 and the sample detection module 17. The dispensing module 13 can transfer the sample from the sample transport module 11 to the reaction cup in the sample incubation module 14. The dispensing module 13 can further transfer the reagent from the first reagent storage mechanism 151 to the reaction cup in the sample incubation module 14 or to the reaction cup in the sample detection module 17. The transfer module 16 can transfer the reaction cup from the sample incubation module 14 to the magnetic bead detection mechanism 171 or the optical detection mechanism.

In this implementation, the sample transport module 11, the cup feeding module 12, the dispensing module 13, the sample incubation module 14, the reagent storage module 15, the transfer module 16 and the sample detection module 17 of the blood analyzer 1 are executed in the following order: the transfer module 16 transfers the empty reaction cup transported by the cup feeding module 12 to the placement station 14311 of the sample incubation module 14 at the cup picking-up and placement position, the sample transport module 11 transports the sample to be tested, the dispensing module 13 aspirates in the sample and then rotates to the dispensing position of the sample incubation module 14 so as to add the sample to the reaction cup in the sample incubation module 14, the dispensing module 13 aspirates in the reagent at the reagent aspirating position of the first reagent storage mechanism 151 and adds the reagent to the reaction cup in the sample incubation module 14, and after the reagent has been added to the sample cup in the sample incubation module 14 and after a period of time has elapsed, the transfer module 16 transfers the reaction cup, in which the sample and the reagent have been added, to the magnetic bead detection mechanism 171 or the optical detection mechanism for magnetic bead detection or optical detection, such that the parameter of the sample are obtained. After performing the above steps, the blood analyzer 1 of the present invention can easily implement modular operations; and it is also convenient to add modules to the blood analyzer 1 to achieve the corresponding functions, such that the blood analyzer is easily expanded, and the blood analyzer 1 of the present invention can also achieve better functions, improve the operation efficiency, and be convenient to use.

Further, the first reagent storage mechanism 151 is provided in a disk-shaped structure. The disk-shaped first reagent storage mechanism 151 can be easily rotated, and thus the reagent thereon can be rotated to the reagent aspirating position, which is convenient for the dispensing module 13 to aspirate in the reagent. Moreover, the first reagent storage mechanism 151 is arranged separated from the sample incubation module 14. That is, the first reagent storage mechanism 151 and the sample incubation module 14 are separately arranged, and there is a certain distance between the two. This can increase the storage capacity of the first reagent storage mechanism 151 and the sample incubation module 14 and ensure the operation efficiency of the blood analyzer 1. Moreover, it is also possible to facilitate the dispensing module 13 to transfer the sample and the reagent. Of course, in other implementations of the present invention, an axis of the first reagent storage mechanism 151 coincides with an axis of the sample incubation module 14. In this case, the sample incubation module 14 is arranged inside the first reagent storage mechanism 151, that is, the first reagent storage mechanism 151 is sheathed outside the sample incubation module 14, which can reduce the occupied space and thus reduce the volume of the blood analyzer 1.

Still further, the blood analyzer 1 further comprises a recovery module 18 and a cup feeding module 12 for transporting the reaction cup. The recovery module 18 is arranged on a side of the sample detection module 17, the cup feeding module 12 is arranged on a side of the sample incubation module 14, and the transfer module 16 can transfer an empty reaction cup transported by the cup feeding module 12 to the placement station 14311 of the sample incubation module 14. The transfer module 16 further can transfer the reaction cup that has been detected by the sample detection module 17 to the recovery module 18. The recovery module 18 has a cup discarding opening, the cup feeding module 12 has an empty cup picking-up position, the blood analyzer 1 has a cup picking-up and placement position provided corresponding to the sample incubation module 14, and the sample detection module 17 has a detection position. At least three of the empty cup picking-up position, the cup discarding opening, the cup picking-up and placement position and the detection position are arranged collinearly and correspond to a movement trajectory of the transfer module 16. For example, it is possible for the cup discarding opening, the empty cup picking-up position and the cup picking-up and placement position to be arranged collinearly, or it is also possible for the empty cup picking-up position, the cup picking-up and placement position and the sample detection module 17 to be arranged collinearly, or it is possible for all of the cup discarding opening, the empty cup picking-up position, the cup picking-up and placement position and the sample detection mechanism to be arranged collinearly, etc.

In this way, the transfer module 16 grasps the empty reaction cup at the empty cup picking-up position and transfers the empty reaction cup to the cup picking-up and placement position on the sample incubation module 14. The transfer module 16 can also remove the reaction cup from the cup picking-up and placement position, and transfer the reaction cup to the sample detection module 17 for detection. After the detection is completed, the detection module transfers the reaction cup from the sample detection module 17 to the cup discarding opening. The face that at least three of the empty cup picking-up position, the cup discarding opening, the cup picking-up and placement position and the detection position are arranged collinearly enables the transfer module 16 to perform a linear reciprocating movement in the same direction, reducing the space occupied by the transfer module 16, thereby reducing the overall size of the blood analyzer 1. The transfer module 16 performing a linear movement can also prevent the transfer module 16 or other components from being interfered with, to ensure the reliability of the movement.

It should be noted that the structures and working principles of the sample transport module 11, the cup feeding module 12, the dispensing module 13, the sample incubation module 14, the reagent storage module 15, the transfer module 16, the stirring module 19, the sample detection module 17 and other parts in the blood analyzer 1 of this implementation are exactly the same as those of the sample transport module 11, the cup feeding module 12, the dispensing module 13, the sample incubation module 14, the reagent storage module 15, the transfer module 16 and the sample detection module 17 of the blood analyzer 1 of the above implementation of the present invention, and will not described in detail here since the structures and working principles of the sample transport module 11, the cup feeding module 12, the dispensing module 13, the sample incubation module 14, the reagent storage module 15, the transfer module 16 and the sample detection module 17 in the above implementation of the present invention have been introduced in detail.

Referring to Figs. 1 to 3 and Figs. 21 to 24, the present invention further provides a blood analyzer 1, comprising a cup feeding module 12 for transporting a reaction cup, a sample transport module 11 for transporting a sample to be tested, a dispensing module 13 for aspirating and discharging the sample or a reagent, a sample incubation module 14 for incubating the sample, a transfer module 16 for transferring the reaction cup, a reagent storage module 15 for storing a reagent, a sample detection module 17 for detecting the sample, and a recovery module 18. The sample incubation module 14 is provided in a disk-shaped structure. The sample incubation module 14 is provided with a plurality of placement stations 14311 for placing the reaction cup. The sample incubation module 14 can rotate and drive the reaction cup in the placement station 14311 to rotate. The reagent storage module 15 comprises a rotatable first reagent storage mechanism 151. The first reagent storage mechanism 151 can store a plurality of reagents. The cup feeding module 12, the transfer module 16 and the sample detection module 17 are located on one side of the sample incubation module 14. The recovery module 18 is arranged on a side of the sample detection module 17. The transfer module 16 can transfer the reaction cup to the placement station 14311 of the sample incubation module 14. The dispensing module 13 is located between the sample incubation module 14, the first reagent storage mechanism 151, the sample transport module 11 and the sample detection module 17. The dispensing module 13 can transfer the sample from the sample transport module 11 to the reaction cup in the sample incubation module 14. The dispensing module 13 can further transfer the reagent from the first reagent storage mechanism 151 to the reaction cup in the sample incubation module 14 or to the reaction cup in the sample detection module 17. The transfer module 16 can transfer the reaction cup from the sample incubation module 14 to the sample detection module 17. The transfer module 16 further can transfer the reaction cup that has been detected by the sample detection module 17 to the recovery module 18.

The recovery module 18 has a cup discarding opening, the cup feeding module 12 has an empty cup picking-up position, the blood analyzer 1 has a cup picking-up and placement position provided corresponding to the sample incubation module 14, and the sample detection module 17 has a detection position. At least three of the empty cup picking-up position, the cup discarding opening, the cup picking-up and placement position and the detection position are arranged collinearly and correspond to a movement trajectory of the transfer module 16. For example, it is possible for the cup discarding opening, the empty cup picking-up position and the cup picking-up and placement position to be arranged collinearly, or it is also possible for the empty cup picking-up position, the cup picking-up and placement position and the sample detection module 17 to be arranged collinearly, or it is possible for all of the cup discarding opening, the empty cup picking-up position, the cup picking-up and placement position and the sample detection mechanism to be arranged collinearly, etc.

In this way, the transfer module 16 grasps the empty reaction cup at the empty cup picking-up position and transfers the empty reaction cup to the cup picking-up and placement position on the sample incubation module 14. The transfer module 16 can also remove the reaction cup from the cup picking-up and placement position, and transfer the reaction cup to the sample detection module 17 for detection. After the detection is completed, the detection module transfers the reaction cup from the sample detection module 17 to the cup discarding opening. The face that at least three of the empty cup picking-up position, the cup discarding opening, the cup picking-up and placement position and the detection position are arranged collinearly enables the transfer module 16 to perform a linear reciprocating movement in the same direction, reducing the space occupied by the transfer module 16, thereby reducing the overall size of the blood analyzer 1. The transfer module 16 performing a linear movement can also prevent the transfer module 16 or other components from being interfered with, to ensure the reliability of the movement.

Further, the first reagent storage mechanism 151 is provided in a disk-shaped structure. The disk-shaped first reagent storage mechanism 151 can be easily rotated, and thus the reagent thereon can be rotated to the reagent aspirating position, which is convenient for the dispensing module 13 to aspirate in the reagent. Moreover, the first reagent storage mechanism 151 is arranged separated from the sample incubation module 14. That is, the first reagent storage mechanism 151 and the sample incubation module 14 are separately arranged, and there is a certain distance between the two. This can increase the storage capacity of the first reagent storage mechanism 151 and the sample incubation module 14 and ensure the operation efficiency of the blood analyzer 1. Moreover, it is also possible to facilitate the dispensing module 13 to transfer the sample and the reagent.

Still further, the sample detection module 17 comprises a magnetic bead detection mechanism 171 and an optical detection mechanism. The magnetic bead detection mechanism 171 performs a magnetic bead detection on the sample, and the optical detection mechanism performs an optical detection on the sample. The magnetic bead detection mechanism 171 can perform a magnetic bead detection on the sample, and the optical detection mechanism can perform an optical detection on the sample. When the sample needs to be subjected to the corresponding detection, the transfer module 16 can transfer the reaction cup, in which the sample and the reagent have been added, from the sample incubation module 14 to the optical detection mechanism or the magnetic bead detection mechanism 171.

It should be noted that the structures and working principles of the sample transport module 11, the cup feeding module 12, the dispensing module 13, the sample incubation module 14, the reagent storage module 15, the transfer module 16, the stirring module 19, the sample detection module 17 and other parts in the blood analyzer 1 of this implementation are exactly the same as those of the sample transport module 11, the cup feeding module 12, the dispensing module 13, the sample incubation module 14, the reagent storage module 15, the transfer module 16 and the sample detection module 17 of the blood analyzer 1 of the above implementation of the present invention, and will not described in detail here since the structures and working principles of the sample transport module 11, the cup feeding module 12, the dispensing module 13, the sample incubation module 14, the reagent storage module 15, the transfer module 16 and the sample detection module 17 in the above implementation of the present invention have been introduced in detail.

Referring to Figs. 1 to 3 and Figs. 21 to 24, the present invention further provides a control method for a blood analyzer 1, wherein the blood analyzer 1 comprises a sample transport module 11 for transporting a sample to be tested, a dispensing module 13 for aspirating and discharging the sample or a reagent, a sample incubation module 14 for incubating the sample, a transfer module 16 for transferring a reaction cup, and a sample detection module 17 for detecting the sample.

The sample incubation module 14 is provided in a disk-shaped structure. The sample incubation module 14 is provided with a plurality of placement stations 14311 for placing the reaction cup. The sample incubation module 14 can rotate and drive the reaction cup in the placement station 14311 to rotate.

The sample detection module 17 comprises a magnetic bead detection mechanism 171 and an optical detection mechanism. The magnetic bead detection mechanism 171 performs a magnetic bead detection on the sample, and the optical detection mechanism performs an optical detection on the sample.

The control method for the blood analyzer 1 comprises the following steps:
obtaining detection information of a test item of the sample;
placing, which can be performed by the transfer module 16, the empty reaction cup to the placement station 14311 of the sample incubation module 14;
aspirating, by the dispensing module 13, the sample at the sample transport module 11 or the reagent at the reagent storage module 15, and adding the sample or the reagent to the reaction cup in the sample incubation module 14; and
transferring, which can be performed by the transfer module 16, the reaction cup, in which the sample and the reagent have been added, to the magnetic bead detection mechanism 171 or the optical detection mechanism.

When the blood analyzer 1 of the present invention is detecting the sample, the main control module 22 of the blood analyzer 1 first obtains the detection information of the test items of the sample, and the main control module 22 then controls the transfer module 16, so as to transfer the empty reaction cup to the placement station 14311 of the sample incubation module 14. The main control module 22 then controls the dispensing module 13, so as to transfer the corresponding sample and the various reagents required for the detection of the items of the sample to the corresponding reaction cups on sample incubation module 14. Subsequently, the main control module 22 controls the transfer module 16 according to the detection information of the item of the sample, so as to transfer the reaction cup, in which the sample and the reagent have been added, to the magnetic bead detection mechanism 171 or the optical detection mechanism, that is, when a magnetic bead detection is performed on the sample, the main control module 22 controls the transfer module 16 according to a control instruction, so as to transfer the reaction cup, in which the sample and the reagent have been added, to the magnetic bead detection mechanism 171; and when an optical detection is performed on the sample, the main control module 22 controls the transfer module 16 according to a control instruction, so as to transfer the reaction cup, in which the sample and the reagent have been added, to the optical detection mechanism.

Further, the detection information is obtained by scanning a detection code on a container containing the sample. In this case, the detection code on the container can be scanned by the scanning mechanism 113 of the main control module 22, and the detection information of the item of the sample is obtained according to the detection code, to facilitate the main controller to issue a control instruction. Of course, in other implementations of the present invention, the detection information is information of a manually-input detection instruction of the sample. After the detection information of the item of the sample is input manually, the main controller controls the movements of the dispensing module 13, the sample incubation module 14, the first reagent storage mechanism 151, the sample detection module 17 and the transfer module 16 respectively, so as to transfer the sample and the corresponding reagent to the reaction cup, such that the corresponding item of the sample can be detected.

Still further, when the detection information is magnetic bead detection information, the transfer module 16 transfers the reaction cup to the magnetic bead detection mechanism 171 for magnetic bead detection. When the detection information of the sample is magnetic bead detection information, the main control module 22 controls the movement of the transfer module 16 so as to transfer the reaction cup to the magnetic bead detection mechanism 171 for magnetic bead detection. When the detection information is optical detection information, the transfer module 16 transfers the reaction cup to the optical detection mechanism for optical detection. When the detection information of the sample is optical detection information, the main control module 22 controls the movement of the transfer module 16 so as to transfer the reaction cup to the optical detection mechanism for optical detection.

Still further, when the optical detection information is immunoturbidimetric detection information, the transfer module 16 transfers the reaction cup to the immunoturbidimetric detection station on the optical detection mechanism for optical detection. When the optical detection information of the sample is immunoturbidimetric detection information, the main control module 22 controls the movement of the transfer module 16 so as to transfer the reaction cup to the immunoturbidimetric detection station on the optical detection mechanism for optical detection.

When the optical detection information is chromogenic substrate detection information, the transfer module 16 transfers the reaction cup to the chromogenic substrate detection station on the optical detection mechanism for optical detection. When the optical detection information of the sample is chromogenic substrate detection information, the main control module 22 controls the movement of the transfer module 16 so as to transfer the reaction cup to the chromogenic substrate detection station on the optical detection mechanism for optical detection. The blood analyzer 1 of the present invention performs, on the sample, the magnetic bead detection and optical detection, and the immunoturbidimetric detection and chromogenic substrate detection based on the optical method, to achieve the detection of different items of the sample to obtain different parameters of the sample.

Referring to Figs. 1 to 3, in one embodiment of the present invention, the dispensing module 13 comprises a puncture needle mechanism 131, an integrated needle mechanism 132 and a reagent needle mechanism 133. The blood analyzer 1 has a three-needle structure. The puncture needle mechanism 131 adds the sample to the reaction cup in the sample incubation module 14. The reagent needle mechanism 133 adds the reagent to the reaction cup in the sample incubation module 14. The integrated needle mechanism 132 adds the sample or the reagent to the reaction cup in the sample incubation module 14.

Referring to Figs. 21 to 23, in another embodiment of the present invention, the dispensing module 13 comprises an integrated needle mechanism 132 and a reagent needle mechanism 133. The blood analyzer 1 has a two-needle structure. The integrated needle mechanism 132 can add the sample or the reagent to the reaction cup in the sample incubation module 14. The reagent needle mechanism 133 can add the reagent to the reaction cup in the sample incubation module 14.

Referring to Fig. 24, in yet another embodiment of the present invention, the dispensing module 13 comprises a dispensing needle mechanism 134. The blood analyzer 1 has a one-needle structure. The dispensing needle mechanism 134 can add the sample or the reagent to the reaction cup in the sample incubation module 14.

The specific structures and working principles of the puncture needle mechanism 131, the integrated needle mechanism 132, the reagent needle mechanism 133, and the dispensing needle mechanism 134 of the blood analyzer 1 have been described above in detail, and will not be described in detail here.

The various technical features of the embodiments described above can be arbitrarily combined. In order to simplify the description, all possible combinations of the various technical features in the above embodiments have not been described. However, any combination of these technical features should be considered to fall with the scope of the disclosure of this description as long as there is no contradiction.

The above-mentioned embodiments merely represent several implementations of the present invention, giving specifics and details thereof, but should not be understood as limiting the scope of the present patent of invention thereby. It should be noted that a person of ordinary skill in the art could also make several alterations and improvements without departing from the spirit of the present invention and these would all fall within the scope of protection of the present invention. Therefore, the scope of protection of the present patent of invention shall be in accordance with the appended claims.

## Claims

1. A blood analyzer, comprising a sample transport module for transporting a sample to be tested, a dispensing module for sucking in and discharging the sample or a reagent, a sample incubation module for incubating the sample, a reagent storage module for storing the reagent, a transfer module for transferring a reaction cup, and a sample detection module for detecting the sample, wherein
the sample incubation module is provided in a disk-shaped structure, is provided with a plurality of placement stations for placing the reaction cup, and is capable of rotating and driving the reaction cup in the placement station to rotate;
the reagent storage module comprises a rotatable first reagent storage mechanism provided in a disk-shaped structure, the first reagent storage mechanism being capable of storing a plurality of reagents, and being arranged separated from the sample incubation module;
the transfer module is capable of transferring the reaction cup to the placement station of the sample incubation module;
the dispensing module is capable of transferring the sample from the sample transport module to the reaction cup in the sample incubation module, and is further capable of transferring the reagent from the first reagent storage mechanism to the reaction cup in the sample incubation module or to the reaction cup in the sample detection module; and
the transfer module is capable of transferring the reaction cup from the sample incubation module to the sample detection module.

2. A blood analyzer, comprising a sample transport module for transporting a sample to be tested, a dispensing module for aspirating and discharging the sample or a reagent, a sample incubation module for incubating the sample, a reagent storage module for storing the reagent, a transfer module for transferring a reaction cup, and a sample detection module for detecting the sample, wherein
the sample incubation module is provided in a disk-shaped structure, is provided with a plurality of placement stations for placing the reaction cup, and is capable of rotating and driving the reaction cup in one of the placement stations to rotate;
the reagent storage module comprises a rotatable first reagent storage mechanism, the first reagent storage mechanism being capable of storing a plurality of reagents;
the sample detection module comprises a magnetic bead detection mechanism and an optical detection mechanism, the magnetic bead detection mechanism performing a magnetic bead detection on the sample, and the optical detection mechanism performing an optical detection on the sample;
the transfer module is capable of transferring the reaction cup to one of the placement stations of the sample incubation module;
the dispensing module is capable of transferring the sample from the sample transport module to the reaction cup in the sample incubation module, and is further capable of transferring the reagent from the first reagent storage mechanism to the reaction cup in the sample incubation module or to the reaction cup in the sample detection module; and
the transfer module is capable of transferring the reaction cupfrom the sample incubation module to the magnetic bead detection mechanism or the optical detection mechanism.

3. The blood analyzer according to claim 2, wherein the first reagent storage mechanism is provided in a disk-shaped structure; and
the first reagent storage mechanism is arranged separately from the sample incubation module.

4. The blood analyzer according to claim 2, wherein the first reagent storage mechanism is provided in a disk-shaped structure; and
an axis of the first reagent storage mechanism coincides with an axis of the sample incubation module.

5. The blood analyzer according to claim 2, further comprising a recovery module and a cup feeding module for transporting the reaction cup, wherein the recovery module has a cup discarding opening, the cup feeding module has an empty cup picking-up position, the blood analyzer has a cup picking-up and placement position corresponding to the sample incubation module, the sample detection module has a detection position, and at least three of the empty cup picking-up position, the cup discarding opening, the cup picking-up and placement position and the detection position are arranged collinearly and correspond to a movement trajectory of the transfer module.

6. A blood analyzer, comprising a cup feeding module for transporting the reaction cup, a sample transport module for transporting a sample to be tested, a dispensing module for sucking in and discharging the sample or a reagent, a sample incubation module for incubating the sample, a transfer module for transferring the reaction cup, a reagent storage module for storing the reagent, a sample detection module for detecting the sample, and a recovery module, wherein
the sample incubation module is provided in a disk-shaped structure, is provided with a plurality of placement stations for placing the reaction cup, and is capable of rotating and driving the reaction cup in the placement station to rotate;
the reagent storage module comprises a rotatable first reagent storage mechanism, the first reagent storage mechanism being capable of storing a plurality of reagents;
the transfer module is capable of transferring the reaction cup to the placement station of the sample incubation module; the dispensing module is capable of transferring the sample from the sample transport module to the reaction cup in the sample incubation module, and is further capable of transferring the reagent from the first reagent storage mechanism to the reaction cup in the sample incubation module or to the reaction cup in the sample detection module;
the transfer module is capable of transferring the reaction cup from the sample incubation module to the sample detection module; the transfer module is further capable of transferring the reaction cup that has been detected by the sample detection module to the recovery module; and
the recovery module has a cup discarding opening, the cup feeding module has an empty cup picking-up workstation, the blood analyzer has a cup picking-up and placement workstation provided corresponding to the sample incubation module, the sample detection module has a detection workstation, and at least three of the empty cup picking-up workstation, the cup discarding opening, the cup picking-up and placement workstation and the detection workstation are arranged collinearly and correspond to a movement trajectory of the transfer module.

7. The blood analyzer according to claim 6, wherein the first reagent storage mechanism is provided in a disk-shaped structure; and
the first reagent storage mechanism is arranged separated from the sample incubation module.

8. The blood analyzer according to claim 6, wherein the sample detection module comprises a magnetic bead method detection mechanism and an optical method detection mechanism, the magnetic bead method detection mechanism performing a magnetic bead method detection on the sample, and the optical method detection mechanism performing an optical method detection on the sample;
the transfer module is capable of transferring the reaction cup, in which the sample and the reagent have been added, from the sample incubation module to the optical method detection mechanism or the magnetic bead method detection mechanism.

9. The blood analyzer according to claim 1, 2 or 6, further comprising a cup feeding module for transporting the reaction cup, wherein
the transfer module comprises a first transfer mechanism and a second transfer mechanism;
the first transfer mechanism is used to transport the reaction cup transported by the cup feeding module to the sample incubation module; and
the second transfer mechanism is used to transfer the reaction cup, in which the sample and the reagent have been added, from the sample incubation module to the detection module.

10. The blood analyzer according to claim 1, wherein the sample detection module comprises a magnetic bead method detection mechanism and an optical method detection mechanism, the magnetic bead method detection mechanism performing a magnetic bead method detection on the sample, and the optical method detection mechanism performing an optical method detection on the sample; and
the transfer module is capable of transferring the reaction cup, in which the sample and the reagent have been added, from the sample incubation module to the optical method detection mechanism or the magnetic bead method detection mechanism.

11. The blood analyzer according to claim 10, wherein the transfer module comprises a first transfer mechanism and a second transfer mechanism; and
there are two said optical method detection mechanisms, which are respectively a first optical detection mechanism and a second optical detection mechanism, and the magnetic bead detection mechanism, the second optical detection mechanism and the first optical detection mechanism are arranged in sequence.

12. The blood analyzer according to claim 11, wherein the first transfer mechanism is used to transport the reaction cup transported by the cup feeding module to the sample incubation module and transfer the reaction cup, in which the sample and the reagent have been added, from the sample incubation module to the first optical detection mechanism; and
the second transfer mechanism is used to transfer the reaction cup, in which the sample and the reagent have been added, from the sample incubation module to the second optical detection mechanism or the magnetic bead detection mechanism.

13. The blood analyzer according to claim 2, 8 or 10, further comprising a main control module, wherein the main control module is electrically connected to the transfer module, and the main control module controls the transfer module according to an instruction, so as to transfer the reaction cup from the sample incubation module to the magnetic bead method detection mechanism or the optical method detection mechanism.

14. The blood analyzer according to claim 2, 8 or 10, wherein there are two said optical method detection mechanisms, which are respectively a first optical method detection mechanism and a second optical method detection mechanism; and
the blood analyzer further comprises a main control module, wherein the main control module is electrically connected to the transfer module, and the main control module controls the transfer module according to an instruction so as to transfer the reaction cup from the sample incubation module to the first optical method detection mechanism or the second optical method detection mechanism.

15. The blood analyzer according to claim 2, 8 or 10, further comprising a main control module, wherein the main control module comprises a main controller and a scanning mechanism, the main controller is electrically connected to the scanning mechanism, and the scanning mechanism is used to scan a detection code on a container which contains the sample and is transported by the sample transport module, to obtain detection information of a test item of the sample in the container;
the main controller is further electrically connected to the dispensing module, the sample incubation module, the first reagent storage mechanism, the sample detection module and the transfer module; and
the scanning mechanism is capable of transmitting the obtained detection information of the test item of the sample to the main controller, the main controller controls the dispensing module according to the detection information so as to transfer the corresponding reagent from the first reagent storage mechanism into the reaction cup in the sample incubation module, and the main controller then controls the movement of the transfer module according to the detection information, such that the transfer module transfers the reaction cup, in which the sample and the reagent have been added, from the sample incubation module to the sample detection module.

16. The blood analyzer according to claim 15, wherein when the detection information is magnetic bead detection information, the main control module controls the movement of the transfer module so as to transfer the reaction cup to the magnetic bead detection mechanism for magnetic bead detection; and
when the detection information is optical detection information, the main control module controls the movement of the transfer module so as to transfer the reaction cup to the optical detection mechanism for optical detection.

17. The blood analyzer according to claim 16, wherein the optical detection mechanism has an immunoturbidimetric detection position and a chromogenic substrate detection position, wherein
when the optical detection information is immunoturbidimetric detection information, the main control module controls the movement of the transfer module so as to transfer the reaction cup to the immunoturbidimetric detection position on the optical detection mechanism for optical detection; and
when the optical detection information is chromogenic substrate detection information, the main control module controls the movement of the transfer module so as to transfer the reaction cup to the chromogenic substrate detection position on the optical detection mechanism for optical detection.

18. The blood analyzer according to claim 1 or 2, further comprising a recovery module, wherein the recovery module comprises a first recovery mechanism and a second recovery mechanism, the first recovery mechanism is provided on one side of the first optical detection mechanism, and the second recovery mechanism is provided on one side of the second optical detection mechanism; and
the first transfer mechanism is further capable of discarding the reaction cup that has been detected by the first optical detection mechanism into the first recovery mechanism, and the second transfer mechanism is further capable of discarding the reaction cup, which has been detected by the second optical detection mechanism or the magnetic bead detection mechanism, into the second recovery mechanism.

19. The blood analyzer according to claim 18, wherein the first recovery mechanism has a first cup discarding opening, and at least three of the first cup discarding opening, the empty cup picking-up workstation, the cup picking-up and placement workstation and the first optical method detection mechanism are arranged collinearly and correspond to a movement trajectory of the first transfer mechanism.

20. The blood analyzer according to claim 1, 2 or 6, wherein the dispensing module comprises a dispensing needle mechanism, wherein
the dispensing needle mechanism is capable of transferring the sample from the sample transport module to the reaction cup in the sample incubation module, and the dispensing module is further capable of transferring the reagent from the first reagent storage mechanism to the reaction cup in the sample incubation module or to the reaction cup in the sample detection module.

21. The blood analyzer according to claim 20, wherein the blood analyzer is provided with a cup picking-up and placement workstation, a dispensing workstation and a cup picking-up workstation which surround the sample incubation module; and the blood analyzer has a dispensing reagent suction workstation at the first reagent storage mechanism, and has a dispensing reagent adding workstation at the sample detection module.

22. The blood analyzer according to claim 1, 2 or 6, wherein the dispensing module comprises an integrated needle mechanism and a reagent needle mechanism, wherein the integrated needle mechanism is used to aspirate and discharge the sample or the reagent, and the reagent needle mechanism is used to aspirate and discharge the reagent;
the integrated needle mechanism is located between the sample incubation module, the reagent storage module and the sample transport module, and the integrated needle mechanism is capable of transferring the sample from the sample transport module to the reaction cup in the sample incubation module, and is further capable of transferring the reagent from the first reagent storage mechanism to the reaction cup in the sample incubation module; and
the reagent needle mechanism is located between the sample incubation module, the reagent storage module and the sample detection module, and the reagent needle mechanism is capable of transferring the reagent from the first reagent storage mechanism to the reaction cup in the sample incubation module or to the reaction cup in the sample detection module.

23. The blood analyzer according to claim 22, wherein the blood analyzer is provided with a cup picking-up and placement workstation, a sample adding workstation, a first reagent adding workstation and a cup picking-up workstation which surround the sample incubation module; and the blood analyzer has a first reagent suction workstation and a second reagent suction workstation at the first reagent storage mechanism, and has a third reagent adding workstation at the sample detection module.

24. The blood analyzer according to claim 1, 2 or 6, wherein the dispensing module comprises a puncture needle mechanism, an integrated needle mechanism and a reagent needle mechanism, wherein the puncture needle mechanism is used to aspirate and discharge the sample, the integrated needle mechanism is used to suck in and discharge the sample or the reagent, and the reagent needle mechanism is used to suck in and discharge the reagent;
the puncture needle mechanism is located between the sample transport module and the sample incubation module, and the puncture needle mechanism is capable of transferring the sample transported by the sample transport module to the reaction cup in the sample incubation module;
the integrated needle mechanism is located between the sample incubation module, the reagent storage module and the sample transport module, and the integrated needle mechanism is capable of transferring the reagent from the first reagent storage mechanism to the reaction cup in the sample incubation module, or the integrated needle mechanism is capable of transferring the sample from the sample transport module to the reaction cup in the sample incubation module; and
the reagent needle mechanism is located between the sample incubation module, the reagent storage module and the sample detection module, and the reagent needle mechanism is capable of transferring the reagent from the first reagent storage mechanism to the reaction cup in the sample incubation module or to the reaction cup in the sample detection module.

25. The blood analyzer according to claim 24, wherein the blood analyzer is provided with a cup picking-up and placement position, a sample adding position, a first reagent adding position, a second reagent adding position and a cup picking-up position which surround the sample incubation module; and the blood analyzer has a first reagent aspirating position and a second reagent aspirating position at the first reagent storage mechanism, and has a third reagent adding position at the sample detection module.

26. The blood analyzer according to claim 22 or 24, wherein the reagent needle mechanism comprises a reagent needle drive assembly, a reagent needle mounting assembly, and two reagent needles, wherein at least one of the two reagent needles has a heating function, the reagent needle drive assembly is in driving connection with the reagent needle mounting assembly, and the two reagent needles are arranged on the reagent needle drive assembly; and
the reagent needle drive assembly drives the reagent needle transmission assembly to move, such that the two reagent needles are capable of moving to the first reagent storage mechanism to aspirate the reagent, moving to the sample incubation module to add the reagent, or moving to the sample detection module to add the reagent.

27. The blood analyzer according to claim 26, wherein when one of the two reagent needles aspirates the reagent on the first reagent storage mechanism, the other reagent needle is located at a first avoidance space;
when one of the two reagent needles adds the reagent to the sample detection module, the other reagent needle is located at a second avoidance space;
when one of the two reagent needles adds the reagent to the sample incubation module, the other reagent needle is located at a third avoidance space; and
when one of the two reagent needles is being cleaned, the other reagent needle is located at a fourth avoidance space.

28. The blood analyzer according to claim 24, further comprising a cleaning module, wherein the cleaning module comprises a puncture needle cleaning mechanism for cleaning a puncture needle of the puncture needle mechanism; and
the cleaning mechanism comprises a puncture needle cleaning pool and a reservoir, the puncture needle cleaning pool is arranged on the puncture needle mechanism, the reservoir is arranged below the puncture needle cleaning pool, and the puncture needle cleaning pool is used to clean the puncture needle of the puncture needle mechanism, and the reservoir is used to receive a cleaning liquid leaked from the puncture needle cleaning pool.

29. The blood analyzer according to claim 1, 2 or 6, further comprising a cup feeding module, wherein
the cup feeding module comprises a reaction cup storage mechanism, a reaction cup transport mechanism and a reaction cup transfer mechanism, the reaction cup transport mechanism is arranged obliquely, the reaction cup storage mechanism is arranged at a lower position of the reaction cup transport mechanism, and the reaction cup transfer mechanism is movably arranged on the reaction cup transport mechanism; and
the reaction cup transport mechanism transports the reaction cup transfer mechanism to a lower position of the reaction cup transport mechanism, the reaction cup storage mechanism transports the reaction cup to the reaction cup transfer mechanism, the reaction cup transport mechanism then transfers the reaction cup transfer mechanism to a higher position of the reaction cup transport mechanism, the reaction cup transfer mechanism has an empty cup picking-up position, and the transfer module grasps the reaction cup at the empty cup picking-up position.

30. The blood analyzer according to claim 1, 2 or 6, wherein the reagent storage module further comprises a second reagent storage mechanism for storing a reagent, the second reagent storage mechanism being provided between the first reagent storage mechanism and the sample transport module; and
the integrated needle mechanism is capable of aspirating the reagent from the second reagent storage mechanism and adding the reagent to the sample incubation module.

31. The blood analyzer according to claim 30, wherein the second reagent storage mechanism is further capable of storing an emergency sample, the second reagent storage mechanism is movable to the dispensing module; and
the dispensing module is capable of aspirating the emergency sample from the second reagent storage mechanism and adding the emergency sample to the reaction cup in the sample incubation module.

32. The blood analyzer according to claim 1, 2 or 6, further comprising a stirring module, wherein the stirring module is arranged close to the sample incubation module, and is capable of performing a two-dimensional movement in a vertical plane; and
the blood analyzer is further provided with a stirring workstation, and the stirring module stirs the reaction cup, in which the sample and the reagent have been added, in the sample incubation module at the stirring workstation.

33. The blood analyzer according to claim 1, 2 or 6, wherein the sample transport module comprises an automatic sample feeding mechanism, which is used to automatically transport a plurality of containers which are arranged on a sample rack in a row, and contain samples;
the automatic sample feeding mechanism is provided with a puncture workstation and a non-puncture position, the non-puncture position and the puncture position are arranged in sequence, and the dispensing module is capable of aspirating the sample from the container at the puncture position and the non-puncture position respectively; and
the distance between the non-puncture position and the puncture position is at least one multiple of the distance between two adjacent said containers.

34. The blood analyzer according to claim 1, 2 or 6, further comprising a test housing, wherein the test housing has a placement cavity and a testing platform covering the placement cavity, and the sample transport module, the dispensing module, the sample incubation module, the reagent storage module, the transfer module and the detection module are all located on the testing platform;
further comprising a main control module and a power supply module, wherein the power supply module is electrically connected to the main control module, the main control module is electrically connected to the sample transport module, the dispensing module, the sample incubation module, the reagent storage module, the transfer module and the detection module respectively, and the main control module and the power supply module are located in the placement cavity; and
further comprising a liquid circuit module, wherein the liquid circuit module comprises a liquid circuit control mechanism, a liquid storage mechanism and a waste liquid mechanism, which are provided in the placement cavity.

35. A control method for a blood analyzer, wherein the blood analyzer comprises a sample transport module for transporting a sample to be tested, a dispensing module for aspirating and discharging the sample or a reagent, a sample incubation module for incubating the sample, a transfer module for transferring the reaction cup, and a sample detection module for detecting the sample, wherein
the sample incubation module is provided in a disk-shaped structure, is provided with a plurality of placement stations for placing the reaction cup, and is capable of rotating and driving the reaction cup in the placement station to rotate;
the sample detection module comprises a magnetic bead detection mechanism and an optical detection mechanism, the magnetic bead detection mechanism performing a magnetic bead detection on the sample, and the optical detection mechanism performing an optical detection on the sample; and
the control method for the blood analyzer comprises the following steps:
obtaining detection information of a test item of the sample;
placing, which can be performed by the transfer module, the empty reaction cup to the placement station of the sample incubation module;
aspirating, by the dispensing module, the sample at the sample transport module or the reagent at the reagent storage module, and adding the sample or the reagent to the reaction cup in the sample incubation module; and
transferring, which can be performed by the transfer module, the reaction cup, in which the sample and the reagent have been added, to the magnetic bead detection mechanism or the optical detection mechanism.

36. The control method for a blood analyzer according to claim 34 or 35, wherein the detection information is obtained by scanning a detection code on a container containing the sample;
or the detection information is information of a manually-input detection instruction of the sample.

37. The control method for a blood analyzer according to claim 36, wherein when the detection information is magnetic bead method detection information, the transfer module transfers the reaction cup to the magnetic bead method detection mechanism for magnetic bead method detection; and
when the detection information is optical method detection information, the transfer module transfers the reaction cup to the optical method detection mechanism for optical method detection.

38. The blood analyzer according to claim 36, wherein the optical method detection mechanism is provided with an immunoturbidimetric method detection station and a chromogenic substrate method detection station, wherein
when the detection information is optical method detection information, the transfer module transfers the reaction cup to the optical method detection mechanism for optical method detection;
when the optical method detection information is immunoturbidimetric method detection information, the transfer module transfers the reaction cup to the immunoturbidimetric method detection station on the optical method detection mechanism for optical method detection; and
when the optical method detection information is chromogenic substrate method detection information, the transfer module transfers the reaction cup to the chromogenic substrate method detection station on the optical method detection mechanism for optical method detection.

39. The control method for a blood analyzer according to claim 35, wherein the dispensing module comprises a dispensing needle mechanism, wherein
the dispensing needle mechanism is capable of adding the sample or the reagent to the reaction cup in the sample incubation module.

40. The control method for a blood analyzer according to claim 35, wherein the dispensing module comprises an integrated needle mechanism and a reagent needle mechanism, wherein
the integrated needle mechanism is capable of adding the sample or the reagent to the reaction cup in the sample incubation module; and
the reagent needle mechanism is capable of adding the reagent to the reaction cup in the sample incubation module.

41. The control method for a blood analyzer according to claim 35, wherein the dispensing module comprises a puncture needle mechanism, an integrated needle mechanism and a reagent needle mechanism, wherein
the puncture needle mechanism adds the sample to the reaction cup in the sample incubation module;
the reagent needle mechanism adds the reagent to the reaction cup in the sample incubation module; and
the integrated needle mechanism adds the sample or the reagent to the reaction cup in the sample incubation module.
